(19) Europäisches Patentamt
European Patent Office
Office européen des brevets

(11) Veröffentlichungsnummer: **0 361 273 B1**

(12) # EUROPÄISCHE PATENTSCHRIFT

(45) Veröffentlichungstag der Patentschrift: **23.03.94**

(21) Anmeldenummer: **89117266.0**

(22) Anmeldetag: **19.09.89**

(51) Int. Cl.$^5$: **C07C 59/42**, C07C 69/606, C07D 213/55, C07D 237/08, C07D 239/26, C07D 207/08, C07D 309/30, C07D 405/06, C07C 59/76, C07C 47/222, A61K 31/365

(54) **7-Substituierte Derivate der 3,5-Dihydroxyhept-6-insäure, Verfahren zu ihrer Herstellung, ihre Verwendung als Arzneimittel, sowie Zwischenprodukte.**

(30) Priorität: **24.09.88 DE 3832570**

(43) Veröffentlichungstag der Anmeldung:
**04.04.90 Patentblatt 90/14**

(45) Bekanntmachung des Hinweises auf die Patenterteilung:
**23.03.94 Patentblatt 94/12**

(84) Benannte Vertragsstaaten:
**AT BE CH DE ES FR GB GR IT LI LU NL SE**

(56) Entgegenhaltungen:
**EP-A- 0 006 452** **EP-A- 0 068 038**
**EP-A- 0 221 025** **DE-A- 3 621 372**
**DE-A- 3 722 807** **DE-A- 3 800 785**

(73) Patentinhaber: **HOECHST AKTIENGESELLSCHAFT**

**D-65926 Frankfurt(DE)**

(72) Erfinder: **Kesseler, Kurt, Dr.**
**Gluckstrasse 20A**
**D-6232 Bad Soden am Taunus(DE)**
Erfinder: **Bartmann, Wilhelm, Prof. Dr.**
**Am Dachsbau 5**
**D-6232 Bad Soden am Taunus(DE)**
Erfinder: **Wess, Günther, Dr.**
**Langenselbolder Weg 35**
**D-6455 Erlensee(DE)**
Erfinder: **Granzer, Ernold, Dr.Dr.**
**Falkensteiner Strasse 24**
**D-6233 Kelkheim(Taunus)(DE)**

EP 0 361 273 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

JOURNAL OF MEDICINAL CHEMISTRY, Band 28, Nr. 3, 1985, Seiten 347-358; G. E. STOKKER et al: "3- Hydroxy-3-methylglutaryl-coenzyme A Reductase Inhibitors. I. Structural Modification of 5-Substituted 3,5-Dihydroxypentanoic Acids and Their Lactone Derivatives"

JOURNAL OF MEDICINAL CHEMISTRY, Band 29, Nr. 2, 1986, Seiten 170-181; G. E. STOKKER et al: "3-Hydroxa-3-methylglutaryl-coenzyme A Reductase Inhibitors. 3. 7-(3,5-Disubstituted (1,1'-biphenyl)-2yl)-3,5-dihydroxy-6-heptenoic Acids and Their Lactone Derivatives"

TETRAHEDRON LETTERS, Band 29, Nr. 8, 1988, Seiten 929, 930, GB; E. BAADER et al: "Synthesis of a novel HMG-CoA reductase inhibitor"

JOURNAL OF THE CHEMICAL SOCIETY - PERKIN TRANSACTIONS I, Nr. 6, Juni 1989, Seiten 1165, 1166, London, GB; N. G. KUNDU et al: "A General Procedure for the Synthesis of Dimethoxypyrimidines and Uracils with Highly Functionalised C-5 Substituents"

## Beschreibung

Die Umwandlung von 3-Hydroxy-3-methylglutarsäure (HMG) zu Mevalonsäure bildet den Schlüsselschritt bei der Biosynthese von Cholesterin. Dieser Schritt wird durch das Enzym 3-Hydroxy-3-methylglutaryl-Coenzym-A-Reduktase (HMG-CoA-Reduktase) katalysiert. Eine Vielzahl von Verbindungen, die die Aktivität der HMG-CoA-Reduktase hemmen, sind beschrieben worden. In den meisten Fällen handelt es sich hierbei um Derivate der 3,5-Dihydroxyheptansäure und 3,5-Dihydroxyhept-6E-ensäure, sowie um Derivate der 3,5-Dihydroxy-6-oxy-hexansäure (vgl. z. B. Drugs of the Future 12, 437 (1987)), die in Position 7 bzw. am Sauerstoffatom durch sterisch anspruchsvolle lipophile Reste substituiert sind. Als geeignete lipophile Teilstrukturen sind z.B. Hexahydronaphthylreste, wie bei den Naturstoffen Compactin (vgl. A. G. Brown et al., J. Chem. Soc. Perkin Trans 1, 15 1976, 1165) und Mevinolin (vgl. A. W. Alberts et al., Proc. Natl. Sci. U.S.A. 77, 3957 (1980)), substituierte Phenylkerne (vgl. z.B. G. E. Stokker et al., J. Med. Chem. 29, 170 (1986)), heteroaromatische Reste (vgl. z. B. Drugs of the Future 12, 437 (1987), EP-A-0 221 025, oder auch dreifach substituierte Ethylengruppen (vgl. z.B. E. Baader et al., Tetrahedron Lett. 29, 929 (1988)) beschrieben worden.

Substitutionsgrad und -muster der genannten lipophilen Reste sind von entscheidender Bedeutung für die biologische Wirksamkeit der HMG-CoA-Reduktase-Inhibitoren. Durch geeignete Substitution der lipophilen Reste können Enzymhemmwerte von $IC_{50} \leqq 1 \cdot 10^{-8}$ mol/Liter erreicht werden.

Über 7-substituierte 3,5-Dihydroxhept-6-insäurederivate und ihre Hemmwirkung auf die HMG-CoA-Reduktase ist nur sehr wenig publiziert worden. In der Literatur ist unseres Wissens nur ein einzelnes Beispiel eines 7-phenylsubstituierten Derivates erwähnt (G. E. Stokker et al., J. Med. Chem. 28 , 346 (1985)). Die Enzymhemmung dieser Verbindung ist jedoch schwach ($IC_{50} \gg 1 \cdot 10^{-6}$ mol/Liter) und darüber hinaus deutlich geringer als die der identisch substituierten 3,5-Dihydroxyheptan- bzw. 3,5 Dihydroxyhept-6E-ensäure.

Im Gegensatz zu diesen Ergebnissen wurde gefunden, daß Derivate der 3,5-Dihydroxyhept-6-insäure der allgemeinen Formeln I und II

a) potente Inhibitoren der HMG-CoA-Reduktase sind ($IC_{50} \leqq 1 \cdot 10^{-8}$ mol/Liter) und

b) eine gleich große oder größere Hemmwirkung haben als die korrespondierenden, d. h. an Position 7 identisch substituierten Derivate der 3,5-Dihydroxyhept-6E-ensäure.

Die Erfindung betrifft daher 7-substituierte 3,5-Dihydroxyhept-6-insäuren und deren Derivate der Formel I

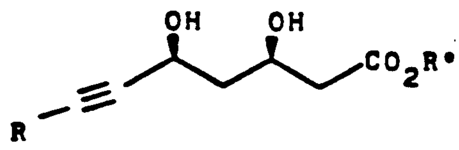

sowie die entsprechenden Laktone der Formel II

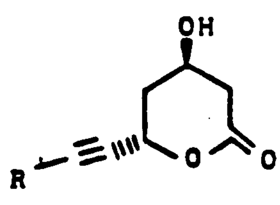

II

In den Formeln I und II bedeutet R

a) einen Rest der Formel a,

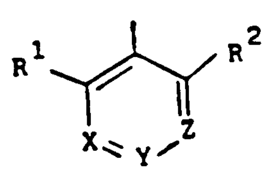

a

worin
$R^1$ und $R^2$ unabhängig voneinander
ein geradkettiger oder verzweigter Alkyl- oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind, und
X = Y-Z eine Gruppe der Formel $CR^3=CR^4-CR^5$, $N=CR^4-CR^5$, $N=N-CR^5$, $N=CR^4-N$ ist, worin
$R^3$, $R^4$, $R^5$ unabhängig voneinander
Wasserstoff, ein geradkettiger oder verzweigter Alkyl-oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind,
b) einen Rest der Formel b,

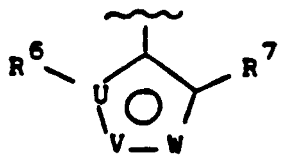

b

worin
$R^6$ und $R^7$ unabhängig voneinander
ein geradkettiger oder verzweigter Alkyl- oder Alkenylrest mit bis zu 6 C-Atomen,
ein gesättigter oder bis zu 2-fach ungesättigter cyclischer Kohlenwasserstoffrest mit 3-6 C-Atomen, ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen und Alkoxy mit bis zu 4 C-Atomen sind, und
U-V-W
eine Gruppe der Formel $C-NR^9-CR^8$, $C-O-CR^8$, $C-S-CR^8$, $C-NR^9-N$, C-O-N (= C-N-O), C-S-N (= C-N-S), $N-CR^{10}=CR^8$, $N-N=CR^8$ oder $N-CR^{10}=N$, ist,
worin
$R^8$ Wasserstoff
ein geradkettiger oder verzweigter Alkyl- oder Alkenylrest mit bis zu 6 C-Atomen,
ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit 1-4 C-Atomen und Hydroxy, ist und
$R^9$, $R^{10}$ unabhängig voneinander
ein geradkettiger oder verzweigter Alkyl- oder Alkenylrest mit bis zu 6 C-Atomen,
ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen und Alkoxy mit bis zu 4 C-Atomen,
sind,
oder
c) einen Rest der Formel c,

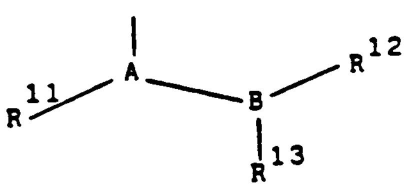

c

worin

A-B eine Gruppe der Formel CH-CH oder C = C ist und

$R^{11}$, $R^{12}$, $R^{13}$ unabhängig voneinander

ein geradkettiger oder verzweigter Alkyl- oder Alkenylrest mit bis zu 20 C-Atomen,

ein gesättigter oder bis zu 2-fach ungesättigter cyclischer Kohlenwasserstoffrest mit 3-6 C-Atomen oder ein Phenylrest, der gegebenenfalls substituiert ist mit 1-3 Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind,

und

$R^{\circ}$ Wasserstoff, einen geradkettigen oder verzweigten Alkylrest mit bis zu 6 C-Atomen, Alkalimetall oder Ammonium.

Unter den Substituenten R sind bevorzugt:

a) ein Rest der Formel a, worin

$R^1$ ein geradkettiger oder verzweigter Alkylrest mit bis zu 4 C-Atomen oder ein Cycloalkylrest mit 3-6 C-Atomen ist,

$R^2$ ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, ist und

X = Y-Z

eine Gruppe der Formel $CR^3 = CR^4$-$CR^5$, N = $CR^4$-$CR^5$, N = N-$CR^5$ oder N = $CR^4$-N ist,

worin

$R^3$, $R^5$ unabhängig voneinander Wasserstoff,

ein geradkettiger oder verzweigter Alkylrest mit bis zu 4 C-Atomen, ein Cycloalkylrest mit 3-6 C-Atomen oder ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, sind und

$R^4$ ein geradkettiger oder verzweigter Alkylrest mit bis zu 4 C-Atomen,

ein Cycloalkylrest mit 3-6 C-Atomen, oder

ein Phenylrest, der gegebenenfalls substituiert ist mit 1-3 gleichen oder verschiedenen Rest ausgewählt aus der Gruppe bestehend aus C1-C4-Alkyl, Fluor, Chlor, Alkoxy mit 1-4 C-Atomen und Hydroxy,

ist,

b) ein Rest der Formel b, worin

$R^6$ ein geradkettiger oder verzweigter Alkylrest mit bis zu 4 C-Atomen, ein Cycloalkylrest mit 3-6 C-Atomen ist,

$R^7$ ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, ist und

U-V-W eine Gruppe der Formel C-$NR^9$-$CR^8$ ist,

worin

$R^8$ Wasserstoff, ein geradkettiger oder verzweigter Alkylrest mit bis zu 4 C-Atomen,

ein Cycloalkylrest mit 3-6 C-Atomen, oder

ein Phenylrest, der gegebenenfalls substituiert ist mit 1-3 gleichen oder verschiedenen Resten ausgewählt aus der Gruppe bestehend aus C1-C4-Alkyl, Fluor and Chlor ist und

$R^9$ ein geradkettiger oder verzweigter Alkylrest mit bis zu 4 C-Atomen,

ein Cycloalkylrest mit 3-6 C-Atomen oder ein Phenylrest, der gegebenenfalls substituiert ist mit 1-3 gleichen oder verschiedenen Resten ausgewählt aus der Gruppe bestehend aus C1-C4-Alkyl, Fluor und Chlor, ist

c) ein Rest der Formel c, worin

A-B eine Gruppe der Formel CH-CH oder C = C ist,

$R^{11}$ ein geradkettiger oder verzweigter Alkylrest mit bis zu 6 C-Atomen,

ein Cycloalkylrest mit 3-6 C-Atomen ist und

$R^{12}$, $R^{13}$ unabhängig voneinander

ein geradkettiger oder verzweigter Alkylrest mit bis zu 6 C-Atomen,

ein Cycloalkylrest mit 3-6 C-Atomen,

ein Phenylrest, der gegebenenfalls substituiert ist mit 1-3 gleichen oder verschiedenen Resten ausgewählt aus der Gruppe bestehend aus C1-C4-Alkyl, Halogen, Alkoxy mit 1-4 C-Atomen und Hydroxy, sind.

Unter den Resten R° sind bevorzugt
Wasserstoff, ein geradkettiger oder verzweigter Alkylrest mit bis zu 4 C-Atomen, Natrium, Kalium.

Unter den Resten R sind besonders bevorzugt:

a) ein Rest der Formel a, worin

X=Y-Z eine Gruppe der Formel $CR^3=CR^4-CR^5$ ist, worin

$R^3$ Wasserstoff
$R^4$ Isopropyl, tert.-Butyl, Phenyl oder 4-Fluorphenyl, und
$R^5$ Wasserstoff bedeuten, und
$R^1$ Isopropyl und
$R^2$ 4-Fluorphenyl sind,

X=Y-Z eine Gruppe der Formel $N=CR^4-CR^5$ ist, worin

$R^4$ Isopropyl, tert.-Butyl, Phenyl oder 4-Fluorphenyl und
$R^5$ Wasserstoff bedeuten, und
$R^1$ Isopropyl oder Cyclopropyl und
$R^2$ 4-Fluorphenyl sind,

X=Y-Z eine Gruppe der Formel $N=N-CR^5$ ist, worin

$R^5$ Phenyl, 4-Fluorphenyl bedeutet, und
$R^1$ Isopropyl und
$R^2$ 4-Fluorphenyl sind,

X=Y-Z eine Gruppe der Formel $N=CR^4-N$ ist, worin

$R^4$ Isopropyl, tert.-Butyl, Phenyl oder 4-Fluorphenyl bedeutet, und
$R^1$ Isopropyl und
$R^2$ Fluorphenyl sind,

b) ein Rest der Formel b, worin

$R^6$ Isopropyl
$R^7$ 4-Fluorphenyl und,

U-V-W eine Gruppe der Formel $C-NR^9-CR^8$, worin

$R^8$ Wasserstoff
$R^9$ Isopropyl oder Phenyl bedeuten, sind,

c) ein Rest der Formel c, worin

A-B eine Gruppe der Formel C=C,

$R^{11}$ Isopropyl und
$R^{12}=R^{13}$ 4-Fluorphenyl oder 4-Fluor-3-methylphenyl sind.

Unter den Resten R° sind besonders bevorzugt Wasserstoff, Methyl, Ethyl, tert.-Butyl, Natrium und Kalium.

Die Erfindung betrifft die reinen Enantiomeren sowie die Racemate der Formel I und Mischungen aus diesen, also die Racemate mit der absoluten Konfiguration 3RS/5RS, sowie die reinen Enantiomeren mit der absoluten Konfiguration 3R/5S.

Die Erfindung betrifft ferner die reinen Enantiomeren sowie die Racemate der allgemeinen Formel II, die aus den obengenannten stereoisomeren Dihydroxycarbonsäure-Derivaten der allgemeinen Formel I hervorgehen. Im einzelnen sind dies die Racemate mit der absoluten Konfiguration 4RS/6SR und die reinen Enantiomeren mit der absoluten Konfiguration 4R/6S.

Die Erfindung betrifft ferner ein Verfahren zur Herstellung der Verbindungen der allgemeinen Formeln I und II, das dadurch gekennzeichnet ist, daß man

a) Aldehyde der Formel III

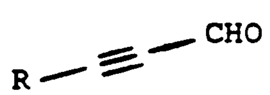

III

worin R die angegebene Bedeutung hat, in die entsprechenden Hydroxyketoester der allgemeinen Formel IV überführt,

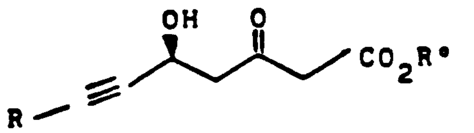

IV

worin R die angegebene Bedeutung hat und R° Alkyl mit 1-6 C-Atomen bedeutet,

b) die Hydroxyketoester der Formel IV in die entsprechenden 3,5-Dihydroxyverbindungen der Formel I überführt.

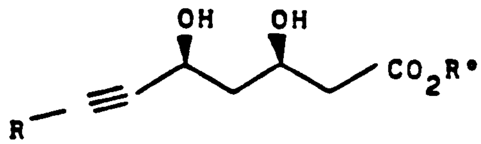

I

worin R die zu Formel I angegebene Bedeutung hat und R° Alkyl mit 1 bis 6 C-Atomen ist, und eine erhaltene Verbindung gegebenenfalls verseift zu einer Verbindung der Formel I, worin R° ein Alkalimetall darstellt, daraus gegebenenfalls die freie Säure (R° = Wasserstoff) in Freiheit setzt und die freie Säure gegebenenfalls in eine Verbindung der Formel I, worin R° die zu Formel I angegebenen Bedeutungen mit Ausnahme von Wasserstoff hat, überführt,

c) und eine erhaltene Verbindung der allgemeinen Formel I, gegebenenfalls in ein Lacton der Formel II,

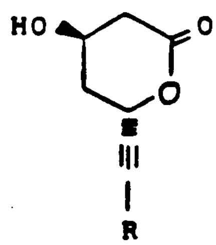

II

worin R die angegebene Bedeutung hat, überführt.

Die Umwandlung von Verbindungen der Formel III in Verbindungen der Formel IV erfolgt je nach Gegebenheiten und Erfordernissen nach verschiedenen Varianten, wie z. B.:

1. Reaktion des Dianions von Acetessigestern mit Aldehyden der Formel III führt in Lösungsmitteln wie THF bei - 78°C bis Raumtemperatur zu racemischen Verbindungen der Formel IV. Dianionen von Acetessigestern können mit verschiedenen Basen, vorzugsweise Natriumhydrid, n-Butyllithium und Lithiumdiisopropylamid (LDA) in etherischen Lösungsmitteln, vorzugsweise in Diethylether, THF oder Dimethoxyethan bei -40°C bis Raumtemperatur hergestellt werden.

2. Umsetzung von Enolaten nicht chiraler Essigsäureester, wie z. B. Methyl-, Ethyl- oder Propylester, die mit starken Basen, wie z. B. Metallamiden, vorzugsweise LDA in etherischen Lösungmitteln, beispielsweise THF bereitet werden, mit Aldehyden der Formel III führt in Lösungsmitteln wie z. B. THF bei Temperaturen zwischen -78°C und 0°C zu racemischen Verbindungen der Formel V,

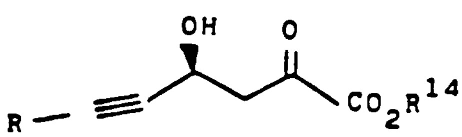

worin $R^{14}$ eine nicht chirale Säureschutzgruppe, wie z. B. die Methyl-, Ethyl- oder Propylgruppe bedeutet. Umsetzung mit einem weiteren Essigsäureesterenolat in Lösungsmitteln wie z. B. THF bei Temperaturen von -78°C bis 30°C führt zu zu racemischen Verbindungen der Formel IV.

3. Umsetzung von Aldehyden der Formel III mit Enolaten optisch aktiver Essigsäureester, vorzugsweise von Lithium- oder Magnesiumenolaten, führt in Lösungsmitteln wie z. B. THF bei Temperaturen von -78°C bis O°C zu optisch aktiven Addukten der Formel V. In diesem Fall bedeutet $R^{14}$ eine geeignete,

optisch aktive Säureschutzgruppe, die die Konfiguration an C-3 bestimmt. Vorzugsweise wird hier die Gruppe

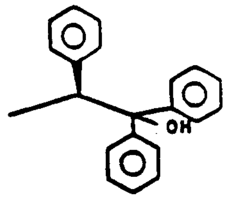

verwendet, die nach R. Devant, U. Mahler und M. Braun (Chem. Ber. 121, 397 (1988)) die 3R-Konfiguration ergibt und aus L-(+)-Mandelsäure hergestellt wird. Es eignen sich aber auch andere optisch aktive Gruppen. Die erhaltenen optisch aktiven Verbindungen der Formel V werden entweder direkt mit nicht chiralen Essigsäurresterenolaten zu optisch aktiven Verbindungen der Formel IV umgesetzt oder zunächst durch Umesterung in Alkylester der Formel V, vorzugsweise Methylester, umgewandelt, die dann nach Variante 2 in Verbindung der Formel IV überführt werden.

Die Umwandlung von Verbindungen der Formel IV in Verbindungen der Formel I erfolgt analog literaturbekannter Verfahren, (siehe z. B. K. Naraska, H. C. Pai, Chem. Lett. 1980, 1415 oder K. M. Chen, K. G Gunderson, G. E. Hardtmann, K. Prasad, O. Repic und M. J. Shapiro, Chem. Lett. 1978, 1923). Zunächst wird eine Verbindung der Formel IV mit einem Trialkyl- oder Alkoxydialkylboran, vorzugsweise Tiethyl- oder Methoxydiethylboran, umgesetzt und dann bei -78 °C bis 0 °C, gegebenenfalls unter Zusatz von Methanol reduziert. Man erhält so Verbindungen der Formel I mit der angegebenen relativen Konfiguration an C-3 und C-5. (3R*, 5S*).

Die Salze und Säuren von Verbindungen der allgemeinen Formel I werden nach allgemein bekannten Methoden erhalten.

Lactone der allgemeinen Formel II werden ebenfalls nach nach an sich bekannten Verfahren erhalten, z. B. durch Wasserabspaltung aus den Dihydroxycarbonsäuren der Formel I ($R^{\circ}$ = H) in Benzol, Hexan oder Toluol unter Zusatz von p-Toluolsulfonsäure bei Raumtemperatur bis Rückflußtemperatur oder aber aus Dihydroxycarbonsäureestern der Formel I, z. B. $R^{\circ}$ = Methyl, Ethyl, tert.-Butyl, in Dichlormethan unter Zusatz starker Säuren, wie z. B. Trifluoessigsäure bei Raumtemperatur bis Rückflußtemperatur.

Die racemischen Verbindungen der Formeln I und II können nach den bekannten Verfahren der Racematsspaltung in die reinen Enantiomeren getrennt werden.

Die im erfindungsgemäßen Verfahren als Ausgangsmaterial verwendeten Aldehyde III, worin R die zur allgemeinen Formel I gegebene Bedeutung hat, erhält man, wie z. B. in Schema 1 dargestellt. Geeignete Aldehyde der allgemeinen Formel VI, worin R die zur Formel I angegebene Bedeutung hat, sind entweder literaturbekannt oder können analog den in der Literatur beschriebenen Methode hergestellt werden:

Aldehyde VI mit Resten R der Formel a, X=Y-Z = $CR^3$= $CR^4$-$CR^5$:G. E. Stokker et al., J. Med. Chem 29, 170 (1986)

Aldehyde VI mit Resten R der Formel a, X=Y-Z = N=$CR^4$-$CR^5$, N=$CR^4$-N: Deutsche Offenlegungsschrift 38 23 045 (entsprechend EP-A-0307342; US-Patentanmeldung Serial No. 07/216458) (korrespondierende Alkohole R-$CH_2$OH werden beschrieben, aus denen Aldehyde der Formel III durch Oxidation nach bekannten Methoden, z. B. mit Pyridiniumchlorochromat, dargestellt werden können)

Aldehyde VI mit Resten R der Formel a, X=Y-Z = N=N-$CR^5$: deutsche Patentanmeldung P 38 00 785.1 (entsprechend US-Patentanmeldung Serial No. 07/294096) Aldehyde VI mit Resten R der Formel b, U-V-W = C-$NR^9$-$CR^8$: Deutsche Offenlegungschrift 37 22 806 (entsprechend EP-A-0300249; US-Patentanmeldung Serial No. 216 423)

Aldehyde VI mit Resten R der Formel b, U-V-W = C-O-$CR^8$, C-S-$CR^8$, N-$CR^{10}$ =$CR^8$:EP-A-O 221 025

Aldehyde VI mit Resten R der Formel b, U-V-W = C-$NR^9$-N, N-N-$CR^8$: WO 86/00307

Aldehyde VI mit Resten der Formel b, U-V-W = N-$CR^{10}$ =N: WO 86/07054

Aldehyde VI mit Resten R der Formel b, U-V-W = C-O-N, C-S-N: Deutsche Offenlegungsschrift 3621372 und dort zitierte Literatur (korrespondierende Alkohole R-$CH_2$OH werden beschrieben, aus denen die Aldehyde der Formel VI durch Oxidation nach an sich bekannten Methoden hergestellt werden)

Aldehyde VI mit Resten R der Formel c: Deutsche Offenlegungsschrift 37 22 807 (entsprechend EP-A-03 06 649; US-Patentanmeldung Serial No. 07/216331).

Die Überführung von Aldehyden der Formel VI in Carbonsäuren der Formel VIII erfolgt z. B. analog der von E. J. Corey und P. L. Fuchs, Tetrahedron Lett. 1972, 3769 beschriebenen Methode durch Darstellung der entsprechenden gem-Dibromolefine der allg. Formel VII und anschließende Umsetzung mit n-Butyllithium und Kohlendioxid.

Durch Veresterung nach an sich bekannten Methoden werden Carbonsäureester der Formel IX, worin $R^{15}$ einen Alkylrest, vorzugsweise einen Methyl- oder Ethylrest bedeutet, hergestellt. Diese werden nach bekannten Verfahren, z. B. durch Reduktion mit Metallhydriden wie $LiAlH_4$, Diisobutylaluminiumhydrid oder Vitride zu Alkoholen der Formel X reduziert. Anschließende Oxidation nach bekannten Verfahren, z. B. mit Pyridiniumchlorochromat, führt zu den Aldehyden der allg. Formel III.

**Schema 1**

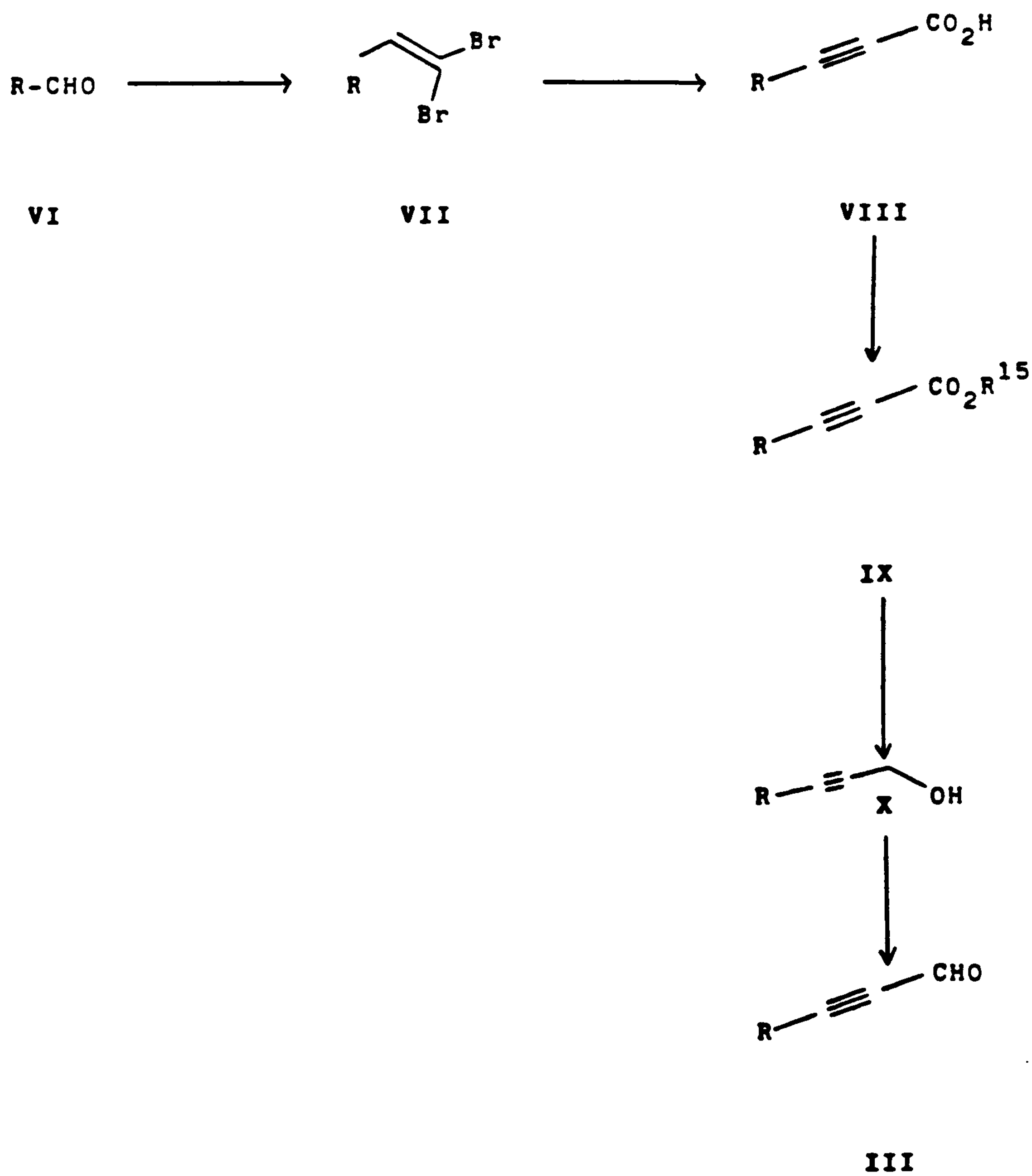

VI VII VIII IX X III

Zur Synthese von Verbindungen der allg. Formeln I und II mit phenolischen Hydroxygruppen ist es zweckmäßig, diese in geeigneter Weise zu schützen. Ausgehend von Aldehyden der allg. Formel III mit geschützten phenolischen Hydroxygruppen stellt man zunächst Verbindungen der allg. Formel I oder II mit

gleichfalls geschützten phenolischen Hydroxygruppen her und überführt diese anschließend in Verbindungen der allg. Formel I oder II mit freien phenolischen Hydroxygruppen. Hierzu geeignete Schutzgruppen, wie z. B. Alkylether oder Silylether, sowie geeignete Verfahren zu ihrer selektiven Einführung und Abspaltung sind allgemein bekannt (vgl. z. B. T. W. Greene: Protective Groups in Organic Synthesis, Wiley & Sons, New York 1981).

Außer den in den Beispielen beschriebenen Verbindungen lassen sich nach dem erfidnungsgemäßen Verfahren die folgenden Verbindungen herstellen:

7-(4-(2,2-Dimethylethyl)-2-(4-fluorphenyl)-6-(1-methylethyl)phenyl)-3R, 5S-dihydroxyhept-6-insäure-tert.-butylester
7-(2,4-Bis-(4-fluorphenyl)-6-(1-methylethyl)phenyl)-3R, 5S-dihydroxyhept-6-insäure-tert.-butylester
7-(6-(2,2-Dimethylethyl)-4-(4-fluorphenyl)-2-(1-methylethyl)pyridin-3-yl)-3R, 5S-dihydroxyhept-6-insäure-tert.-butylester
7-(2,6-Bis-(1-methylethyl)-4-(4-fluorphenyl)pyridin-3-yl)-3R, 5S-dihydroxyhept-6-insäure-tert.-butylester
7-(2-Cyclopropyl-4-(4-fluorphenyl)-6-(1-methylethyl)pyridin-3-yl)-3R, 5S dihydroxyhept-6-insäure-tert.-butylester
7-(2-Cyclopropyl-6-(2,2-dimethylethyl)-4-(4-fluorphenyl) pyridin-3-yl)-3R, 5S dihydroxyhept-6-insäure-tert.-butyl-ester
7-(4,6-Bis-(4-fluorphenyl)-2-cyclopropylpyridin-3-yl)-3R, 5S-dihydroxyhept-6-insäure-tert.-butylester
7-(4-(4-Fluorphenyl)-6-(1-methylethyl)-3-phenylpyridazin-5-yl)-3R, 5S-dihydroxyhept-6-insäure tert.-butylester
7-(6-(2,2-Dimethylethyl)-4-(4-fluorphenyl)-2-(1-methylethyl)pyrimidin-3-yl)-3R, 5S-dihydroxyhept-6-insäure-tert.-butylester
7-(4,6-Bis-(4-fluorphenyl)-2-(1-methylethyl)pyrimidin-3-yl)-3R,5S-dihydroxyhept-6-insäure-tert.-butylester
7-(4-(2,2-Dimethyl)-2-(4-fluorphenyl)-6-(1-methylethyl)phenyl)-3R, 5S-dihydroxyhept-5-insäure Natriumsalz
7-(2,4-Bis-(4-fluorphenyl)-6-(2-methylethyl)phenyl)-3R, 5S-dihydroxyhept-6-insäure Natriumsalz
7-(6-(2,2-Dimethylethyl-4-(4-fluorphenyl)-2-(1-methylethyl)pyridin-3-yl)-3R, 5S-dihydroxyhept-6-insäure Natriumsalz
7-(2,6-Bis-(1-methylethyl)-4-(4-fluorphenyl)pyridin-3-yl)-3R, 5S-dihydroxyhept-6-insäure Natriumsalz
7-(2-Cyclopropyl-4-(4-fluorphenyl)-6-(1-methylethyl)pyridin-3-yl)-3R, 5S-dihydroxyhept-6-insäure Natriumsalz
7-(2-Cyclopropyl-6-(2,2-dimethylethyl)-4-(4-fluorphenyl)pyridin-3-yl)-3R, 5S-dihydroxyhept-6-insäure Natriumsalz
7-(4,6-Bis-(4-fluorphenyl)-2-cyclopropylpyridin-3-yl)-3R, 5S-dihydroxyhept-6-insäure Natriumsalz
7-(4-(4-Fluorphenyl)-6-(1-methylethyl)-3-phenylpyridazin-5-yl)-3R, 5S-dihydroxyhept-6-insäure Natriumsalz
7-(6-(2,2,-Dimethylethyl)-4-(4-fluorphenyl)-2-(1-methylethyl)pyrimidin-3-yl)-3R, 5S-dihydroxyhept-6-insäure Natriumsalz
7-(4,6-Bis-(4-fluorphenyl)-2-(1-methylethyl)pyrimidin-3-yl)-3R,5S-dihydroxyhept-6-insäure Natriumsalz
6S-(2-(4-(2,2-Dimethylethyl)-2-(4-fluorphenyl)6-(1-methylethyl)phenyl)ethinyl-4R-hydroxy-3,4,5,6-tetrahydro-2H-pyran-2-on
6S-(2-(2,4-Bis-(4-fluorphenyl)-6-(1-methylethyl)phenyl)ethinyl)-4R-hydroxy-3,4,5,6-tetrahydro-2H-pyran-2-on
6S-(2-(6-(2,2-Dimethylethyl)-4-(4-fluorphenyl)-2-(1-methylethyl)pyridin-3-yl)ethinyl)-4R-hydroxy-3,4,5,6-tetrahydro-2H-pyran-2-on
6S-(2-(2,6-Bis-(1-methylethyl)-4-(4-fluorphenyl)pyridin-3-yl)ethinyl)-4R-hydroxy-3,4,5,6-tetrahydro-2H-pyran-2-on
6S-(2-(2-Cyclopropyl-4-(4-fluorphenyl-6-(1-methylethyl)pyridin-3-yl)ethinyl)-4R-hydroxy-3,4,5,6-tetrahydro-2H-pyran-2-on
6S-(2-(2-Cyclopropyl-6-(2,2-dimethylethyl)-4-(4-fluorphenyl)pyridin-3-yl)ethinyl)-4R-hydroxy-3,4,5,6-tetrahydro-2H-pyran-2-on
6S-(2-(4,6-Bis-(4-fluorphenyl)-2-cyclopropylpyridin-3-yl) ethinyl)-4R-hydroxy-3,4,5,6-tetrahydro-2H-pyran-2-on
6S-(2-(4-fluorphenyl)-6-(1-methylethyl)-3-phenypyridazin-5-yl)ethinyl)-4R-hydroxy-3,4,5,6-tetrahydro-2H-pyran-2-on
6S-(2-(6-(2,2-Dimethylethyl)-4-(4-fluorphenyl)-2-(1-methylethyl)pyrimdin-3-yl)ethinyl)-4R-hydroxy-3,4,5,6-tetrahydro-2H-pyran-2-on
6S-(2-(4,6-Bis-(4-fluorphenyl)-2-(1-methylethyl)pyrimidin-3-yl)ethinly)-4R-hydroxy-3,4,5,6-tetrahydro-2H-pyran-2-on

**Biologische Testsysteme:**

1. HMG-CoA-Reduktase-Aktivität in Enzympräparationen
Die HMG-CoA-Reduktase-Aktivität wurde an solubilisierten Enzympräparationen aus Lebermikrosomen von Ratten gemessen, die nach Umstellung im Tag-Nach-Rhytmus mit Cholestyramin (®Cuemid) induziert wurden.
Als Substrat diente (Sm R) $^{14}$C-HMG-CoA, die Konzentration von NADPH wurde während der Inkubation durch ein regenerierendes System aufrechterhalten. Die Abtrennung von $^{14}$-C-Mevalonat vom Substrat und anderen Produkten (z. B. $^{14}$C-HMG) erfolgte über Säulenelution, wobei das Elutionsprofil jeder Einzelprobe ermittel wurde. Auf die ständige Mitführung von $^{3}$H-Mevalonat wurde verzichtet, weil es sich bei der Bestimmung um die Relativangabe der Hemmwirkung handelt. In einer Versuchsreihe wurden jeweils die enzymfreie Kontrolle, der enzymhaltige Normalansatz (= 100 %) und solche mit Präparatezusätzen, Endkonzentration $10^{-5}$ bis $10^{-9}$ M, zusammen behandelt. Jeder Einzelwert wurde als Mittelwert aus 3 Parallelproben gebildet. Die Signifikanz der Mittelwertunterschiede zwischen präparatefreien und präparatehaltigen Proben wurde nach dem t-Test beurteilt.

Nach der oben beschriebenen Methode wurden von den erfindungsgemäßen Verbindungen z. B. folgende Hemmwerte auf die HMG-CoA-Reduktase ermittelt [$IC_{50}$/mol/Liter bedeutet die molare Konzentraltion der Verbindung pro Liter, die für eine 50 %ige Hemmung erforderlich ist].

Die so für Verbindungen der allgemeinen Formel I erhaltenen $IC_{50}$ Werte wurden mit denjenigen verglichen, die für die Enzymhemmung der gleich substituierten 3,5-Dihydroxyhept-6-E-ensäurederivate (Ref. Verbindung) erhalten wurden ($IC_{50}$Ref.). Der Quotient $IC_{50}$Ref/$IC_{50}$ ergibt die relative Wirksamkeit.

Im einzelnen ergaben sich z. B. die in Tabelle 1 aufgeführten Werte

**Tabelle I**

| Verbindungen gemäß Beispiel | R | R* | $IC_{50}$/mol/L | Ref. Verbindung | rel. Wirksamkeit $IC_{50}$ Ref./$IC_{50}$ |
|---|---|---|---|---|---|
| 7 a | a; X=Y-Z:N = $CR^4CR^5$, $R^1$=$iC_3H_7$, $R^2$ = 4-$FC_6H_4$, $R^4$ = $C_6H_5$, $R_5$ = H | Na | $3.0 \times 10^{-9}$ | 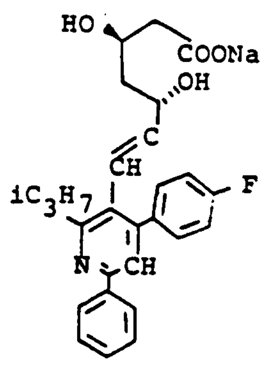 (Deutsche Offenlegungsschrift 38 23 045) | 0.97 |
| 7 1 | c, $R^{11}$=$iC_3H_7$, $R^{12}$= $R^{13}$=4-$FC_6H_4$ A-B = C=C | Na | $5.7 \times 10^{-9}$ | 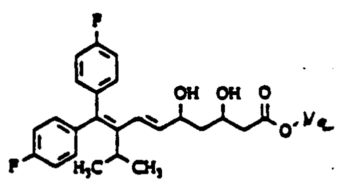 (Tetrahedron Lett. <u>29</u>, 929 (1988)) | 1.75 |

2. Supprimierung bzw. Inhibierung der HMG-CoA- Reduktase in Zellkulturen von HEP-G2-Zellen

Monolayers von HEP-G2-Zellen in lipoproteinfreiem Nährmedium wurden mit entsprechenden Konzentrationen der Prüfsubstanzen eine bestimmte Zeit (z. B. 1 Stunde) vorinkubiert, nach Zugabe des markierten Präkursors, z. B. $^{14}$C-Natriumacetat wurde die Inkubation fortgesetzt (z. B. 3 Stunden). Nach Zugabe eines internen Standards ($^3$H-Chloresterin) wurde ein Teil der Zellen alkalisch verseift. Die Lipide der verseiften Zellen wurden mit Chloroform/Methanol extrahiert. Dieses Lipidgemisch wurde nach Zusatz von Träger-Cholesterin präparativ dünnschichtchromatographische getrennt, die Cholesterinbande nach dem Sichtbarmachen mit Jod-Dampf isoliert und die aus dem $^{14}$C-Präkursor gebildetet Menge $^{14}$C-Choleterin szintigraphisch bestimmt. In einem aliquoten Teil der Zellen wurde Zellprotein bestimmt, so daß die in der Zeiteinheit pro mg Zellprotein gebildete Menge $^{14}$C-Cholesterin berechnet werden kann. Durch Vergleich dieses Wertes mit der Menge an $^{14}$C-Cholesterin, die pro mg Zellprotein und Zeiteinheit in einer gleichbehandelten jedoch prüfsubstanzfreien Kultur gebildet wurde, ergab sich die Hemmwirkung des jeweiligen Prüfpärparates auf die Cholesterin-Biosynthese von HEP-G2-Zellkulturen.

**Prüfung von Substanzen auf Hemmung der Cholesterin-Biosynthese in Zellkulturen konfluente Zellkultur (Monolayer) von HEP-G2-Zellen**

1. lipoproteinfreies Medium (DMEM)
2. Inkubation mit Prüfpräparaten
3. Inkubation mit $^{14}C$-Acetat
4. Cytolyse
5. DC-Trennung des Reaktionsprodukts $^{14}C$-Cholesterin
6. Isolierung des $^{14}C$-Cholesterin
7. Szintillationsmessung

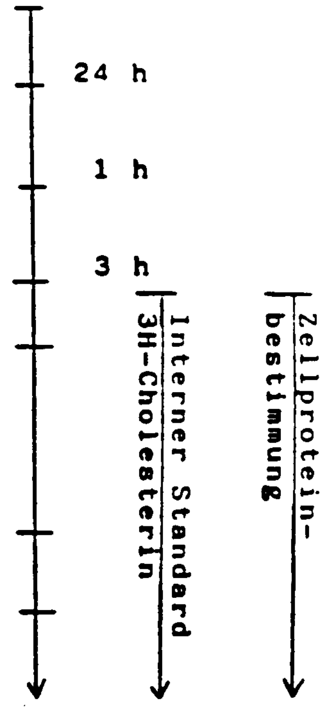


**8. Ergebnis**
**in nmol $^{14}C$-Cholesterin/mg Zellprotein im Vergleich zur Lösungsmittelkontrolle**

Nach der oben beschriebenen Methode wurden von den erfindungsgemäßen Verbindungen z. B. folgende Hemmwerte der Cholesterinbiosynthese (in HEP-G2-Zellen) ermittelt (der $IC_{50}$/mol ist diejenige Konzentration der Verbindung, die eine 50 %ige Hemmung der Cholesterinbiosynthese bewirkt). $IC_{50}$ Werte wurde ermittelt für die erfindungsgemäßen Verbindungen, sowie für gleich substituierte 3,5-Dihydroxyhept-6E-ensäurederivate ($IC_{50}$ Ref.). Der Quotient $IC_{50}$ Ref./$IC_{50}$ ergab die relative Wirksamkeit der erfindungsgemäßen Verbindungen. So wurde z. B. für die Verbindung gemäß Beispiel 8 a (R = a; X=Y-Z = N=$CR^4$-$CR^5$, $R^1$=$iC_3H_7$, $R^2$=4-$FC_6H_4$, $R^4$=$C_6H_5$, $R^5$=H) ein $IC_{50}$ von $9x10^{-9}$ mol/L bestimmt, sowie eine relative Wirksamkeit von 2,67, bezogen auf das analoge Hept-6E-ensäurederivat (vergleiche Tabelle I, Ref. Verb./deutsche Offenlegungsschrift 38 23 045, Beispiel 11 e). Verbindung gemäß Beispiel 8e (R = a; X=Y-Z=N=$CR^4$-N; $R^1$=$iC_3H_7$, $R^2$ = 4-$FC_6H_4$, $R^4$ = 4-$C_6H_4$) hemmt die Cholesterinbiosynthese mit einem $IC_{50}$ von $1,1x10^{-8}$ mol/l. Dies entspricht der 1,6-fachen relativen Wirksamkeit, bezogen auf das analoge Hept-6E-ensäurederivat.

Die Verbindungen der allgemeinen Formel I bzw. II zeichnen sich aus durch starke Hemmung der HMG-CoA-Reduktase, des geschwindigkeitsbestimmenden Enzyms der Cholesterinbiosynthese.

Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterinbiosynthese (vgl. J. R. Sabine in CRC Series in Enzyme Biology: 3-Hydroxy-3-methylglutaryl Coenzym A Reductase, CRC Press Inc., Boca Raten, Florida 1983 (ISBN 0-8493-6551-1)).

Hohe Cholesterinspiegel werden mit einer Reihe von Erkrankungen, wie z. B. koronarer Herzkrankheit oder Arteriosklerose in Verbindung gebracht. Daher ist die Senkung erhöhter Cholesterinspiegel zur Vorbeugung und Behandlung solcher Erkrankungen ein therapeutisches Ziel.

Ein Ansatzpunkt dazu liegt in der Hemmung bzw. Verminderung der endogenen Cholesterinbiosynthese. Hemmstoffe der HMG-CoA-Reduktase blockieren die Cholesterinbiosynthese auf einer frühen Stufe.

Die Verbindung der allgemeinen Formel I bzw. II eignen sich daher als Hypolipidemika und zur Behandlung bzw. Prophylaxe arteriosklerotischer Veränderungen.

Die Erfindung betrifft daher auch pharmazeutische Präparate auf Basis dieser Verbindung sowie ihre Verwendung als Arzneimittel, insbesondere als Hypolipidemika und zur Prophylaxe arteriosklerotischer Veränderungen.

Die Anwendung der Verbindungen der Formel I bzw. II als Hypolipidemika oder Antiarteriosklerotika erfolgt in oralen Dosen von 3 bis 2500 mg, vorzugsweise jedoch im Dosisbereich von 10 - 500 mg. Diese Tagesdosen können nach Bedarf auch in zwei bis vier Einzeldosen aufgeteilt werden oder in Retardform verabreicht werden. Das Dosierungsschema kann vom Typ, Alter, Gewicht, Geschlecht und medizinischen Zustand des Patienten abhängen.

Ein zusätzlicher cholesterinsenkender Effekt läßt sich durch gleichzeitige Gabe der erfindungsgemäßen Verbindungen mit gallensäurebindenen Stoffen, wie z. B. Anionenaustauscherharzen, erzielen. Die Gallensäureausscheidung führt zu einer verstärkten Neusynthese und damit zu einem erhöhten Cholesterinabbau (vgl. M. S. Brown, P. T. Koranen und J. C. Goldstein, Science 212, 628 (1981); M. S. Brown, J. C. Goldstein, Spektrum der Wissenschaft 1985, 1, 96).

Die erfindungsgemäßen Verbindungen der Formel I bzw. II können in Form der δ-Lactone, als freie Säuren, in Form ihrer physiologisch unbedenklichen anorganischen oder organischen Salze oder als Ester zur Anwendung kommen. Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z. B. Ethanol, Ethylenglykol oder Glycerin, in Triacetin, in Alkohol-Acetaldehyddiacetalgemischen, Ölen wie z. B. Sonnenblumenöl oder Lebertran, Ethern, wie z. B. Diethylenglykoldimethylether, oder auch Polyethern wie z. B. Polyethylenglykol, oder auch in Gegenwart anderer pharmakologisch unbedenklicher Polymerträger wie z. B. Polyvinylpyrrolidon oder in festen Zubereitungen zur Anwendung gelangen.

Für die Verbindungen der Formel I bzw. II sind feste, oral verabreichbare Zubereitungsformen bevorzugt, die die üblichen Hilfsstoffe enthalten können. Sie werden nach üblichen Methoden hergestellt.

Als Zubereitungen für die orale Anwendung eignen sich insbesondere Tabletten, Dragees oder Kapseln. Eine Dosierungseinheit enthält vorzugsweise 10 bis 500 mg Wirkstoff.

Die Verbindungen der Formeln III und IV sind neu und stellen wertvolle Zwischenprodukte für die Herstellung von Verbindungen der Formel I dar. Die Erfindung betrifft daher auch diese Verbindungen sowie Verfahren zu ihrer Herstellung.

Vorbemerkung: NMR-Spektren wurden, sofern nicht anderes angegeben, in $CDCl_3$ mit internem Standard TMS gemessen. Zur Klassifizierung von NMR-Signalen gelten folgende Abkürzungen: s = Singulett, d = Dublett, t = Triplett, q = Quartett, h = Heptett, m = Multiplett.
Schmelzpunikte sind unkorrigiert.
Es gelten folgende Substituentenabkürzungen:
i = iso, t = tertiär, c = cyclo.

**Beispiel 1**

Allgemeine Vorschrift zur Darstellung der Verbindungen der allgemeinen Formel VII

**Beispiel 1 a**

1,1-Dibrom-2-(4-(4-fluorphenyl)-2-(1-methylethyl)-6-phenyl pyridin-3-yl)ethen
(R = a; X = Y-Z = N = $C(C_6H_5)$-CH, $R^1 = iC_3H_7$, $R^2$ = 4-$FC_6H_4$)

Eine Lösung von 26,5 g (80 mmol) Tetrabromkohlenstoff in 500 ml Dichlormethan wurde mit 5,2 g (80 mmol) aktiviertem Zn-Staub und 21,0 g (80 mmol) Triphenylphosphin versetzt. Die Suspension wurde 30 h bei Raumtemperatur gerührt und anschließend mit einer Lösung von 12,8 g (40 mmol) 4-(4-Fluorphenyl)-2-(1-methylethyl)-6-phenylpyridin-3-aldehyd in 400 ml Dichlormethan versetzt. Das Reaktionsgemisch wurde 48 h zum Rückfluß erhitzt und filtriert. Die festen Bestandteile wurden mit 400 ml Dichlormethan gewaschen, die Filtrate vereinigt, mit Wasser ausgeschüttelt, über $MgSO_4$ getrocknet und eingedampft. Chromatographische Reinigung des Rückstandes lieferte 13,0 g (68 %) der Titelverbindung.

Fp: 116 - 118 °C

$^1$H-NMR: $\delta$ = 1,4(d, J = 7Hz, 6H), 3,2(h, J = 7Hz, 1H), 7,1(m, 2H), 7,3-7,5(m, 6H), 7,5(s, 1H), 8,1(m, 2H).

MS: m/e 478, 476, 474 ($M^+$ + H) $C_{22}H_{18}Br_2FN$

## Beispiel 1 b - 1 m

Im Analogie zu Beispiel 1a wurden die Beispiele der Tabelle 1 erhalten.

Tabelle 1

R = 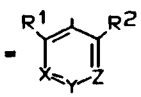   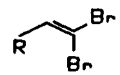

| Beispiel | X | Y | Z | $R^1$ | $R^2$ | Ausbeute % | MS : m/e = |
|---|---|---|---|---|---|---|---|
| 1b | N | 4-$FC_6H_4$-C | CH | i$C_3H_7$ | 4-$FC_6H_4$ | 79 | 495, 493, 491 ($M^+$) $C_{22}H_{17}Br_2F_2N$ |
| 1c | N | $C_6H_5$-C | CH | c$C_3H_7$ | 4-$FC_6H_4$ | 71 | 477, 475, 473 ($M^+$) $C_{22}H_{16}Br_2FN$ Fp. 153 - 155 °C |
| 1d | N | $C_6H_5$-C | N | i$C_3H_7$ | 4-$FC_6H_4$ | 46 | 478, 476, 474 ($M^+$) $C_{21}H_{17}Br_2FN_2$ |
| 1e | N | 4-$FC_6H_4$-C | N | i$C_3H_7$ | 4-$FC_6H_4$ | 86 | 496, 494, 492 ($M^+$) $C_{21}H_{16}Br_2F_2N_2$ |
| 1f | N | i$C_3H_7$-C | N | i$C_3H_7$ | 4-$FC_6H_4$ | 53 | 444, 442, 440 ($M^+$) $C_{18}H_{19}Br_2FN_2$ |
| 1g | CH | $C_6H_5$-C | CH | i$C_3H_7$ | 4-$FC_6H_4$ | 68 | 476, 474, 472 ($M^+$) $C_{23}H_{19}Br_2F$ |

Fortsetzung Tabelle 1

| Beispiel | X | Y | Z | $R^1$ | $R^2$ | Ausbeute % | MS : m/e = |
|---|---|---|---|---|---|---|---|
| 1h | CH | $iC_3H_7$-C | CH | $iC_3H_7$ | 4-$FC_6H_4$ | 76 | 442, 440, 438 ($M^+$) $C_{20}H_{21}Br_2F$ |
| 1i | N | N | 4-$FC_6H_4$-C | $iC_3H_7$ | 4-$FC_6H_4$ | 50 | 478, 476, 474 ($M^+$) $C_{21}H_{17}Br_2FN_2$ |

Fortsetzung Tabelle 1

R = 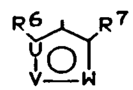

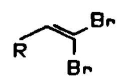

| Beispiel | U | V | W | $R^6$ | $R^7$ | Ausbeute % | MS : m/e = |
|---|---|---|---|---|---|---|---|
| 1j | C | $C_6H_5$-N | C-H | $iC_3H_7$ | 4-$FC_6H_4$ | 59 | 465, 463, 461 ($M^+$) $C_{21}H_{18}Br_2FN$ |
| 1k | C | $iC_3H_7$-N | C-H | $iC_3H_7$ | 4-$FC_6H_4$ | 63 | 431, 429, 427 ($M^+$) $C_{18}H_{20}Br_2FN$ |

**Fortsetzung Tabelle 1**

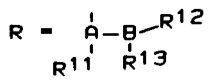

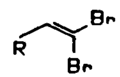

| Beispiel | A-B | $R^{11}$ | $R^{12}$ | $R^{13}$ | Ausbeute % | MS : m/e = |
|---|---|---|---|---|---|---|
| 11 | C=C | $iC_3H_7$ | $4-FC_6H_4$ | $4-FC_6H_4$ | 58 | 444, 442, 440 ($M^+$) $C_{19}H_{16}Br_2F_2$ |
| 1m | C=C | $iC_3H_7$ | 4-F-3-$CH_3C_6H_3$ | 4-F-3-$CH_3C_6H_3$ | 51 | 472, 470, 468 ($M^+$) $C_{21}H_{20}F_2Br_2$ |

**Beispiel 2**

Allgemeine Vorschrift zur Darstellung der Verbindungen der allgemeinen Formel IX

**Beispiel 2a**

3-(-4-(4-Fluorphenyl)-2-(1-methylethyl)-6-phenylpyridin-3-yl)prop-2-insäuremethylester
(R = a; X = Y-Z = N = C($C_6H_5$)-CH, $R^1$ = i$C_3H_7$, $R^2$ = 4F-$C_6H_4$, $R^{15}$ = $CH_3$)

A) 3-(4-(4-Fluorphenyl)-2-(1-methylethyl)-6-phenylpyridin-3-yl)prop-2-insäure
Zu einer Lösung von 11.5 g (24.2 mmol) 1,1-Dibrom-2-(4-(4-fluorphenyl)-2-(1-methylethyl)-6-phenylpyridin-3-yl)ethen (Beispiel 1a) in 70 ml THF wurden bei -70 °C 30.3 ml (48.4 mmol) einer Lösung von n-Butyllithium in Hexan 1.6 M getropft. Die resultierende Lösung wurde 1 h bei -70 °C, dann 1 h bei Raumtemperatur gerührt und anschließend auf -60 °C gekühlt. Nach Zugabe von 10.7 g (242 mmol) zerstoßenem Trockeneis wurde das Reaktionsgemisch langsam auf Raumtemperatur gebracht, auf Eiswasser hydrolysiert, mit verdünnter Salzsäure angesäuert und mehrfach mit Ether extrahiert. Die vereinigten organischen Phasen wurden mit ges. NaCl-Lösung gewaschen, über $MgSO_4$ getrocknet und eingedampft. Zurück blieben 11.0 g der Titelverbindung die ohne Reinigung weiter umgesetzt wurde. Für analytische Zwecke wurde eine kleine Menge des Rohproduktes auf Kieselgel (Dichlormethan/Methanol 9:1) gereinigt.

Fp: 152-153 °C
NMR: $\delta$ = 1.4 (m, 7H), 3.7 (h, J = 7Hz, 1H), 7.2 (m, 2H), 7.4-7.6 (m, 6H), 8.2 (m, 2H).
MS m/e = 360 ($M^+$ + H) $C_{23}H_{18}FNO_2$

B) 3-(4-(4-Fluorphenyl)-2-(1-methylethyl)-6-phenylpyridin-3-yl)prop-2-insäuremethylester
11.0 g der rohen 3-(4-(4-Fluorphenyl)-2-(1-methylethyl)-6-phenylpyridin-3-yl)prop-2-insäure wurden in 400 ml Diethylether gelöst und portionsweise mit etherischer Diazomethanlösung versetzt, bis die Umsetzung nach DC vollständig war. Nach Abdampfen des Solvens wurde der zurückbleibende rohe Ester säulenchromatographisch gereinigt (Silicagel; Cyclohexan/Dichlormethnan 1:1). Erhalten wurde die Titelverbindung in einer Ausbeute von 8.85 g, entsprechend 98 %, bezogen auf das eingesetzte Dibromid

FP: 118-120 °C
NMR: $\delta$ = 1.4 (d, J = 7Hz, 6H), 3.7 (h, J = 7Hz, 1H), 3.8 (s, 3H), 7.0-7.8 (m, 8H), 8.2 (m, 2H)
MS: m/e = 374 ($M^+$ + H) $C_{24}H_{20}FNO_2$

**Beispiel 2b-2m:**

In Analogie zu Beispiel 2a wurden die in Tablle 2 aufgeführten Verbindungen erhalten

**Tabelle 2**

R = 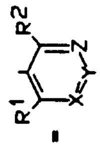

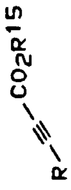

| Beispiel | X | Y | Z | $R^1$ | $R^2$ | $R^{15}$ | Ausbeute % | MS : m/e = |
|---|---|---|---|---|---|---|---|---|
| 2b | N | 4-$FC_6H_4$-C | CH | i$C_3H_7$ | 4-$FC_6H_4$ | $CH_3$ | 75 | 391 ($M^+$) $C_{24}H_{19}F_2NO_2$ |
| 2c | N | $C_6H_5$-C | CH | c$C_3H_7$ | 4-$FC_6H_4$ | $CH_3$ | 78 | 373 ($M^+$) $C_{24}H_{18}FNO_2$<br>Fp. 144 - 148 °C |
| 2d | N | $C_6H_5$-C | N | i$C_3H_7$ | 4-$FC_6H_4$ | $CH_3$ | 51 | 374 ($M^+$) $C_{23}H_{19}FN_2O_2$ |
| 2e | N | 4-$FC_6H_4$-C | N | i$C_3H_7$ | 4-$FC_6H_4$ | $CH_3$ | 70 | 392 ($M^+$) $C_{23}H_{18}F_2N_2O_2$;<br>Fp 141 °C |
| 2f | N | i$C_3H_7$-C | N | i$C_3H_7$ | 4-$FC_6H_4$ | $CH_3$ | 76 | 340 ($M^+$) $C_{20}H_{21}FN_2O_2$ |

Fortsetzung Tabelle 2

| Beispiel | X | Y | Z | $R^1$ | $R^2$ | $R^{15}$ | Ausbeute % | MS : m/e = |
|---|---|---|---|---|---|---|---|---|
| 2g | CH | $C_6H_5$-C | CH | $iC_3H_7$ | 4-$FC_6H_4$ | $CH_3$ | 83 | 372 ($M^+$) $C_{25}H_{21}FO_2$ |
| 2h | CH | $iC_3H_7$-C | CH | $iC_3H_7$ | 4-$FC_6H_4$ | $CH_3$ | 71 | 338 ($M^+$) $C_{22}H_{23}FO_2$ |
| 2i | N | N | 4-$FC_6H_4$-C | $iC_3H_7$ | 4-$FC_6H_4$ | $CH_3$ | 70 | 374 ($M^+$) $C_{23}H_{19}FN_2O_2$ |

Fortsetzung Tabelle 2

R = 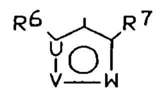

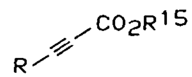

| Beispiel | U | V | W | $R^1$ | $R^2$ | $R^{15}$ | Ausbeute % | MS : m/e = |
|---|---|---|---|---|---|---|---|---|
| 2j | C | $C_6H_5$-N | C-H | $iC_3H_7$ | 4-$FC_6H_4$ | $CH_3$ | 71 | 361 ($M^+$) $C_{23}H_{20}FNO_2$ |
| 2k | C | $iC_3H_7$-N | C-H | $iC_3H_7$ | 4-$FC_6H_4$ | $CH_3$ | 70 | 327 ($M^+$) $C_{20}H_{22}FNO_2$ |

Fortsetzung Tabelle 2

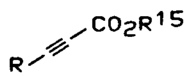

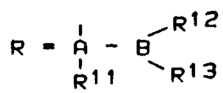

| Beispiel | A-B | $R^{11}$ | $R^{12}$ | $R^{13}$ | $R^{15}$ | Ausbeute % | MS : m/e = |
|---|---|---|---|---|---|---|---|
| 21 | C=C | $iC_3H_7$ | $4-FC_6H_4$ | $4-FC_6H_4$ | $CH_3$ | 94 | 340 ($M^+$) $C_{21}H_{18}F_2O_2$ |
| 2m | C=C | $iC_3H_7$ | 4-F-3-$CH_3C_6H_3$ | 4-F-3-$CH_3C_6H_3$ | $CH_3$ | 76 | 368 ($M^+$) $C_{23}H_{22}F_2O_2$ |

**Beispiel 2**

Allgemeine Vorschrift zur Darstellung der Verbindungen der allgemeinen Formel X

**Beispiel 2a**

3(-4-(4-Fluorphenyl)-2-(1-methylethyl)-6-phenylpyridin-3-yl)prop-2-in-1-ol
(R = a; X = Y-Z = N = $C(C_6H_5)$-CH, $R^1 = iC_3H_7$, $R^2$ = 4-$FC_6H_4$)

Eine Lösung von 8.80 g (23.6 mmol) 3-(4-(4-Fluorphenyl)-2-(1-methylethyl)-6-phenylpyridin-3-yl)prop-2-insäuremethylester (Beispiel 2a) in 100 ml Toluol wurde bei 0 °C tropfenweise mit 49 ml (58.6 mmol) einer Lösung von DIBAH in Toluol, 1.2 M versetzt und bei 0-20 °C unter DC-Kontrolle bis zur vollständigen Umsetzung gerührt (1.5 h). Nach Zusatz von 100 ml Ethylacetat wurde weitere 30 min gerührt, dann wurde das Gemisch auf 100 ml ges. NaCl-Lösung gegeben, bis zur Auflösung der Aluminiumsalze mit verdünnter Salzsäure angesäuert und mehrfach mit Ethylacetat ausgeschüttelt. Die vereinigten organischen Extrakte wurden mit ges. $NaHCO_3$-Lösung und Wasser gewaschen, über $MgSO_4$ getrocknet und eingedampft. Der Rückstand wurde chromatographisch gereinigt (Silicagel; Cyclohexan/Dichlormethan 1:1). Erhalten wurden 7.14 g (88 %) der Titelverbindung.

FP: 119-121 °C

NMR: $\delta$ = 1.4 (d, J = 7Hz, 6H), 1.6 (s, 1H), 3.6 (h, J = 7 Hz, 1H), 4.5 (s, 2H), 7.0-7.7 (m, 8H), 8.2 (m, 2H).

MS: m/e = 346 ($M^+$ + H) $C_{23}H_{20}FNO$

**Beispiel 3b-3m**

In Analogie zu Beispiel 3a wurden die in Tabelle 3 aufgeführten Verbindungen erhalten.

Tabelle 3

R = 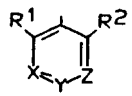 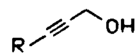

| Beispiel | X | Y | Z | $R^1$ | $R^2$ | Ausbeute % | MS : m/e = |
|---|---|---|---|---|---|---|---|
| 3b | N | 4-$FC_6H_4$-C | CH | $iC_3H_7$ | 4-$FC_6H_4$ | 94 | 363 ($M^+$) $C_{23}H_{19}F_2NO$ |
| 3c | N | $C_6H_5$-C | CH | $cC_3H_7$ | 4-$FC_6H_4$ | 97 | 345 ($M^+$) $C_{23}H_{18}FNO$; Fp. 105-109°C |
| 3d | N | $C_6H_5$-C | N | $iC_3H_7$ | 4-$FC_6H_4$ | 93 | 346 ($M^+$) $C_{22}H_{19}FN_2O$; Fp 163 °C |
| 3e | N | 4-$FC_6H_4$-C | N | $iC_3H_7$ | 4-$FC_6H_4$ | 98 | 364 ($M^+$) $C_{22}H_{18}F_2N_2O_2$; Fp 142°C |
| 3f | N | $iC_3H_7$-C | N | $iC_3H_7$ | 4-$FC_6H_4$ | 91 | 312 ($M^+$) $C_{19}H_{21}FN_2O$ |
| 3g | CH | $C_6H_5$-C | CH | $iC_3H_7$ | 4-$FC_6H_4$ | 95 | 344 ($M^+$) $C_{24}H_{21}FO$ |

Fortsetzung Tabelle 3

| Beispiel | X | Y | Z | $R^1$ | $R^2$ | Ausbeute % | MS : m/e = |
|---|---|---|---|---|---|---|---|
| 3h | CH | $iC_3H_7$-C | CH | $iC_3H_7$ | 4-$FC_6H_4$ | 95 | 310 ($M^+$) $C_{21}H_{23}FO$ |
| 3i | N | N | 4-$FC_6H_4$-C | $iC_3H_7$ | 4-$FC_6H_4$ | 86 | 346 ($M^+$) $C_{22}H_{19}FN_2O$ |

Fortsetzung Tabelle 3

R = 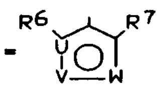

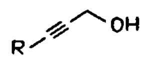

| Beispiel | U | V | W | $R^6$ | $R^7$ | Ausbeute % | MS : m/e = |
|---|---|---|---|---|---|---|---|
| 3j | C | $C_6H_5$-N | C-H | i$C_3H_7$ | 4-$FC_6H_4$ | 96 | 333 ($M^+$) $C_{22}H_{20}FNO$ |
| 3k | C | i$C_3H_7$-N | C-H | i$C_3H_7$ | 4-$FC_6H_4$ | 90 | 299 ($M^+$) $C_{19}H_{22}FNO$ |

Fortsetzung Tabelle 3

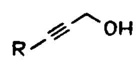

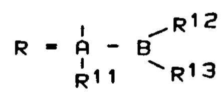

| Beispiel | A-B | $R^{11}$ | $R^{12}$ | $R^{13}$ | Ausbeute % | MS : m/e = |
|---|---|---|---|---|---|---|
| 31 | C=C | $iC_3H_7$ | $4-FC_6H_4$ | $4-FC_6H_4$ | 74 | 312 ($M^+$) $C_{22}H_{16}F_2O$ |
| 3m | C=C | $iC_3H_7$ | 4-F-<br>$3-CH_3C_6H_3$ | 4-F-<br>$3-CH_3C_6H_3$ | 81 | 340 ($M^+$) $C_{22}H_{22}F_2O$ |

**Beispiel 4**

Allgemeine Vorschrift zur Darstellung der Verbindungen der allgemeinen Formel III

**Beispiel 4 a**

3-(4-(4-Fluorphenyl)-2-(1-methylethyl)-6-phenylpyridin-3-yl)prop-2-inal
(R = a; X = Y-Z = N = $C(C_6H_5)$-CH, $R^1 = iC_3H_7$, $R^2$ = 4-$FC_6H_4$)

Eine Lösung von 7,14 g (20,7 mmol) 3-(4-(4-Fluorphenyl)-2-(1-methylethyl)-6-phenylpyridin-3-yl)prop-2-in-1-ol (Beispiel 3 a) und 6,68 g (31,0 mmol) Pyridiniumchlorochromat in 150 ml Dichlormethan wurde bei Raumtemperatur gerührt. Das Fortschreiten der Umsetzung wurde per DC kontrolliert. Nach 3 h war alles Ausgangsmaterial umgesetzt. Die Reaktionslöung wurde durch eine Silicagelschicht filtriert und eingedampft. Säulenchromatographie des Rückstands (Silicagel; Cyclohexan/Ethylacetat 20 : 1) lieferte 6,4 g (90 %) der Titelverbindung.

NMR: $\delta$ = 1,4(d, J = 7Hz, 6H), 3,8(h, J = 7Hz, 1H), 7,2(m, 2H), 7,4-7,6(m, 6H), 8,2(m, 2H), 9,7(s, 1H).
MS: m/e = 344 ($M^+$ + H) $C_{23}H_{18}FNO$

**Beispiel 4 b - 4 m**

In Analogie zu Beispiel 4 a wurden die in Tabelle 4 auf geführten Verbindungen erhalten.

**Tabelle 4**

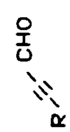

R =

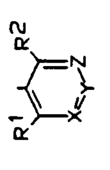

| Beispiel | X | Y | Z | $R^1$ | $R^2$ | Ausbeute % | MS : m/e = |
|---|---|---|---|---|---|---|---|
| 4b | N | $4-FC_6H_4-C$ | CH | $iC_3H_7$ | $4-FC_6H_4$ | 85 | 361 ($M^+$) $C_{23}H_{17}F_2NO$ |
| 4c | N | $C_6H_5-C$ | CH | $cC_3H_7$ | $4-FC_6H_4$ | 82 | 343 ($M^+$) $C_{23}H_{16}FNO$ |
| 4d | N | $C_6H_5-C$ | N | $iC_3H_7$ | $4-FC_6H_4$ | 65 | 344 ($M^+$) $C_{22}H_{17}FN_2O$ |
| 4e | N | $4-FC_6H_4-C$ | N | $iC_3H_7$ | $4-FC_6H_4$ | 70 | 362 ($M^+$) $C_{22}H_{16}F_2N_2O$ |
| 4f | N | $iC_3H_7-C$ | N | $iC_3H_7$ | $4-FC_6H_4$ | 75 | 310 ($M^+$) $C_{19}H_{19}FN_2O$ |

Fortsetzung Tabelle 4

| Beispiel | X | Y | Z | $R^1$ | $R^2$ | Ausbeute % | MS : m/e = |
|---|---|---|---|---|---|---|---|
| 4g | CH | $C_6H_5$-C | CH | i$C_3H_7$ | 4-$FC_6H_4$ | 89 | 342 ($M^+$) $C_{24}H_{19}FO$ |
| 4h | CH | i$C_3H_7$-C | CH | i$C_3H_7$ | 4-$FC_6H_4$ | 95 | 308 ($M^+$) $C_{21}H_{21}FO$ |
| 4i | N | N | 4-$FC_6H_4$-C | i$C_3H_7$ | 4-$FC_6H_4$ | 60 | 344 ($M^+$) $C_{22}H_{17}FN_2O$ |

Fortsetzung Tabelle 4

R = 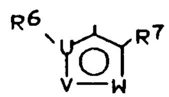 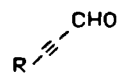

| Beispiel | U | V | W | $R^6$ | $R^7$ | Ausbeute % | MS : m/e = |
|---|---|---|---|---|---|---|---|
| 4j | C | $C_6H_5$-N | C-H | $iC_3H_7$ | 4-$FC_6H_4$ | 88 | 331 ($M^+$) $C_{22}H_{18}NO$ |
| 4k | C | $iC_3H_7$-N | C-H | $iC_3H_7$ | 4-$FC_6H_4$ | 77 | 297 ($M^+$) $C_{19}H_{20}FNO$ |

Fortsetzung Tabelle 4

R = 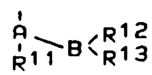

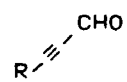

| Beispiel | A-B | $R^{11}$ | $R^{12}$ | $R^{13}$ | Ausbeute % | MS : m/e = |
|---|---|---|---|---|---|---|
| 4l | C=C | $iC_3H_7$ | 4-$FC_6H_4$ | 4-$FC_6H_4$ | 70 | 318 ($M^+$) $C_{20}H_{16}F_2O$ |
| 4m | C=C | $iC_3H_7$ | 4-F-3-$CH_3C_6H_3$ | 4-F-3-$CH_3C_6H_3$ | 73 | 338 ($M^+$) $C_{22}H_{20}F_2O$ |

**Beispiel 5**

Allgemeine Vorschrift zur Darstellung von Verbindungen der allgemeinen Formel IV

**Beispiel 5 a**

7-(4-(4-Fluorphenyl)-2-(1-methylethyl)-6-phenylpyridin-3-yl)-5SR-hydroxy-3-oxo-hept-6-insäuremethylester (R = a; X = Y-Z = N = C($C_6H_5$)-CH, $R^1$ = i$C_3H_7$, $R^2$ = 4-F$C_6H_4$, R° = $CH_3$)

Eine Lösung von 4.84 g (47.9 mmol) Diisopropylamin in 50 ml THF wurde bei 0 °C tropfenweise mit 26 ml (42 mmol) n-Butyllithium in Hexan 1.6 M versetzt. Nach 30 minütigem Nachrühren wurde die Lösung auf -78 °C gekühlt, und 1.57 g (13.6 mmol) Acetessigsäuremethylester wurden zugetropft. Nach Ende der Zugabe wurde die resultierende Lösung 15 min bei 0 °C gerührt und erneut auf -78 °C gebracht. Anschließend wurde eine Lösung von 1.3 g (9.0 mmol)
3-(4-(4-Fluorphenyl)-2-(1-methylethyl)-6-phenyl-pyridin-3-yl) prop-2-inal (Beispiel 4 a) in 30 ml THF zugetropft. Das Reaktionsgemisch wurde 2 h bei -78 °C gerührt, mit 1 M Salzsäure angesäuert und mehrfach mit Ether extrahiert. Die vereinigten organischen Phasen wurden mit $NaHCO_3$-Lösung und Wasser gewaschen, über Magnesiumsulfat getrocknet und eingedampft. Eine chromatographische Reinigung des Rückstands lieferte 2.09 g (50 %) der Titelverbindung.

NMR: $\delta$ = 1,4(d, J = 7Hz, 6H), 2,8(d, J = 6Hz, 1H), 2,9(dd, J = 18Hz, 4Hz, 1H), 3,0(dd, J = 18Hz, 6Hz, 1H), 3,5(s, 2H), 3,7(h, J = 7Hz, 1H), 3,8(s, 3H), 5,0(m, 1H), 7,2(m, 2H), 7,4-7,7(m, 6H), 8,1(m, 2H).

MS: m/e = 460 ($M^+$ + H) $C_{28}H_{26}FNO_4$

**Beispiel 5 b - 5 m**

Im Analogie zu Beispiel 5 a wurden die in Tabelle 5 aufgeführten Verbindungen erhalten

Tabelle 5

R = 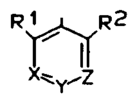 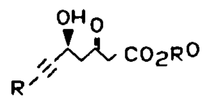

| Beispiel | X | Y | Z | $R^1$ | $R^2$ | $R^0$ | Ausbeute % | MS : m/e = |
|---|---|---|---|---|---|---|---|---|
| 5b | N | 4-$FC_6H_4$-C | CH | i$C_3H_7$ | 4-$FC_6H_4$ | $CH_3$ | 64 | 477 ($M^+$) $C_{28}H_{25}F_2NO_4$ |
| 5c | N | $C_6H_5$-C | CH | c$C_3H_7$ | 4-$FC_6H_4$ | $CH_3$ | 67 | 459 ($M^+$) $C_{28}H_{24}FNO_4$ |
| 5d | N | $C_6H_5$-C | N | i$C_3H_7$ | 4-$FC_6H_4$ | $CH_3$ | 51 | 460 ($M^+$) $C_{27}H_{25}FN_2O_4$ |
| 5e | N | 4-$FC_6H_4$-C | N | i$C_3H_7$ | 4-$FC_6H_4$ | $CH_3$ | 59 | 478 ($M^+$) $C_{27}H_{24}F_2N_2O_4$ |
| 5f | N | i$C_3H_7$-C | N | i$C_3H_7$ | 4-$FC_6H_4$ | $CH_3$ | 68 | 426 ($M^+$) $C_{24}H_{27}FN_2O_4$ |

Fortsetzung Tabelle 5

| Beispiel | X | Y | Z | $R^1$ | $R^2$ | $R^0$ | Ausbeute % | MS : m/e = |
|---|---|---|---|---|---|---|---|---|
| 5g | CH | $C_6H_4$-C | CH | $iC_3H_7$ | 4-$FC_6H_4$ | $CH_3$ | 63 | 458 ($M^+$) $C_{29}H_{27}F_2O_4$ |
| 5h | CH | $iC_3H_7$-C | CH | $iC_3H_7$ | 4-$FC_6H_4$ | $CH_3$ | 60 | 424 ($M^+$) $C_{26}H_{29}FO_4$ |
| 5i | N | N | 4-$FC_6H_4$-C | $iC_3H_7$ | 4-$FC_6H_4$ | $CH_3$ | 48 | 460 ($M^+$) $C_{27}H_{25}FN_2O_4$ |

Fortsetzung Tabelle 5

R = 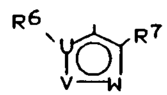 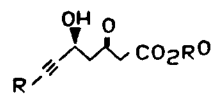

| Bei-spiel | U | V | W | $R^6$ | $R^7$ | $R^0$ | Ausbeute % | MS : m/e = |
|---|---|---|---|---|---|---|---|---|
| 5j | C | $C_6H_5$-N | C-H | $iC_3H_7$ | 4-$FC_6H_4$ | $CH_3$ | 55 | 447 ($M^+$) $C_{27}H_{26}FNO_4$ |
| 5k | C | $iC_3H_7$-N | C-H | $iC_3H_7$ | 4-$FC_6H_4$ | $CH_3$ | 63 | 413 ($M^+$) $C_{24}H_{28}FNO_4$ |

Fortsetzung Tabelle 5

R = 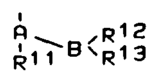

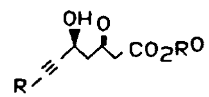

| Beispiel | A-B | $R^{11}$ | $R^{12}$ | $R^{13}$ | $R^{0}$ | Ausbeute % | MS : m/e = |
|---|---|---|---|---|---|---|---|
| 51 | C=C | $iC_3H_7$ | $4-FC_6H_4$ | $4-FC_6H_4$ | $CH_3$ | 75 | 426 ($M^+$) $C_{25}H_{24}F_2O_4$ |
| 5m | C=C | $iC_3H_7$ | $4-F-3-CH_3C_6H_3$ | $4-F-3-CH_3C_6H_3$ | $CH_3$ | 61 | 454 ($M^+$) $C_{27}H_{28}F_2O_4$ |

**Beispiel 6**

Allgemeine Vorschrift zur Darstellung von Verbindungen der allgemeinen Formel I

**Beispiel 6 a**

3RS, 5SR-Dihydroxy-7-(4-(4-fluorphenyl)-2-(1-methyl-ethyl-6-phenylpyridin-3-yl)hept-6-insäuremethylester
(R = a; X = Y-Z = N = C($C_6H_5$)-CH, $R^1$ = i$C_3H_7$, $R^2$ = 4-F$C_6H_4$, $R^0$ = $CH_3$)

6.75 ml einer 1M-Lösung von Triethylboran in THF wurden mit 40 ml THF verdünnt und bei 0 ° C mit 10 ml Methanol tropfenweise versetzt. Die resultierende Lösung wurde 1 h bei 0 ° C gerührt und auf -78 ° C gekühlt. Nach tropfenweiser Zugabe einer Lösung von 2.07 g (4.5 mmol) des Ketoesters (aus Beispiel 5a) in wenig THF wurde 30 min bei 78 ° C gerührt. Anschließend wurden 340 mg (9.0 mmol ) Natriumborhydrid zugegeben und das resultierende Gemisch 3 h bei -78 ° C weitergerührt. Zur Aufarbeitung wurde das Reaktionsgemisch mit Ethylacetat verdünnt, auf ges. Ammoniumchloridlösung gegeben und mehrfach mit Ethylacetat extrahiert. Die organischen Phasen wurden getrocknet ($MgSO_4$) und eingeengt, der Rückstand wurde zur Entfernung von Borsäureestern 3 x jeweils in Methanol aufgenommen und eingedampft. Das zurückbleibende Rohprodukt wurde durch Säulenchromatographie (Cyclohexan/Ethylacetat 4 : 1) gereinigt. Die Ausbeute an reiner Titelverbindung betrug 1.45 g (70 %).

Fp: 134 ° C
NMR: $\delta$ = 1.4(d, J = 7Hz, 6H), 1.8(m, 1H), 2.0(m, 1H), 2.4-2.6 (m, 3H), 2.7(d, J = 3Hz, 1H), 3.4(d, J = 2Hz, 1H), 3.4(s, 3H), 4.2(m, 1H), 5.3(m, 1H), 7.2(m, 2H), 7.4-7-6(m, 6H), 8.1(m, 2H)
MS: m/e = 462 ($M^+$ + H) $C_{28}H_{28}FNO_4$

**Beispiel 6 b - 6 m**

Im Analogie zu Beispiel 6 a wurden die in Tabelle 6 aufgeführten Verbindungen erhalten.

Tabelle 6

R = 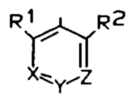

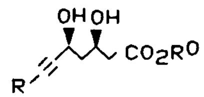

| Beispiel | X | Y | Z | $R^1$ | $R^2$ | $R^0$ | Ausbeute % | 1H-NMR: δ/ppm<br>MS : m/e = |
|---|---|---|---|---|---|---|---|---|
| 6b | N | 4-$FC_6H_4$-C | CH | i$C_3H_7$ | 4-$FC_6H_4$ | $CH_3$ | 77 | 1.4(d,J=7Hz,6H), 1.8(m,1H), 2.0(m,1H), 2.5(m,2H),2.7(s,1H), 3.4(s,1H),3.8(s,3H),4.1(h,J=7Hz,1H), 4.2(m,1H),4.8(m,1H),7.1-8.1(m,9H).<br>479 ($M^+$) $C_{28}H_{27}F_2NO_4$ |
| 6c | N | $C_6H_5$-C | CH | c$C_3H_7$ | 4-$FC_6H_4$ | $CH_3$ | 80 | 1.1(m,2H),1.3(m,2H),1.8(m,1H) 2.0 (m,1H), 2.5-2.7(m,2H), 2.8(d,J= 4Hz,1H), 3.4(d,J=3Hz,1H), 3.7(s,3H),4.2(m,1H),4.9(m,1H), 7.1(m,2H),7.3-7.6(m,6H),8.0(m,2H).<br>461 ($M^+$) $C_{28}H_{26}FNO_4$; Fp. 96-100°C |
| 6d | N | $C_6H_5$-C | N | i$C_3H_7$ | 4-$FC_6H_4$ | $CH_3$ | 74 | 1.5(d,J=Hz,6H),1.8(m,1H),2.0(m,1H) 2.6(m,2H),2.7(s,1H),3.4 (s,1H),3.7 (s,3H) ,4.0(h,J=7Hz,1H), |

Fortsetzung Tabelle 6

| Beispiel | X | Y | Z | $R^1$ | $R^2$ | $R^0$ | Ausbeute % | 1H-NMR: δ/ppm<br>MS : m/e = |
|---|---|---|---|---|---|---|---|---|
| | | | | | | | | 4.2(m,1H),4.8(m,1H),7.2(m,2H), 7.4-7.8(m,5H),8.6(m,2H).<br>462 ($M^+$) $C_{27}H_{27}FN_2O_4$ |
| 6e | N | 4-$FC_6H_4$-C | N | i$C_3H_7$ | 4-$FC_6H_4$ | CH3 | 66 | 1.4(d,J=7Hz,6H),1.8(m,1H),2.0(m,1H) 2.5(m,2H),3.0(s,1H),3.5(s,1H),3.7(s, 3H),3.7(h,J=7Hz,1H),4.2(m,1H),4.9 (m,1H),7.1(m,4H),8.1(m,2H) 8.6(m,2H).<br>480 ($M^+$) $C_{27}H_{26}F_2N_2O_4$; Fp. 73-77 °C |
| 6f | N | i$C_3H_7$-C | N | i$C_3H_7$ | 4-$FC_6H_4$ | $CH_3$ | 61 | 1.3(d,J=7Hz,6H),1.5(d,7Hz,6H),1.8 (m,1H),2.0(m,1H),2.6(m,2H),2.7(s,1H), 3.4(s,1H),3.5(h,J=7Hz,1H),3.7 (s,3H) ,4.0(h,J=7Hz,1H),4.2(m,1H), 4.8(m,1H),7.1(m,2H), 7.6(m,2H).<br>428 ($M^+$) $C_{24}H_{29}FN_2O_4$ |

Fortsetzung Tabelle 6

| Beispiel | X | Y | Z | $R^1$ | $R^2$ | $R^0$ | Ausbeute % | 1H-NMR: δ/ppm<br>MS : m/e = |
|---|---|---|---|---|---|---|---|---|
| 6g | CH | $C_6H_5$-C | CH | i$C_3H_7$ | 4-F$C_6H_4$ | $CH_3$ | 79 | 1.3(d,J=7Hz,6H),1.8(m,1H),2.0(m,1H) 2.5(m,2H),2.7(s,1H),3.4(s,1H),3.7 (s,3H),3.9(h,J=7Hz,1H),4.2(m,1H),4.8 (m,1H)7.1(m,2H),7.4-7.7(m,9H).<br>460 ($M^+$) $C_{29}H_{29}FO_4$ |
| 6h | CH | i$C_3H_7$-C | CH | i$C_3H_7$ | 4-F$C_6H_4$ | $CH_3$ | 72 | 1.1-1.2(m,12H),1.8(m,1H),2.0(m,1H), 2.5(m,2H),2.7(s,1H),3.4(m,2H),3.7(s,3H) 3.9(h,J=7Hz,1H),4.2(m,1H),4.8(m,1H), 7.0-7.4(m,6H).<br>426 ($M^+$) $C_{26}H_{31}FO_4$ |
| 6i | N | N | 4-F$C_6H_4$-C | i$C_3H_7$ | 4-FC $_6H_4$ | $CH_3$ | 53 | 1.5(d,J=7Hz,6H),1.8(m,1H),2.0(m,1H), 2,5(m,2H),2.7(s,1H),3.4(s,1H),3.7(s,3H) 3.8(h,J=7Hz,1H),4.3(m,1H),4.8(m,1H), 6.9-7.5(m,8H).<br>462($M^+$) $C_{27}H_{26}FN_2O_4$ |

Fortsetzung Tabelle 6

R = 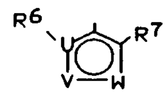

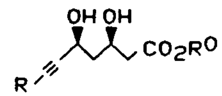

| Beispiel | U | V | W | $R^6$ | $R^7$ | $R^0$ | Ausbeute % | 1H-NMR: δ/ppm<br>MS : m/e = |
|---|---|---|---|---|---|---|---|---|
| 6j | C | $C_6H_5$-N | C-H | i$C_3H_7$ | 4-F$C_6H_4$ | $CH_3$ | 73 | 1.3(d,J=7Hz,6H),1.8(m,1H) 2.0(m,1H),2.5(m,2H),2.7(s, 1H),3.4(s,1H),3.6(h,J=7Hz, 1H),3.7(s,3H),4.2(m,1H), 4.8(m,1H),6.6(s,1H),6.8-7.2(m,7H),7.5(m,2H).<br>449 ($M^+$) $C_{27}H_{28}FNO_4$ |
| 6k | C | i$C_3H_7$-N | C-H | i$C_3H_7$ | 4-F$C_6H_4$ | $CH_3$ | 65 | 1.4(d,J=7Hz,6H),1.5(d,J=7Hz 6H),1.8(m,1H),2.0(m,1H), 2.5(m,2H),2.8(s,1H),3.5(h, J=7Hz,1H),3.7(s,3H), 4.2(m,1H),4.5(h,J=7Hz, 1H),4.8(m,1H),6.6(s,1H),7.0 (m,2H),7.4(m,2H).<br>415 ($M^+$) $C_{24}H_{30}FNO_4$ |

Fortsetzung Tabelle 6

R = 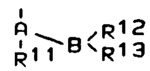

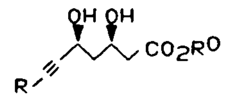

| Beispiel | A-B | $R^{11}$ | $R^{12}$ | $R^{13}$ | $R^0$ | Ausbeute % | 1H-NMR: δ/ppm: MS : m/e = |
|---|---|---|---|---|---|---|---|
| 61 | C=C | $iC_3H_7$ | $4-FC_6H_4$ | $4-FC_6H_4$ | $CH_3$ | 67 | 1.1(d,J=7Hz,6H),1.7 (m,1H),1.9(m,1H),2.4-2.5(m,2H),2.6(d,J=3Hz,1H),2.7(h,J=7Hz,1H),3.4(d,J=3Hz,1H),3.7 (s,3H),4.2(m,1H),4.7 (m,1H),6.9-7.1(m,6H),7.3(m,2H). 428 ($M^+$) $C_{25}H_{26}F_2O_4$ |
| 6m | C=C | $iC_3H_7$ | $4-F-3-CH_3C_6H_3$ | $4-F-3-CH_3C_6H_3$ | $CH_3$ | 58 | 1.1(d,J=7Hz,6H),1.7 (m,1H),1.9(m,1H)2.2-2.5(m,8H),2.6(d,J=4Hz 1H),2.7(h,J=7Hz,1H) 3.5(d,J=4Hz,1H),3.7 (s,3H),4.2(m,1H),4.7 (m,1H),6.9-7.1(m,6H). 456 ($M^+$) $C_{27}H_{30}F_2O_4$ |

**Beispiel 7**

Allgemeine Vorschrift zur Darstellung der Verbindungen der allgemeinen Formel I

**Beispiel 7 a**

3RS, 5SR-Dihydroxy-7-(4-(4-fluorphenyl)-2-(1-methylethyl)-6-phenylpyridin-3-yl)hept-6-insäure Natriumsalz (R = a; X = Y-Z = N = $C(C_6H_5)$-CH, $R^1 = iC_3H_7$, $R^2$ = 4-$FC_6H_4$, $R^0$ = Na)

1.40 g (3.02 mmol) 3RS, 5-SR-Dihydroxy-7-(4-(4-fluorphenyl)-2-(1-methylethyl)-6-phenylpyridin-3yl)hept-6-in-säuremethylester (Beispiel 6 a) wurden in 40 ml abs. Ethanol gelöst und mit 3 ml (30 mmol) 1 M Natronlauge versetzt. Die Reaktionslösung wurde unter DC-Kontrolle bei Raumtemperatur gerührt. Nach 2 h war kein Ausgangsmaterial mehr nachzuweisen. Das Lösungsmittel wurde abgedampft, der feste Rückstand mit Toluol versetzt und erneut eingedampft. Nach Trocknung im Hochvakuum blieben 1.47 g (100 %) der Titelverbindung in Form weißer Kristalle zurück.

$^1$H-NMR($D_2O$): 1.1(d, J = 7Hz, 6H), 1.6(m, 1H), 1.8(m, 1H), 2.1(dd, J = 15Hz, 9Hz, 1H), 2.3(dd, J = 15Hz, 3Hz, 1H), 3.4(h, J = 7Hz, 1H), 3.9(m, 1H), 4.4(dd, J = 6Hz, 6Hz, 1H), 6.5(s, 1H), 6.7-6.9(m, 7H), 7.3(m, 2H).

**Beispiel 7 b - 7 m**

In Analogie zu Beispiel 7 a wurden die in Tabelle 7 aufgeführten Verbindungen erhalten

Fortsetzung Tabelle 7

R = 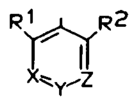 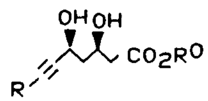

| Beispiel | X | Y | Z | $R^1$ | $R^2$ | $R^0$ | Ausbeute % |
|---|---|---|---|---|---|---|---|
| 7b | N | $4\text{-}FC_6H_4\text{-}C$ | CH | $iC_3H_7$ | $4\text{-}FC_6H_4$ | Na | 98 |
| 7c | N | $C_6H_5\text{-}C$ | CH | $cC_3H_7$ | $4\text{-}FC_6H_4$ | Na | 95 |
| 7d | N | $C_6H_5\text{-}C$ | N | $iC_3H_7$ | $4\text{-}FC_6H_4$ | Na | 99 |
| 7e | N | $4\text{-}FC_6H_4\text{-}C$ | N | $iC_3H_7$ | $4\text{-}FC_6H_4$ | Na | 99 |
| 7f | N | $iC_3H_7\text{-}C$ | N | $iC_3H_7$ | $4\text{-}FC_6H_4$ | Na | 91 |

Fortsetzung Tabelle 7

| Beispiel | X | Y | Z | $R^1$ | $R^2$ | $R^0$ | Ausbeute % |
|---|---|---|---|---|---|---|---|
| 7g | CH | $C_6H_5$-C | CH | $iC_3H_7$ | 4-$FC_6H_4$ | Na | 95 |
| 7h | CH | $iC_3H_7$-C | CH | $iC_3H_7$ | 4-$FC_6H_4$ | Na | 98 |
| 7i | N | N | 4-$FC_6H_4$-C | $iC_3H_7$ | 4-$FC_6H_4$ | Na | 96 |

Fortsetzung Tabelle 7

R = 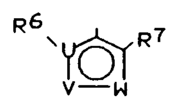 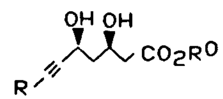

| Beispiel | U | V | W | $R^6$ | $R^7$ | $R^0$ | Ausbeute % |
|---|---|---|---|---|---|---|---|
| 7j | C | $C_6H_5$-N | C-H | $iC_3H_7$ | 4-$FC_6H_4$ | Na | 91 |
| 7k | C | $iC_3H_7$-N | C-H | $iC_3H_7$ | 4-$FC_6H_4$ | Na | 94 |

**Fortsetzung Tabelle 7**

R = 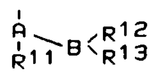

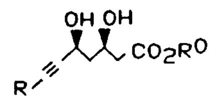

| Beispiel | A-B | $R^{11}$ | $R^{12}$ | $R^{13}$ | $R^0$ | Ausbeute % |
|---|---|---|---|---|---|---|
| 7l | C=C | $iC_3H_7$ | $4\text{-}FC_6H_4$ | $4\text{-}FC_6H_4$ | Na | 99 |
| 7m | C=C | $iC_3H_7$ | $4\text{-}F\text{-}3\text{-}CH_3C_6H_3$ | $4\text{-}F\text{-}3\text{-}CH_3C_6H_3$ | Na | 93 |

**Beispiel 8**

Allgemeine Vorschrift zur Darstellung der Verbindungen der allgemeinen Formel II

**Beispiel 8 a**

6RS-2-(4-(4-Fluorphenyl)-2-(1-methylethyl)-6-phenylpyridin 3-yl)ethinyl-4SR-hydroxy-3,4,5,6-tetrahydro-2H-pyran-2-on (R = a; X = Y-Z = N = C($C_6H_5$)-CH, $R^1$-i$C_3H_7$, $R^2$ = 4-F$C_6H_4$)

Eine Lösung von 1.40 g (2.85 mmol) 3RS, 5SR-Dihydroxy-7-(4-(4-fluorphenyl-2-(1-methylethyl)-6-phenylpyridin-3-yl) hept-6-insäure Natriumsalz (Beispiel 7 a), in 50 ml Wasser wurde mit 3.3 ml (3.3 mmol) 1 M Salzsäure versetzt. Die entstandene Suspension wurde mit Ethylacetat mehrfach extrahiert. Die vereinigten organischen Schichten wurden mehrmals mit gesättigter NaCl-Lösung gewaschen, über $MgSO_4$ getrocknet und eingedampft, das zurückbleibende Harz wurde im Hochvakuum getrocknet, anschließend in 20 ml abs. THF aufgenommen und unter Eiskühlung mit 0.32 g (3.14 mmol) Triethylamin versetzt. Nach 10 min wurden 0.31 g (2.85) Chlorameisensäuremethylester zugetropft, und die resultierende Lösung bei 0 °C gerührt. Das Fortschreiten der Umsetzung wurde dünnschichtchromatographisch verfolgt. Nach 2 h wurde das Gemisch auf Wasser gegeben und mehrfach mit Ether ausgeschüttelt. Die verreinigten organischen Extrakte wurden mit Wasser und ges. NaCl-Lösung gewaschen, über $MgSO_4$ getrocknet und eingedampft. Chromatographische Reinigung des Rückstands (Silicagel; Cyclohexan/Ethylacetat 2 : 1) lieferte 0.95 g 78 %) der Titelverbindung.

Fp: 161 °C

$^1$H-NMR: 1.4(d, J = 7Hz, 6H), 1.9(s, 1H), 2.1(m, 2H), 2.5 (dd, J = 18Hz, 6Hz, 1H), 2.7(dd, J = 18Hz, 6Hz, 1H), 3.7(h, J = 7Hz, 1H), 4.3(m, 1H), 5.5(dd, J = 6Hz, 5Hz, 1H), 7.2(m, 2H), 7.4-7.6(m, 6H), 8.1(m, 2H)

MS: m/e = 430 ($M^+$ + H) $C_{27}H_{24}FNO_3$

**Beispiel 8 b - 8 m**

Im Analogie zu Beispiel 8a wurden die in Tabelle 8 aufgeführten Verbindungen erhalten.

Tabelle 8

R = 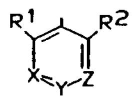

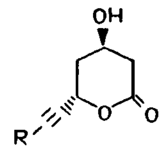

| Beispiel | X | Y | Z | $R^1$ | $R^2$ | Ausbeute % | 'H-NMR: δ/ppm: MS : m/e = |
|---|---|---|---|---|---|---|---|
| 8b | N | $4-FC_6H_4-C$ | CH | $iC_3H_7$ | $4-FC_6H_4$ | 75 | 1.4(d,J=7Hz,6H),1.9(s,1H),2.1 (m,2H),2.6(m,1H),2.7(m,1H),3.8 (h,J=7Hz,1H),4.3(m,1H),5.5 (dd,J=6Hz,5Hz,1H),7.0-8.1(m, 9H). 447 ($M^+$) $C_{27}H_{23}F_2NO_3$ |
| 8c | N | $C_6H_5-C$ | CH | $cC_3H_7$ | $4-FC_6H_4$ | 71 | 1.1(m,2H),1.3(m,2H),2.0(s,1H) 2.2(m,2H),2.5-2.8(m,3H),4.3(m, 1H),5.5(dd,J=6Hz,5Hz,1H),7.2 (m,2H), 7.4-7.6(m,6H),8.0(m, 2H). |

Fortsetzung Tabelle 8

| Beispiel | X | Y | Z | $R^1$ | $R^2$ | Ausbeute % | 1H-NMR: δ/ppm: MS : m/e = |
|---|---|---|---|---|---|---|---|
| | | | | | | | 429 ($M^+$) $C_{27}H_{22}FNO_3$; Fp. 152-157 °C |
| 8d | N | $C_6H_5$-C | N | $iC_3H_7$ | 4-$FC_6H_4$ | 74 | 1.5(d,J=7Hz,6H),2.0(s,1H),2.1(m,2H),2.6(m,1H),2.8(m,1H),3.7(h,J=7Hz,1H),4.3(m,1H),5.6(dd,J=5Hz,6Hz,1H),7.2(m,2H),7.4-7.8(m,5H),8.6(m,2H). 430 ($M^+$) $C_{26}H_{23}FN_2O_3$ |
| 8e | N | 4-$FC_6H_4$-C | N | $iC_3H_7$ | 4-$FC_6H_4$ | 74 | 1.4(d,J=7Hz,6H),2.0(s,1H),2.1(m,2H),2.6(m,1H),2.8(m,1H),3.6(h,J=7Hz,1H),4.1(m,1H),5.6(dd,J=5Hz,6Hz,1H),7.1(m,4H),8.0(m,2H),8.6(m,2H). 448 ($M^+$) $C_{26}H_{22}F_2N_2O_3$; Fp: 174-180 °C |
| 8f | N | $iC_3H_7$-C | N | $iC_3H_7$ | 4-$FC_6H_4$ | 68 | 1.3(d,J=7Hz,6H),1.5(d,J=7Hz,6H),1.8(s,1H),2.1(m,2H),2.6(m,1H),2.8(m,1H),3.5(h,J=7Hz,1H),3.6(h,J=7Hz,1H),4.3(m,1H), |

Fortsetzung Tabelle 8

| Beispiel | X | Y | Z | $R^1$ | $R^2$ | Ausbeute % | 1H-NMR:δ/ppm: MS : m/e = |
|---|---|---|---|---|---|---|---|
| | | | | | | | 5.5(dd,J=6Hz,5Hz,1H),7.2(m,2H) 7.6(m,2H). 396 ($M^+$) $C_{23}H_{25}FN_2O_3$ |
| 8g | CH | $C_6H_5$-C | CH | i$C_3H_7$ | 4-F$C_6H_4$ | 80 | 1.3(d,J=7Hz,6H),1.8(s,1H),2.1 (m,2H),2.5(m,1H),2.7(m,1H), 3.6(h,J=7Hz,1H),4.3(m,1H), 5.5(dd,J=6Hz,5Hz,1H),7.1(m, 2H),7.3-7.5(m,7H),7.6(m,2H) 428 ($M^+$) $C_{28}H_{25}FO_3$ |
| 8h | CH | i$C_3H_7$ | CH | i$C_3H_7$ | 4-F$C_6H_4$ | 77 | 1.1-1.2(m,12H),1.8(s,1H),2.1 (m,2H),2.5(m,1H),2.7(m,1H),3.1 (h,J=7Hz,1H),3.6(h,J=7Hz,1H), 4.2(m,1H),5.4(dd,J=6Hz,5Hz,1H), 7.0-7.4(m,6H). 394 ($M^+$) $C_{25}H_{27}FO_3$ |

Fortsetzung Tabelle 8

| Beispiel | X | Y | Z | $R^1$ | $R^2$ | Ausbeute % | 1H-NMR: δ/ppm: MS : m/e = |
|---|---|---|---|---|---|---|---|
| 8i | N | N | 4-$FC_6H_4$-C | i$C_3H_7$ | 4-$FC_6H_4$ | 59 | 1.4(d,J=7Hz,6H),1.8(s,1H), 2.1(m,2H),2.5(m,1H),2.8(m,1H) 3.8(h,J=7Hz,1H),4.3(m,1H),5.5 (dd,J=6Hz,5Hz,1H),7.1-7.5(m, 8H).<br>448($M^+$) $C_{26}H_{22}F_2N_2O_3$ |

Fortsetzung Tabelle 8

R = 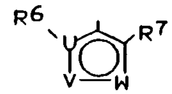 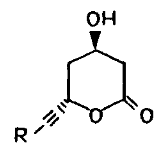

| Beispiel | U | V | W | $R^6$ | $R^7$ | Ausbeute % | $^1H$-NMR: δ/ppm MS : m/e = |
|---|---|---|---|---|---|---|---|
| 8j | C | $C_6H_5$-N | C-H | $iC_3H_7$ | $4-FC_6H_4$ | 74 | 1.3(d,J=7Hz,6H),1.8(s,1H),2.0 (m,2H),2.2(m,1H),2.4(m,1H),3.3 (h,J=7Hz,1H),4.2(m,1H),5.3(dd, J=6Hz,5Hz,1H),6.6(s,1H),6.8-7.2 (m,7H),7.5(m,2H). 417 ($M^+$) $C_{26}H_{24}FNO_3$ |
| 8k | C | $iC_3H_7$-N | C-H | $iC_3H_7$ | $4-FC_6H_4$ | 69 | 1.4(d,J=7Hz,6H),1.5(d,J=Hz,6H), 1.9(s,1H),2.1(m,2H),2.5(m,1H), 2.7(m,1H),3.5(h,J=7Hz,1H),4.3 (m,1H),4.5(h,J=7Hz,1H),5.5(dd, J=6Hz,5Hz,1H),6.6(s,1H),7.1(m 2H),7.3(m,2H). 383 ($M^+$) $C_{23}H_{26}FNO_3$ |

Fortsetzung Tabelle 8

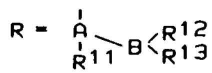

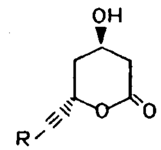

| Beispiel | A-B | $R^{11}$ | $R^{12}$ | $R^{13}$ | Ausbeute % | $^1H$-NMR: δ/ppm MS: m/e |
|---|---|---|---|---|---|---|
| 81 | C=C | $iC_3H_7$ | $4\text{-}FC_6H_4$ | $4\text{-}FC_6H_4$ | 81 | 1.1(d,J=7Hz,6H),1.9(s,1H),2.1(m,2H),2.4-2.8(m,3H),4.2(m,1H),5.3(dd,J=6Hz,5Hz,1H),6.9-7.1(m,8H). 396 ($M^+$) $C_{24}H_{22}F_2O_3$ |
| 8m | C=C | $iC_3H_7$ | $4\text{-}F\text{-}3\text{-}CH_3C_6H_3$ | $4\text{-}F\text{-}3\text{-}CH_3C_6H_3$ | 75 | 1.1(d,J=7Hz,6H),1.9(s,1H),2.0-2.3(m,8H),2.4-2.8(m,3H),4.2(m,1H),5.3(dd,J=6Hz,5Hz,1H),6.9-7.1(m,6H). 424 ($m^+$) $C_{26}H_{26}F_2O_3$ |

**Patentansprüche**
**Patentansprüche für folgende Vertragsstaaten : AT, BE, CH, DE, FR, GB, IT, LI, LU, NL, SE**

**1.** 7-Substituierte 3,5-Dihydroxyhept-6-insäuren und deren Derivate der Formel I

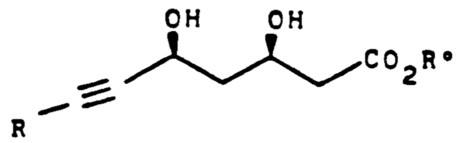

I

sowie die entsprechenden Laktone der Formel II

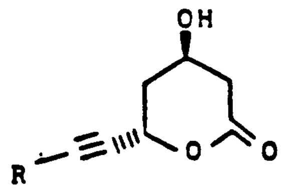

II

wobei in den Formeln I und II R

a) einen Rest der Formel a,

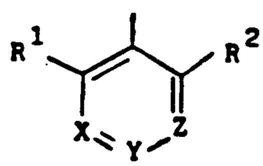

a

worin

$R^1$ und $R^2$ unabhängig voneinander

ein geradkettiger oder verzweigter Alkyl- oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der der Gruppe bestehend aus geradkettigem oder verzweigtem Alkyl mit bis zu 4 C-Atomen, Halogen, Alkoxy mit bis zu 4 C-Atomen und Hydroxy, sind, und

X = Y-Z eine Gruppe der Formel $CR^3=CR^4-CR^5$, $N=CR^4-CR^5$, $N=N-CR^5$, $N=CR^4-N$ ist, worin

$R^3$, $R^4$, $R^5$ unabhängig voneinander

Wasserstoff, ein geradkettiger oder verzweigter Alkyl-oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind

b) einen Rest der Formel b,

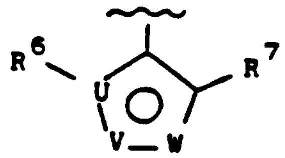

b

worin
$R^6$ und $R^7$ unabhängig voneinander
ein geradkettiger oder verzweigter Alkyl- oder Alkenylrest mit bis zu 6 C-Atomen,
ein gesättigter oder bis zu 2-fach ungesättigter caclischer Kohlenwasserstoffrest mit 3-6 C-Atomen, ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen und Alkoxy mit bis zu 4 C-Atomen sind, und
U-V-W
eine Gruppe der Formel C-$NR^9$-$CR^8$, C-O-$CR^8$, C-S-$CR^8$, C-$NR^9$-N, C-O-N (= C-N-O), C-S-N (= C-N-S), N-$CR^{10}$ = $CR^8$, N-N = $CR^8$ oder N-$CR^{10}$ = N, ist,
worin
$R^8$ Wasserstoff, ein geradkettiger oder verzweigter Alkyl- oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit 1-4 C-Atomen und Hydroxy, ist und
$R^9$, $R^{10}$ unabhängig voneinander
ein geradkettiger oder verzweigter Alkyl- oder Alkenylrest mit bis zu 6 C-Atomen,
ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen und Alkoxy mit bis zu 4 C-Atomen, sind,
oder
c) einen Rest der Formel c,

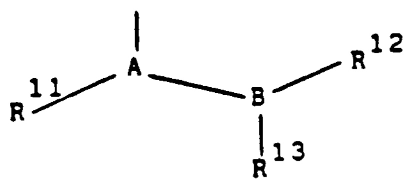

c

worin
A-B eine Gruppe der Formel CH-CH oder C = C ist und
$R^{11}$, $R^{12}$, $R^{13}$ unabhängig voneinander
ein geradkettiger oder verzweigter Alkyl- oder Alkenylrest mit bis zu 20 C-Atomen,
ein gesättigter oder bis zu 2-fach ungesättigter cyclischer Kohlenwasserstoffrest mit 3-6 C-Atomen oder ein Phenylrest, der gegebenenfalls substituiert ist mit 1-3 Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind,
und
R° Wasserstoff, einen geradkettigen oder verzweigten Alkylrest mit bis zu 6 C-Atomen, Alkalimetall oder Ammonium, bedeuten.

**2.** Verbindungen gemäß Anspruch 1, dadurch gekennzeichnet, daß R
a) ein Rest der Formel a, worin
$R^1$ ein geradkettiger oder verzweigter Alkylrest mit bis zu 4 C-Atomen oder ein Cycloalkylrest

mit 3-6 C-Atomen ist;

$R^2$ ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, ist und

X=Y-Z

eine GRuppe der Formel $CR^3=CR^4-CR^5$, $N=CR^4-CR^5$, $N=N-CR^5$ oder $N=CR^4-N$ ist, worin $R^3$, $R^5$ unabhängig voneinander Wasserstoff,

ein geradkettiger oder verzweigter Alkylrest mit bis zu 4 C-Atomen, ein Cycloalkylrest mit 3-6 C-Atomen oder ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, sind und

$R^4$ ein geradkettiger oder verzweigter Alkylrest mit bis zu 4 C-Atomen, ein Cycloalkylrest mit 3-6 C-Atomen, oder ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, ist,

b) ein Rest der Formel b, worin

$R^6$ ein geradkettiger oder verzweigter Alkylrest mit bis zu 4 C-Atomen, ein Cycloalkylrest mit 3-6 C-Atomen ist,

$R^7$ ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy, mit 1-4 C-Atomen und Hydroxy, ist und

U-V-W eine Gruppe der Formel $C-NR^9-CR^8$ ist,

worin

$R^8$ Wasserstoff, ein geradkettiger oder verzweigter Alkylrest mit bis zu 4 C-Atomen, ein Cycloalkylrest mit 3-6 C-Atomen, oder ein Phenylrest, der gegebenenfalls substituiert ist mit 1-3 gleichen oder verschiedenen Resten ausgewählt aus der Gruppe bestehend aus C1-C4-Alkyl, Fluor und Chlor, ist und

$R^9$ ein geradkettiger oder verzweigter Alkylrest mit bis zu 4 C-Atomen, ein Cycloalkylrest mit 3-6 C-Atomen oder ein Phenylrest, der gegebenenfalls substituiert ist mit 1-3 gleichen oder verschiedenen Resten ausgewählt aus der Gruppe bestehend aus C1-C4-Alkyl, Fluor und Chlor, ist

c) oder ein Rest der Formel c ist, worin

A-B eine Gruppe der Formel CH-CH oder C=C, ist

$R^{11}$ ein geradkettiger oder verzweigter Alkylrest mit bis zu 6 C-Atomen, ein Cycloalkylrest mit 3-6 C-Atomen ist und

$R^{12}$, $R^{13}$ unabhängig voneinander ein geradkettiger oder verzweigter Alkylrest mit bis zu 6 C-Atomen, ein Cycloalkylrest mit 3-6 C-Atomen,

ein Phenylrest, der gegebenenfalls substituiert ist mit 1-3 gleichen oder verschiedenen Reste ausgewählt aus der Gruppe bestehend aus C1-C4-Alkyl, Halogen, Alkoxy mit 1-4 C-Atomen und Hydroxy, sind,

und $R^\circ$ Wasserstoff, ein geradkettiger oder verzweigter Alkylrest mit bis zu 4 C-Atomen, Natrium, Kalium ist.

**3.** Verbindungen gemäß Anspruch 1, dadurch gekennzeichnet, daß R

a) ein Rest der Formel a, worin

X=Y-Z eine Gruppe der Formel $CR^3=CR^4-CR^5$ ist, worin

$R^3$ Wasserstoff

$R^4$ Isopropyl, tert.-Butyl, Phenyl oder 4-Fluorphenyl, und

$R^5$ Wasserstoff bedeuten, und

$R^1$ Isopropyl und

$R^2$ 4-Fluorphenyl sind,

X=Y-Z eine Gruppe der Formel $N=CR^4-CR^5$ ist, worin

$R^4$ Isopropyl, tert.-Butyl, Phenyl oder 4-Fluorphenyl und

$R^5$ Wasserstoff bedeuten, und

$R^1$ Isopropyl oder Cyclopropyl und

$R^2$ 4-Fluorphenyl sind,

X=Y-Z eine Gruppe der formel $N=N-CR^5$ ist, worin

$R^5$ Phenyl, 4-Fluorphenyl bedeutet, und
$R^1$ Isopropyl und
$R^2$ 4-Fluorphenyl sind,
X=Y-Z eine Gruppe der Formel N=C$R^4$-N ist, worin
$R^4$ Isopropyl, tert.-Butyl, Phenyl oder 4-Fluorphenyl bedeutet, und
$R^1$ Isopropyl und
$R^2$ Fluorphenyl sind,
b) ein Rest der Formel b, worin
$R^6$ Isopropyl
$R^7$ 4-Fluorphenyl und,
U-V-W eine Gruppe der Formel C-N$R^9$-C$R^8$, worin
$R^8$ Wasserstoff
$R^9$ Ispropyl oder Phenyl bedeuten, sind, oder
c) ein Rest der Formel c ist, worin
A-B eine Gruppe der Formel C=C,
$R^{11}$ Isopropyl und
$R^{12}$ = $R^{13}$ 4-Fluorphenyl oder 4-Fluor-3-methylphenyl sind, und
R° Wasserstoff, Methyl, Ethyl, tert.-Butyl, Natrium oder Kalium ist.

**4.** Verbindungen gemäß Anspruch 1, dadurch gekennzeichnet, daß sie die Formel I besitzen.

**5.** Verfahren zur Herstellung der Verbindungen der allgemeinen Formeln I und II gemäß Anspruch 1 dadurch gekennzeichnet, daß man
a) Aldehyde der Formel III

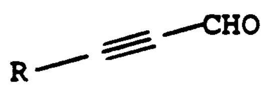

III

worin R die angegebene Bedeutung hat, in die entsprechenden Hydroxyketoester der allgemeinen Formel IV

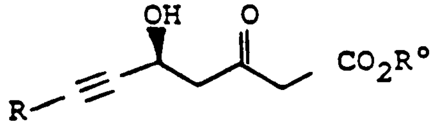

IV

worin R° Alkyl mit 1-6 C-Atomen bedeutet, überführt.
b) die Hydroxyketoester der Formel IV in die entsprechenden 3,5-Dihydroxyverbindungen der Formel I überführt,

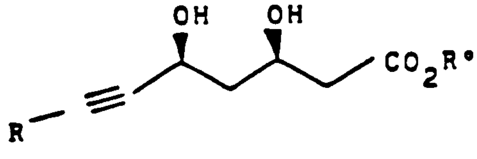

I

worin R die zu Formel I angegebene Bedeutung hat und R° Alkyl mit 1 bis 6 C-Atomen ist, und eine erhaltene Verbindung gegebenenfalls verseift zu einer Verbindung der Formel I, worin R° ein Alkalimetall darstellt, daraus gegebenenfalls die freie Säure (R° = Wasserstoff) in Freiheit setzt und die freie Säure gegebenenfalls in Verbindung der Formel I, worin R° die zu Formel I angegebenen Bedeutungen mit Ausnahme von Wasserstoff hat, überführt,
c) und eine erhaltene Verbindung der allgemeinen Formel I, gegebenenfalls in ein Lacton der Formel II,

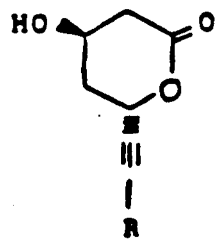

II

worin R die angegebenen Bedeutungen hat, überführt.

6. Pharmazeutische Präparate, enthaltend eine Verbindung gemäß Anspruch 1.

7. Verwendung einer Verbindung gemäß Anspruch 1 zur Herstellung eines Arzneimittels zur Prophylaxe und Therapie der Hypercholesterinämie.

8. Verbindungen der Formel III,

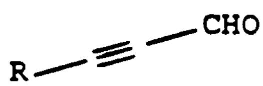

worin R die in Anpruch 1 zu Formel I angegebene Bedeutung hat.

9. Verbindungen der Formel IV, worin R die in Anspruch 1 zu Formel I angegebene Bedeutung hat und R° Alkyl mit 1 bis 6 C-Atomen ist.

**Patentansprüche für folgenden Vertragsstaat : ES**

1. Verfahren zur Herstellung von 7-substituierten 3,5-Dihydroxyhept-6-insäuren und deren Derivaten der Formel I

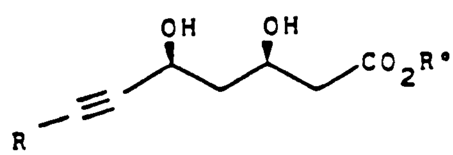

I

sowie von den entsprechenden Laktonen der Formel II

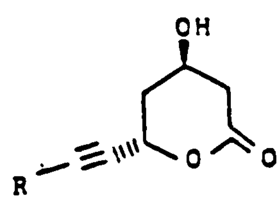

II

wobei in den Formeln I und II R

a) einen Rest der Formel a,

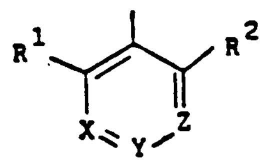

a

worin
$R^1$ und $R^2$ unabhängig voneinander
ein geradkettiger oder verzweigter Alkyl- oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der der Gruppe bestehend aus geradkettigem oder verzweigtem Alkyl mit bis zu 4 C-Atomen, Halogen, Alkoxy mit bis zu 4 C-Atomen und Hydroxy, sind, und

X = Y-Z eine Gruppe der Formel $CR^3=CR^4-CR^5$, $N=CR^4-CR^5$, $N=N-CR^5$, $N=CR^4-N$ ist, worin

$R^3$, $R^4$, $R^5$ unabhängig voneinander
Wasserstoff, ein geradkettiger oder verzweigter Alkyl-oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind,
b) einen Rest der Formel b,

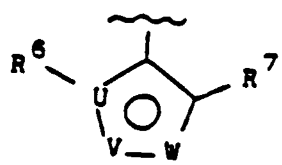

b

worin
$R^6$ und $R^7$ unabhängig voneinander
ein geradkettiger oder verzweigter Alkyl- oder Alkenylrest mit bis zu 6 C-Atomen,
ein gesättigter oder bis zu 2-fach ungesättigter caclischer Kohlenwasserstoffrest mit 3-6 C-Atomen, ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen und Alkoxy mit bis zu 4 C-Atomen sind, und
U-V-W
eine Gruppe der Formel $C-NR^9-CR^8$, $C-O-CR^8$, $C-S-CR^8$, $C-NR^9-N$, C-O-N (= C-N-O), C-S-N (= C-N-S), $N-CR^{10}=CR^8$, $N-N=CR^8$ oder $N-CR^{10}=N$, ist,
worin
$R^8$ Wasserstoff, ein geradkettiger oder verzweigter Alkyl- oder Alkenylrest mit bis zu 6-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit 1-4 C-Atomen und Hydroxy, ist und
$R^9$, $R^{10}$ unabhängig voneinander
ein geradkettiger oder verzweigter Alkyl- oder Alkenylrest mit bis zu 6 C-Atomen,
ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen und Alkoxy mit bis zu 4 C-Atomen, sind,
oder

c) einen Rest der Formel c,

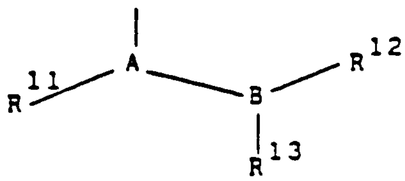

c

worin
A-B eine Gruppe der Formel CH-CH oder C = C ist und
$R^{11}$, $R^{12}$, $R^{13}$ unabhängig voneinander
ein geradkettiger oder verzweigter Alkyl- oder Alkenylrest mit bis zu 20 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind,
und

R° Wasserstoff, einen geradkettigen oder verzweigten Alkylrest mit bis zu 6 C-Atomen, Alkalimetall oder Ammonium, bedeuten, dadurch gekennzeichnet, daß man

a) Aldehyde der Formel III

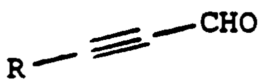

III

worin R die angegebene Bedeutung hat, in die entsprechenden Hydroxyketoester der allgemeinen Formel IV

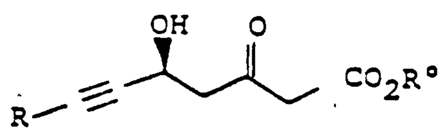

IV

worin R° Alkyl mit 1-6 C-Atomen bedeutet, überführt.

b) die Hydroxyketoester der Formel IV in die entsprechenden 3,5-Dihydroxyverbindungen der Formel I überführt,

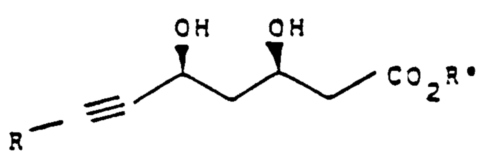

I

worin R die zu Formel I angegebene Bedeutung hat und R° Alkyl mit 1 bin 6 C-Atomen ist, und eine erhaltene Verbindung gegebenenfalls verseift zu einer Verbindung der Formel I, worin R° ein Alkalimetall darstellt, daraus gegebenenfalls die freie Säure (R° = Wasserstoff) in Freiheit setzt und die freie Säure gegebenenfalls in Verbindung der Formel I, worin R° die zu Formel I angegebenen Bedeutungen mit Ausnahme von Wasserstoff hat, überführt,

c) und eine erhaltene Verbindung der allgemeinen Formel I, gegebenenfalls in ein Lacton der Formel II,

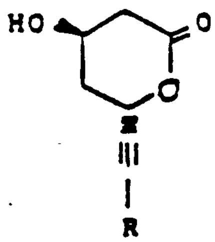

II

worin R die angegebenen Bedeutungen hat, überführt.

**2.** Verfahren gemäß Anspruch 1, dadurch gekennzeichnet, daß man eine Verbindung der Formel I bzw. Formel II, worin R

a) ein Rest der Formel a, worin

$R^1$ ein geradkettiger oder verzweigter Alkylrest mit bis zu 4 C-Atomen oder ein Cycloalkylrest mit 3-6 C-Atomen ist;

$R^2$ ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, ist und

X=Y-Z

eine GRuppe der Formel $CR^3=CR^4-CR^5$, $N=CR^4-CR^5$, $N=N-CR^5$ oder $N=CR^4-N$ ist, worin $R^3$, $R^5$ unabhängig voneinander Wasserstoff,

ein geradkettiger oder verzweigter Alkylrest mit bis zu 4 C-Atomen, ein Cycloalkylrest mit 3-6 C-Atomen oder ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, sind und

$R^4$ ein geradkettiger oder verzweigter Alkylrest mit bis zu 4 C-Atomen,
ein Cycloalkylrest mit 3-6 C-Atomen, oder
ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, ist,

b) ein Rest der Formel b, worin

$R^6$ ein geradkettiger oder verzweigter Alkylrest mit bis zu 4 C-Atomen, ein Cycloalkylrest mit 3-6 C-Atomen ist,

$R^7$ ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy, mit 1-4 C-Atomen und Hydroxy, ist und

U-V-W eine Gruppe der Formel $C-NR^9-CR^8$ ist,

worin

$R^8$ Wasserstoff, ein geradkettiger oder verzweigter Alkylrest mit bis zu 4 C-Atomen,
ein Cycloalkylrest mit 3-6 C-Atomen, oder
ein Phenylrest, der gegebenenfalls substituiert ist mit 1-3 gleichen oder verschiedenen Resten ausgewählt aus der Gruppe bestehend aus C1-C4-Alkyl, Fluor und Chlor, ist und

$R^9$ ein geradkettiger oder verzweigter Alkylrest mit bis zu 4 C-Atomen,
ein Cycloalkylrest mit 3-6 C-Atomen oder ein Phenylrest, der gegebenenfalls substituiert ist mit 1-3 gleichen oder verschiedenen Resten ausgewählt aus der Gruppe bestehend aus C1-C4-Alkyl, Fluor und Chlor, ist

c) oder ein Rest der Formel c ist, worin

A-B eine Gruppe der Formel CH-CH oder C=C, ist

$R^{11}$ ein geradkettiger oder verzweigter Alkylrest mit bis zu 6 C-Atomen,
ein Cycloalkylrest mit 3-6 C-Atomen ist und

$R^{12}$, $R^{13}$ unabhängig voneinander ein geradkettiger oder verzweigter Alkylrest mit bis zu 6 C-Atomen, ein Cycloalkylrest mit 3-6 C-Atomen,

ein Phenylrest, der gegebenenfalls substituiert ist mit 1-3 gleichen oder verschiedenen Reste ausgewählt aus der Gruppe bestehend aus C1-C4-Alkyl, Halogen, Alkoxy mit 1-4 C-Atomen und Hydroxy, sind,

und $R^{\circ}$ Wasserstoff, ein geradkettiger oder verzweigter Alkylrest mit bis zu 4 C-Atomen, Natrium,

Kalium ist, herstellt.

**3.** Verfahren gemäß Anspruch 1, dadurch gekennzeichnet, daß man eine Verbindung der Formel I bzw. Formel II, worin R
a) ein Rest der Formel a, worin
X=Y-Z eine Gruppe der Formel $CR^3=CR^4-CR^5$ ist, worin
$R^3$ Wasserstoff
$R^4$ Isopropyl, tert.-Butyl, Phenyl oder 4-Fluorphenyl, und
$R^5$ Wasserstoff bedeuten, und
$R^1$ Isopropyl und
$R^2$ 4-Fluorphenyl sind,
X=Y-Z eine Gruppe der Formel $N=CR^4-CR^5$ ist, worin
$R^4$ Isopropyl, tert.-Butyl, Phenyl oder 4-Fluorphenyl und
$R^5$ Wasserstoff bedeuten, und
$R^1$ Isopropyl oder Cyclopropyl und
$R^2$ 4-Fluorphenyl sind,
X=Y-Z eine Gruppe der Formel $N=N-CR^5$ ist, worin
$R^5$ Phenyl, 4-Fluorphenyl bedeutet, und
$R^1$ Isopropyl und
$R^2$ 4-Fluorphenyl sind,
X=Y-Z eine Gruppe der Formel $N=CR^4-N$ ist, worin
$R^4$ Isopropyl, tert.-Butyl, Phenyl oder 4-Fluorphenyl bedeutet, und
$R^1$ Isopropyl und
$R^2$ Fluorphenyl sind,
b) ein Rest der Formel b, worin
$R^6$ Isopropyl
$R^7$ 4-Fluorphenyl und,
U-V-W eine Gruppe der Formel $C-NR^9-CR^8$, worin
$R^8$ Wasserstoff
$R^9$ Isopropyl oder Phenyl bedeuten, sind, oder
c) ein Rest der Formel c ist, worin
A-B eine Gruppe der Formel C=C,
$R^{11}$ Isopropyl und
$R^{12}$ = $R^{13}$ 4-Fluorphenyl oder 4-Fluor-3-methylphenyl sind, und
$R^{\circ}$ Wasserstoff, Methyl, Ethyl, tert.-Butyl, Natrium oder Kalium ist, herstellt

**4.** Verfahren gemäß Anspruch 1, dadurch gekennzeichnet, daß man eine Verbindung der Formel I herstellt.

**5.** Verfahren zur Herstellung eines pharmazeutischen Präparates, dadurch gekennzeichnet, daß man eine Verbindung erhältlich gemäß Anspruch 1 in einem weiteren Verfahrensschritt in eine geeignete Darreichungsform überführt.

**6.** Verfahren zur Herstellung von Verbindungen der Formel III,

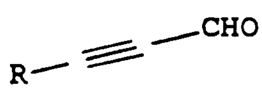

worin R die in Anspruch 1 zu Formel I angegebene Bedeutung hat, dadurch gekennzeichnet, daß man Carbonsäuren der Formel VIII $R-\equiv-CO_2H$ nach an sich bekannten Methoden in die Aldehyde der Formel III überführt.

**Patentansprüche für folgenden Vertragsstaat : GR**

**1.** Verfahren zur Herstellung von 7-substituierten 3,5-Dihydroxyhept-6-insäuren und deren Derivaten der Formel I

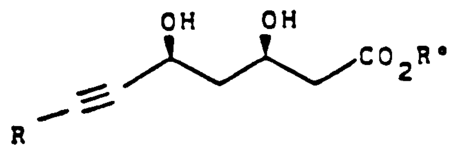

I

sowie von den entsprechenden Laktonen der Formel II

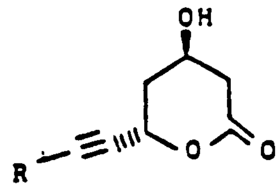

II

wobei in den Formeln I und II R

a) einen Rest der Formel a,

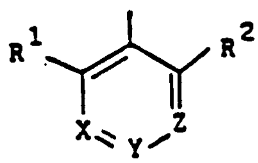

a

worin

$R^1$ und $R^2$ unabhängig voneinander

ein geradkettiger oder verzweigter Alkyl- oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der der Gruppe bestehend aus geradkettigem oder verzweigtem Alkyl mit bis zu 4 C-Atomen, Halogen, Alkoxy mit bis zu 4 C-Atomen und Hydroxy, sind, und

X = Y-Z eine Gruppe der Formel $CR^3=CR^4-CR^5$, $N=CR^4-CR^5$, $N=N-CR^5$, $N=CR^4-N$ ist, worin

$R^3$, $R^4$, $R^5$ unabhängig voneinander

Wasserstoff, ein geradkettiger oder verzweigter Alkyl-oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind,

b) einen Rest der Formel b,

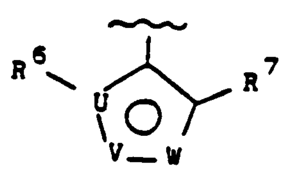

b

worin

$R^6$ und $R^7$ unabhängig voneinander

ein geradkettiger oder verzweigter Alkyl- oder Alkenylrest mit bis zu 6 C-Atomen,

ein gesättigter oder bis zu 2-fach ungesättigter caclischer Kohlenwasserstoffrest mit 3-6 C-Atomen, ein Phenylrest, der gegebenenfalls substituiert ist mit 1-3 gleichen oder verschiedenen Resten

ausgewählt aus der Gruppe bestehend aus geradkettigem oder verzweigtem Alkyl mit bis zu 4 C-Atomen, Halogen und Alkoxy mit bis zu 4 C-Atomen sind, und

U-V-W

eine Gruppe der Formel C-$NR^9$-$CR^8$, C-O-$CR^8$, C-S-$CR^8$, C-$NR^9$-N, C-O-N (= C-N-O), C-S-N (= C-N-S), N-$CR^{10}$ = $CR^8$, N-N = $CR^8$ oder N-$CR^{10}$ = N, ist,

worin

$R^8$ Wasserstoff, ein geradkettiger oder verzweigter Alkyl- oder Alkenylrest mit bis zu 6 C-Atomen, ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen, Alkoxy mit 1-4 C-Atomen und Hydroxy, ist und

$R^9$, $R^{10}$ unabhängig voneinander

ein geradkettiger oder verzweigter Alkyl- oder Alkenylrest mit bis zu 6 C-Atomen,

ein gesättigter oder bis zu 2-fach ungesättigter cyclischer Kohlenwasserstoffrest mit 3-6 C-Atomen oder ein Phenylrest, der gegebenenfalls substituiert ist mit 1-3 gleichen oder verschiedenen Resten ausgewählt aus der Gruppe bestehend aus geradkettigem oder verzweigtem Alkyl mit bis zu 4 C-Atomen, Halogen und Alkoxy mit bis zu 4 C-Atomen, sind,

oder

c) einen Rest der Formel c,

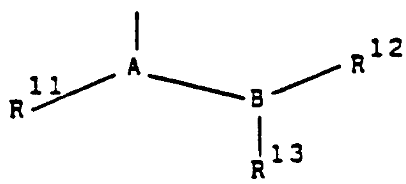

c

worin

A-B eine Gruppe der Formel CH-CH oder C = C ist und

$R^{11}$, $R^{12}$, $R^{13}$ unabhängig voneinander

ein geradkettiger oder verzweigter Alkyl- oder Alkenylrest mit bis zu 20 C-Atomen,

ein gesättigter oder bis zu 2-fach ungesättigter cyclischer Kohlenwasserstoffrest mit 3-6 C-Atomen oder ein Phenylrest, der gegebenenfalls substituiert ist mit 1-3 Resten ausgewählt aus der Gruppe bestehend aus geradkettigem oder verzweigtem Alkyl mit bis zu 4 C-Atomen, Halogen, Alkoxy mit bis zu 4 C-Atomen und Hydroxy, sind,

und

R° Wasserstoff, einen geradkettigen oder verzweigten Alkylrest mit bis zu 6 C-Atomen, Alkalimetall oder Ammonium, bedeuten, dadurch gekennzeichnet, daß man

a) Aldehyde der Formel III

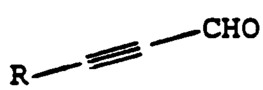

III

worin R die angegebene Bedeutung hat, in die entsprechenden Hydroxyketoester der allgemeinen Formel IV

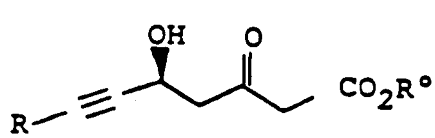

IV

worin R° Alkyl mit 1-6 C-Atomen bedeutet, überführt.

b) die Hydroxyketoester der Formel IV in die entsprechenden 3,5-Dihydroxyverbindungen der Formel I überführt,

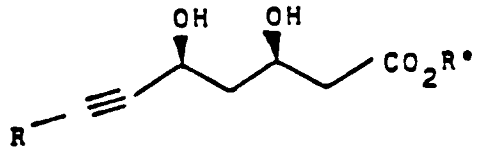

I

worin R die zu Formel I angegebene Bedeutung hat und R° Alkyl mit 1 bis 6 C-Atomen ist, und eine erhaltene Verbindung gegebenenfalls verseift zu einer Verbindung der Formel I, worin R° ein Alkalimetall darstellt, daraus gegebenenfalls die freie Säure (R° = Wasserstoff) in Freiheit setzt und die freie Säure gegebenenfalls in Verbindung der Formel I, worin R° die zu Formel I angegebenen Bedeutungen mit Ausnahme von Wasserstoff hat, überführt,

c) und eine erhaltene Verbindung der allgemeinen Formel I, gegebenenfalls in ein Lacton der Formel II,

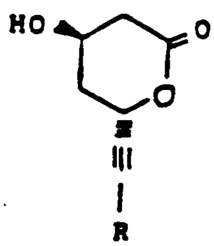

II

worin R die angegebenen Bedeutungen hat, überführt.

**2.** Verfahren gemäß Anspruch 1, dadurch gekennzeichnet, daß man eine Verbindung der Formel I bzw. Formel II, worin R

a) ein Rest der Formel a, worin

$R^1$ ein geradkettiger oder verzweigter Alkylrest mit bis zu 4 C-Atomen oder ein Cycloalkylrest mit 3-6 C-Atomen ist;

$R^2$ ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, ist und

X=Y-Z

eine GRuppe der Formel $CR^3=CR^4-CR^5$, $N=CR^4-CR^5$, $N=N-CR^5$ oder $N=CR^4-N$ ist, worin $R^3$, $R^5$ unabhängig voneinander Wasserstoff,

ein geradkettiger oder verzweigter Alkylrest mit bis zu 4 C-Atomen, ein Cycloalkylrest mit 3-6 C-Atomen oder ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, sind und

$R^4$ ein geradkettiger oder verzweigter Alkylrest mit bis zu 4 C-Atomen,
ein Cycloalkylrest mit 3-6 C-Atomen, oder
ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy mit 1-4 C-Atomen und Hydroxy, ist,

b) ein Rest der Formel b, worin

$R^6$ ein geradkettiger oder verzweigter Alkylrest mit bis zu 4 C-Atomen, ein Cycloalkylrest mit 3-6 C-Atomen ist,

$R^7$ ein Phenylrest, der gegebenenfalls substituiert ist mit 1-3 gleichen oder verschiedenen Resten ausgewählt aus der Gruppe bestehend aus C1-C4-Alkyl, Fluor, Chlor, Alkoxy, mit 1-4 C-Atomen und Hydroxy, ist und

U-V-W eine Gruppe der Formel $C-NR^9-CR^8$ ist,

worin

$R^8$ Wasserstoff, ein geradkettiger oder verzweigter Alkylrest mit bis zu 4 C-Atomen,
ein Cycloalkylrest mit 3-6 C-Atomen, oder
ein Phenylrest, der gegebenenfalls substituiert ist mit 1-3 gleichen oder verschiedenen Resten ausgewählt aus der Gruppe bestehend aus C1-C4-Alkyl, Fluor und Chlor, ist und

$R^9$ ein geradkettiger oder verzweigter Alkylrest mit bis zu 4 C-Atomen, ein Cycloalkylrest mit 3-6 C-Atomen oder ein Phenylrest, der gegebenenfalls substituiert ist mit 1-3 gleichen oder verschiedenen Resten ausgewählt aus der Gruppe bestehend aus C1-C4-Alkyl, Fluor und Chlor, ist

c)oder ein Rest der Formel c ist, worin

A-B eine Gruppe der Formel CH-CH oder C = C, ist

$R^{11}$ ein geradkettiger oder verzweigter Alkylrest mit bis zu 6 C-Atomen, ein Cycloalkylrest mit 3-6 C-Atomen ist und

$R^{12}$, $R^{13}$ unabhängig voneinander ein geradkettiger oder verzweigter Alkylrest mit bis zu 6 C-Atomen, ein Cycloalkylrest mit 3-6 C-Atomen, ein Phenylrest, der gegebenenfalls substituiert ist mit 1-3 gleichen oder verschiedenen Reste ausgewählt aus der Gruppe bestehend aus C1-C4-Alkyl, Halogen, Alkoxy mit 1-4 C-Atomen und Hydroxy, sind,

und R° Wasserstoff, ein geradkettiger oder verzweigter Alkylrest mit bis zu 4 C-Atomen, Natrium, Kalium ist, herstellt.

**3.** Verfahren gemäß Anspruch 1, dadurch gekennzeichnet, daß man eine Verbindung der Formel I bzw. Formel II, worin R

a) ein Rest der Formel a, worin

X = Y-Z eine Gruppe der Formel $CR^3 = CR^4-CR^5$ ist, worin

$R^3$ Wasserstoff

$R^4$ Isopropyl, tert.-Butyl, Phenyl oder 4-Fluorphenyl, und

$R^5$ Wasserstoff bedeuten, und

$R^1$ Isopropyl und

$R^2$ 4-Fluorphenyl sind,

X = Y-Z eine Gruppe der Formel $N = CR^4-CR^5$ ist, worin

$R^4$ Isopropyl, tert.-Butyl, Phenyl oder 4-Fluorphenyl und

$R^5$ Wasserstoff bedeuten, und

$R^1$ Isopropyl oder Cyclopropyl und

$R^2$ 4-Fluorphenyl sind,

X = Y-Z eine Gruppe der Formel $N = N-CR^5$ ist, worin

$R^5$ Phenyl, 4-Fluorphenyl bedeutet, und

$R^1$ Isopropyl und

$R^2$ 4-Fluorphenyl sind,

X = Y-Z eine Gruppe der Formel $N = CR^4-N$ ist, worin

$R^4$ Isopropyl, tert.-Butyl, Phenyl oder 4-Fluorphenyl bedeutet, und

$R^1$ Isopropyl und

$R^2$ Fluorphenyl sind,

b) ein Rest der Formel b, worin

$R^6$ Isopropyl

$R^7$ 4-Fluorphenyl und,

U-V-W eine Gruppe der Formel $C-NR^9-CR^8$, worin

$R^8$ Wasserstoff

$R^9$ Isopropyl oder Phenyl bedeuten, sind, oder

c) ein Rest der Formel c ist, worin

A-B eine Gruppe der Formel C = C,

$R^{11}$ Isopropyl und

$R^{12} = R^{13}$ 4-Fluorphenyl oder 4-Fluor-3-methylphenyl sind, und

R° Wasserstoff, Methyl, Ethyl, tert.-Butyl, Natrium oder Kalium ist.

**4.** Verbindungen gemäß Anspruch 1, dadurch gekennzeichnet, daß sie die Formel I besitzen.

**5.** Verfahren zur Herstellung eines pharmazeutischen Präparates, dadurch gekennzeichnet, daß man eine Verbindung erhältlich gemäß Anspruch 1 in einem weiteren Verfahrensschritt in eine geeignete Darreichungsform überführt.

**6.** Verbindungen der Formel III,

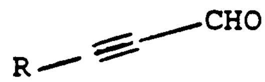

worin R die in Anspruch 1 zu Formel I angegebene Bedeutung hat.

**7.** Verbindungen der Formel IV, worin R die in Anspruch 1 zu Formel I angegebene Bedeutung hat und R° Alkyl mit 1 bis 6 C-Atomen ist.

**Claims**

**Claims for the following Contracting States : AT, BE, CH, DE, FR, GB, IT, LI, LU, NL, SE**

**1.** 7-Substituted 3,5-dihydroxyhept-6-ynoic acids and the derivatives thereof of the formula I

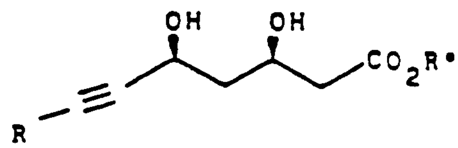

I

as well as the corresponding lactones of the formula II

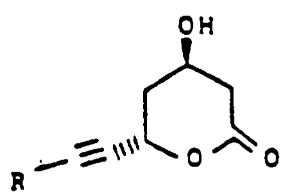

II

where, in formulae I and II, R denotes

a) a radical of the formula a

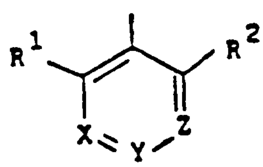

a

in which

$R^1$ and $R^2$ are, independently of one another, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having up to 4 carbon atoms and hydroxyl, and

X = Y-Z is a group of the formula $CR^3 = CR^4-CR^5$, $N = CR^4- CR^5$, $N = N-CR^5$, $N = CR^4-N$, in which

$R^3$, $R^4$, $R^5$ are, independently of one another, hydrogen, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having up to 4 carbon atoms and hydroxyl,

b) a radical of the formula b

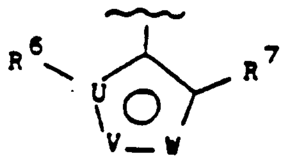

b

in which $R^6$ and $R^7$ are, independently of one another, a straight-chain or branched alkyl or alkenyl radical having up to 6 carbon atoms, a saturated or up to doubly unsaturated cyclic hydrocarbon radical having 3-6 carbon atoms, a phenyl radical which is optionally substituted by 1-3 identical or different radicals selected from the group comprising straight-chain or branched alkyl having up to 4 carbon atoms, halogen and alkoxy having up to 4 carbon atoms, and

U-V-W is a group of the formula $C\text{-}NR^9\text{-}CR^8$, $C\text{-}O\text{-}CR^8$, $C\text{-}S\text{-}CR^8$, $C\text{-}NR^9\text{-}N$, C-O-N (= C-N-O), C-S-N (= C-N-S), $N\text{-}CR^{10}=CR^8$, $N\text{-}N=CR^8$ or $N\text{-}CR^{10}=N$, in which

$R^8$ is hydrogen, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having 1-4 carbon atoms and hydroxyl, and

$R^9$, $R^{10}$ are, independently of one another, a straight-chain or branched alkyl or alkenyl radical having up to 6 carbon atgoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen and alkoxy having up to 4 carbon atoms,

or

c) a radical of the formula c

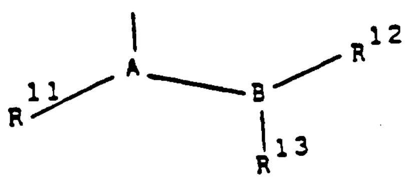

c

in which

A-B is a group of the formula CH-CH or C=C, and

$R^{11}$, $R^{12}$, $R^{13}$ are, independently of one another, a straight-chain or branched alkyl or alkenyl radical having up to 20 carbon atoms, a saturated or up to doubly unsaturated cyclic hydrocarbon radical having 3-6 carbon atoms or a phenyl radical which is optionally substituted by 1-3 radicals selected from the group comprising straight-chain or branched alkyl having up to 4 carbon atoms, halogen, alkoxy having up to 4 carbon atoms and hydroxyl,

and

$R^\circ$ denotes hydrogen, a straight-chain or branched alkyl radical having up to 6 carbon atoms, alkali metal or ammonium.

**2.** Compounds as claimed in claim 1, wherein R is

a) a radical of the formula a in which

$R^1$ is a straight-chain or branched alkyl radical having up to 4 carbon atoms or a cycloalkyl radical having 3-6 carbon atoms,

$R^2$ is a phenyl radical which is optionally substituted by 1-3 identical or different radicals selected from the group comprising C1-C4-alkyl, fluorine, chlorine, alkoxy having 1-4 carbon atoms and hydroxyl, and

X=Y-Z is a group of the formula $CR^3=CR^4\text{-}CR^5$, $N=CR^4\text{-}CR^5$, $N=N\text{-}CR^5$ or $N=CR^4\text{-}N$,

in which

$R^3$, $R^5$ are, independently of one another, hydrogen, a straight-chain or branched alkyl radical having up to 4 carbon atoms, a cycloalkyl radical having 3-6 carbon atoms or a phenyl radical which is optionally substituted by 1-3 identical or different radicals selected from the group comprising $C_1$-$C_4$-alkyl, fluorine, chlorine, alkoxy having 1-4 carbon atoms and hydroxyl, and

$R^4$ is a straight-chain or branched alkyl radical having up to 4 carbon atoms, a cycloalkyl radical having 3-6 carbon atoms, or a phenyl radical which is optionally substituted by 1-3 identical or different radicals selected from the group comprising $C_1$-$C_4$-alkyl, fluorine, chlorine, alkoxy having 1-4 carbon atoms and hydroxyl,

b) a radical of the formula b in which

$R^6$ is a straight-chain or branched alkyl radical having up to 4 carbon atoms, a cycloalkyl radical having 3-6 carbon atoms,

$R^7$ is a phenyl radical which is optionally substituted by 1-3 identical or different radicals selected from the group comprising $C_1$-$C_4$-alkyl, fluorine, chlorine, alkoxy having 1-4 carbon atoms and hydroxyl, and

U-V-W is a group of the formula C-$NR^9$-$CR^8$

in which

$R^8$ is hydrogen, a straight-chain or branched alkyl radical having up to 4 carbon atoms, a cycloalkyl radical having 3-6 carbon atoms, or a phenyl radical which is optionally substituted by 1-3 identical or different radicals selected from the group comprising $C_1$-$C_4$-alkyl, fluorine and chlorine, and

$R^9$ is a straight-chain or branched alkyl radical having up to 4 carbon atoms, a cycloalkyl radical having 3-6 carbon atoms or a phenyl radical which is optionally substituted by 1-3 identical or different radicals selected from the group comprising $C_1$-$C_4$-alkyl, fluorine and chlorine,

c) or a radical of the formula c in which

A-B is a group of the formula CH-CH or C=C,

$R^{11}$ is a straight-chain or branched alkyl radical having up to 6 carbon atoms, a cycloalkyl radical having 3-6 carbon atoms, and

$R^{12}$, $R^{13}$ are, independently of one another, a straight-chain or branched alkyl radical having up to 6 carbon atoms, a cycloalkyl radical having 3-6 carbon atoms, a phenyl radical which is optionally substituted by 1-3 identical or different radicals selected from the group comprising $C_1$-$C_4$-alkyl, halogen, alkoxy having 1-4 carbon atoms and hydroxyl,

and $R^\circ$ is hydrogen, a straight-chain or branched alkyl radical having up to 4 carbon atoms, sodium, potassium.

**3.** Compounds as claimed in claim 1, wherein R is

a) a radical of the formula a in which

X=Y-Z is a group of the formula $CR^3$=$CR^4$-$CR^5$ in which

$R^3$ denotes hydrogen,

$R^4$ denotes isopropyl, tert.-butyl, phenyl or 4-fluorophenyl, and

$R^5$ denotes hydrogen, and

$R^1$ is isopropyl and

$R^2$ is 4-fluorophenyl,

X=Y-Z is a group of the formula N=$CR^4$-$CR^5$ in which

$R^4$ denotes isopropyl, tert.-butyl, phenyl or 4-fluorophenyl and

$R^5$ denotes hydrogen, and

$R^1$ is isopropyl or cyclopropyl and

$R^2$ is 4-fluorophenyl,

X=Y-Z is a group of the formula N=N-$CR^5$ in which

$R^5$ denotes phenyl, 4-fluorophenyl, and

$R^1$ is isopropyl and

$R^2$ is 4-fluorophenyl,

X=Y-Z is a group of the formula N=$CR^4$-N in which

$R^4$ denotes isopropyl, tert.-butyl, phenyl or 4-fluorophenyl, and

$R^1$ is isopropyl and

$R^2$ is fluorophenyl,

b) a radical of the formula b in which
$R^6$ is isopropyl
$R^7$ is 4-fluorophenyl, and
U-V-W is a group of the formula C-$NR^9$-$CR^8$ in which
$R^8$ denotes hydrogen
$R^9$ denotes isopropyl or phenyl, or
c) a radical of the formula c in which
A-B is a group of the formula C=C,
$R^{11}$ is isopropyl and
$R^{12}$ = $R^{13}$ are 4-fluorophenyl or 4-fluoro-3-methylphenyl, and
R° is hydrogen, methyl, ethyl, tert.-butyl, sodium or potassium.

**4.** Compounds as claimed in claim 1, which have the formula I.

**5.** A process for the preparation of the compounds of the formulae I and II as claimed in claim 1, which comprises
a) converting aldehydes of the formula III

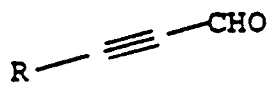

III

in which R has the specified meaning, into the corresponding hydroxy keto esters of the formula IV

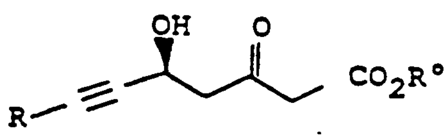

IV

in which R° denotes alkyl having 1-6 carbon atoms,
b) converting the hydroxy keto esters of the formula IV into the corresponding 3,5-dihydroxy compounds of the formula I

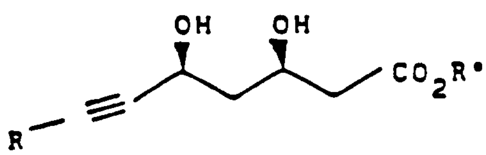

I

in which R has the meaning specified for formula I, and R° is alkyl having 1 to 6 carbon atoms, and, where appropriate, hydrolyzing a resulting compound to a compound of the formula I in which R° represents an alkali metal, liberating therefrom where appropriate the free acid (R° = hydrogen), and converting the free acid where appropriate into a compound of the formula I in which R° has the meanings specified for formula I with the exception of hydrogen,
c) and converting a resulting compound of the formula I where appropriate into a lactone of the formula II

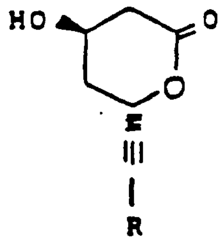

II

in which R has the specified meanings.

6. A pharmaceutical product containing a compound as claimed in claim 1.

7. The use of a compound as claimed in claim 1 for preparing a pharmaceutical for the prophylaxis and therapy of hypercholesterolemia.

8. A compound of the formula III

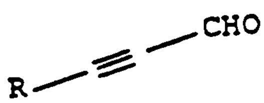

in which R has the meaning specified for formula I in claim 1.

9. A compound of the formula IV in which R has the meaning specified for formula I in claim 1, and R° is alkyl having 1 to 6 carbon atoms.

**Claims for the following Contracting State : ES**

1. A process for the preparation of 7-substituted 3,5-dihydroxyhept-6-ynoic acids and the derivatives thereof of the formula I

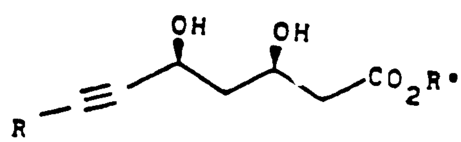

I

as well as the corresponding lactones of the formula II

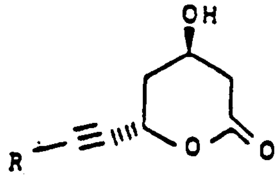

II

where, in formulae I and II, R denotes

a) a radical of the formula a

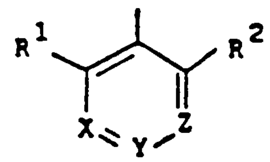

a

in which
$R^1$ and $R^2$ are, independently of one another, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having up to 4 carbon atoms and hydroxyl, and
X = Y-Z is a group of the formula $CR^3 = CR^4-CR^5$, $N = CR^4-CR^5$, $N = N-CR^5$, $N = CR^4-N$, in which
$R^3$, $R^4$, $R^5$ are, independently of one another, hydrogen, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having up to 4 carbon atoms and hydroxyl,
b) a radical of the formula b

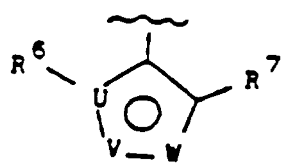

b

in which $R^6$ and $R^7$ are, independently of one another, a straight-chain or branched alkyl or alkenyl radical having up to 6 carbon atoms, a saturated or up to doubly unsaturated cyclic hydrocarbon radical having 3-6 carbon atoms, a phenyl radical which is optionally substituted by 1-3 identical or different radicals selected from the group comprising straight-chain or branched alkyl having up to 4 carbon atoms, halogen and alkoxy having up to 4 carbon atoms, and
U-V-W is a group of the formula $C-NR^9-CR^8$, $C-O-CR^8$, $C-S-CR^8$, $C-NR^9-N$, C-O-N (= C-N-O), C-S-N (= C-N-S), $N-CR^{10} = CR^8$, $N-N = CR^8$ or $N-CR^{10} = N$, in which
$R^8$ is hydrogen, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having 1-4 carbon atoms and hydroxyl, and
$R^9$, $R^{10}$ are, independently of one another, a straight-chain or branched alkyl or alkenyl radical having up to 6 carbon atgoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen and alkoxy having up to 4 carbon atoms,
or
c) a radical of the formula c

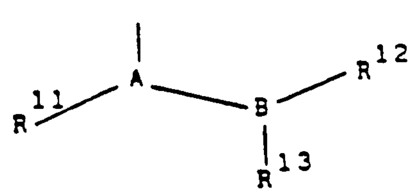

c

in which
A-B is a group of the formula CH-CH or C=C, and
$R^{11}$, $R^{12}$, $R^{13}$ are, independently of one another, a straight-chain or branched alkyl or alkenyl radical having up to 20 carbon atoms, a saturated or up to doubly unsaturated cyclic hydrocarbon radical having 3-6 carbon atoms or a phenyl radical which is optionally substituted by 1-3 radicals selected from the group comprising straight-chain or branched alkyl having up to 4 carbon atoms, halogen, alkoxy having up to 4 carbon atoms and hydroxyl,
and
R° denotes hydrogen, a straight-chain or branched alkyl radical having up to 6 carbon atoms, alkali metal or ammonium, which comprises

a) converting aldehydes of the formula III

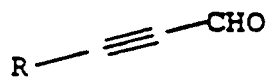

III

in which R has the specified meaning, into the corresponding hydroxy keto esters of the formula IV

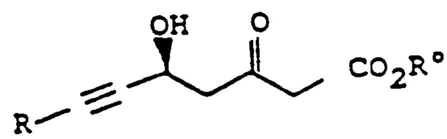

IV

in which R° denotes alkyl having 1-6 carbon atoms,

b) converting the hydroxy keto esters of the formula IV into the corresponding 3,5-dihydroxy compounds of the formula I

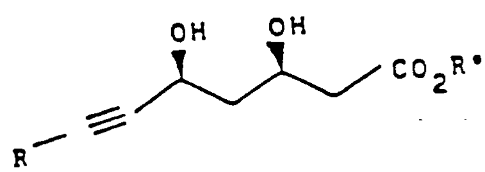

I

in which R has the meaning specified for formula I, and R° is alkyl having 1 to 6 carbon atoms, and, where appropriate, hydrolyzing a resulting compound to a compound of the formula I in which R° represents an alkali metal, liberating therefrom where appropriate the free acid (R° = hydrogen), and converting the free acid where appropriate into a compound of the formula I in which R° has the meanings specified for formula I with the exception of hydrogen,

c) and converting a resulting compound of the formula I where appropriate into a lactone of the formula II

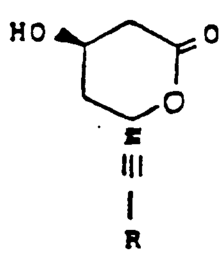

II

in which R has the specified meanings.

**2.** The process as claimed in claim 1, which comprises preparing a compound of the formula I or formula II in which R is

a) a radical of the formula a in which

$R^1$ is a straight-chain or branched alkyl radical having up to 4 carbon atoms or a cycloalkyl radical having 3-6 carbon atoms,

$R^2$ is a phenyl radical which is optionally substituted by 1-3 identical or different radicals selected from the group comprising $C_1$-$C_4$-alkyl, fluorine, chlorine, alkoxy having 1-4 carbon atoms and hydroxyl, and

X=Y-Z is a group of the formula $CR^3=CR^4-CR^5$, $N=CR^4-CR^5$, $N=N-CR^5$ or $N=CR^4-N$,

in which

$R^3$, $R^5$ are, independently of one another, hydrogen, a straight-chain or branched alkyl radical having up to 4 carbon atoms, a cycloalkyl radical having 3-6 carbon atoms or a phenyl radical which is optionally substituted by 1-3 identical or different radicals selected from the group comprising $C_1$-$C_4$-alkyl, fluorine, chlorine, alkoxy having 1-4 carbon atoms and hydroxyl, and

$R^4$ is a straight-chain or branched alkyl radical having up to 4 carbon atoms, a cycloalkyl radical having 3-6 carbon atoms, or a phenyl radical which is optionally substituted by 1-3 identical or different radicals selected from the group comprising $C_1$-$C_4$-alkyl, fluorine, chlorine, alkoxy having 1-4 carbon atoms and hydroxyl,

b) a radical of the formula b in which

$R^6$ is a straight-chain or branched alkyl radical having up to 4 carbon atoms, a cycloalkyl radical having 3-6 carbon atoms,

$R^7$ is a phenyl radical which is optionally substituted by 1-3 identical or different radicals selected from the group comprising $C_1$-$C_4$-alkyl, fluorine, chlorine, alkoxy having 1-4 carbon atoms and hydroxyl, and

U-V-W is a group of the formula $C-NR^9-CR^8$

in which

$R^8$ is hydrogen, a straight-chain or branched alkyl radical having up to 4 carbon atoms, a cycloalkyl radical having 3-6 carbon atoms, or a phenyl radical which is optionally substituted by 1-3 identical or different radicals selected from the group comprising $C_1$-$C_4$-alkyl, fluorine and chlorine, and

$R^9$ is a straight-chain or branched alkyl radical having up to 4 carbon atoms, a cycloalkyl radical having 3-6 carbon atoms or a phenyl radical which is optionally substituted by 1-3 identical or different radicals selected from the group comprising $C_1$-$C_4$-alkyl, fluorine and chlorine,

c) or a radical of the formula c in which

A-B is a group of the formula CH-CH or C=C,

$R^{11}$ is a straight-chain or branched alkyl radical having up to 6 carbon atoms, a cycloalkyl radical having 3-6 carbon atoms, and

$R^{12}$, $R^{13}$ are, independently of one another, a straight-chain or branched alkyl radical having up to 6 carbon atoms, a cycloalkyl radical having 3-6 carbon atoms, a phenyl radical which is optionally substituted by 1-3 identical or different radicals selected from the group comprising $C_1$-$C_4$-alkyl, halogen, alkoxy having 1-4 carbon atoms and hydroxyl,

and $R^\circ$ is hydrogen, a straight-chain or branched alkyl radical having up to 4 carbon atoms, sodium, potassium.

**3.** The process as claimed in claim 1, which comprises preparing a compound of the formula I or formula II in which R is

a) a radical of the formula a in which

X=Y-Z is a group of the formula $CR^3=CR^4-CR^5$ in which

$R^3$ denotes hydrogen,

$R^4$ denotes isopropyl, tert.-butyl, phenyl or 4-fluorophenyl, and

$R^5$ denotes hydrogen, and

$R^1$ is isopropyl and

$R^2$ is 4-fluorophenyl,

X=Y-Z is a group of the formula $N=CR^4-CR^5$ in which

$R^4$ denotes isopropyl, tert.-butyl, phenyl or 4-fluorophenyl and

$R^5$ denotes hydrogen, and
$R^1$ is isopropyl or cyclopropyl and
$R^2$ is 4-fluorophenyl,
X=Y-Z is a group of the formula N=N-$CR^5$ in which
$R^5$ denotes phenyl, 4-fluorophenyl, and
$R^1$ is isopropyl and
$R^2$ is 4-fluorophenyl,
X=Y-Z is a group of the formula N=$CR^4$-N in which
$R^4$ denotes isopropyl, tert.-butyl, phenyl or 4-fluorophenyl, and
$R^1$ is isopropyl and
$R^2$ is fluorophenyl,
b) a radical of the formula b in which
$R^6$ is isopropyl
$R^7$ is 4-fluorophenyl, and
U-V-W is a group of the formula C-$NR^9$-$CR^8$ in which
$R^8$ denotes hydrogen
$R^9$ denotes isopropyl or phenyl, or
c) a radical of the formula c in which
A-B is a group of the formula C=C,
$R^{11}$ is isopropyl and
$R^{12}$ = $R^{13}$ are 4-fluorophenyl or 4-fluoro-3-methylphenyl, and
R° is hydrogen, methyl, ethyl, tert.-butyl, sodium or potassium.

**4.** The process as claimed in claim 1, which comprises preparing a compound of the formula I.

**5.** A process for the preparation of a pharmaceutical product, wherein a compound obtainable according to claim 1 is converted in a further process step into a suitable administration form.

**6.** A process for the preparation of compounds of the formula III

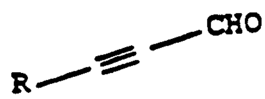

in which R has the meaning specified for formula I in claim 1, wherein carboxylic acids of the formula VIII R-≡-$CO_2H$ are converted by methods known per se into the aldehydes of the formula III.

**Claims for the following Contracting State : GR**

**1.** A process for the preparation of 7-substituted 3,5-dihydroxyhept-6-ynoic acids and the derivatives thereof of the formula I

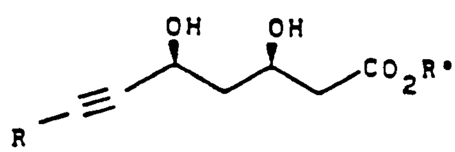

I

as well as the corresponding lactones of the formula II

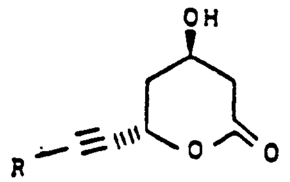

II

where, in formulae I and II, R denotes

a) a radical of the formula a

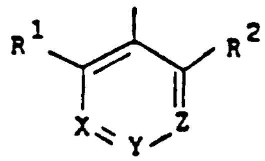

a

in which

$R^1$ and $R^2$ are, independently of one another, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having up to 4 carbon atoms and hydroxyl, and

X=Y-Z is a group of the formula $CR^3=CR^4-CR^5$, $N=CR^4-CR^5$, $N=N-CR^5$, $N=CR^4-N$, in which

$R^3$, $R^4$, $R^5$ are, independently of one another, hydrogen, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having up to 4 carbon atoms and hydroxyl,

b) a radical of the formula b

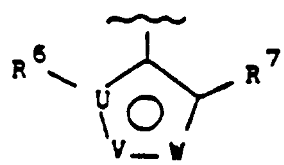

b

in which $R^6$ and $R^7$ are, independently of one another, a straight-chain or branched alkyl or alkenyl radical having up to 6 carbon atoms, a saturated or up to doubly unsaturated cyclic hydrocarbon radical having 3-6 carbon atoms, a phenyl radical which is optionally substituted by 1-3 identical or different radicals selected from the group comprising straight-chain or branched alkyl having up to 4 carbon atoms, halogen and alkoxy having up to 4 carbon atoms, and

U-V-W is a group of the formula $C-NR^9-CR^8$, $C-O-CR^8$, $C-S-CR^8$, $C-NR^9-N$, C-O-N (= C-N-O), C-S-N (= C-N-S), $N-CR^{10}=CR^8$, $N-N=CR^8$ or $N-CR^{10}=N$, in which

$R^8$ is hydrogen, a straight-chain or branched alkyl or alkenyl radical having up to 6 carbon atoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen, alkoxy having 1-4 carbon atoms and hydroxyl, and

$R^9$, $R^{10}$ are, independently of one another, a straight-chain or branched alkyl or alkenyl radical having up to 6 carbon atgoms, a saturated or up to doubly unsaturated cyclic hydrocarbon radical having 3-6 carbon atoms or a phenyl radical which is optionally substituted by 1-3 identical or different radicals selected from the group comprising straight-chain or branched alkyl having up to 4 carbon atoms, halogen and alkoxy having up to 4 carbon atoms,

or

c) a radical of the formula c

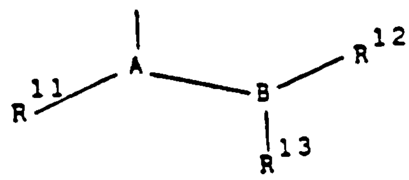

c

in which
A-B is a group of the formula CH-CH or C=C, and
$R^{11}$, $R^{12}$, $R^{13}$ are, independently of one another, a straight-chain or branched alkyl or alkenyl radical having up to 20 carbon atoms, a saturated or up to doubly unsaturated cyclic hydrocarbon radical having 3-6 carbon atoms or a phenyl radical which is optionally substituted by 1-3 radicals selected from the group comprising straight-chain or branched alkyl having up to 4 carbon atoms, halogen, alkoxy having up to 4 carbon atoms and hydroxyl,
and
R° denotes hydrogen, a straight-chain or branched alkyl radical having up to 6 carbon atoms, alkali metal or ammonium, which comprises

a) converting aldehydes of the formula III

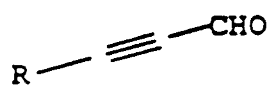

III

in which R has the specified meaning, into the corresponding hydroxy keto esters of the formula IV

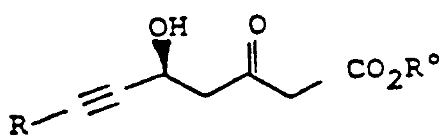

IV

in which R° denotes alkyl having 1-6 carbon atoms,

b) converting the hydroxy keto esters of the formula IV into the corresponding 3,5-dihydroxy compounds of the formula I

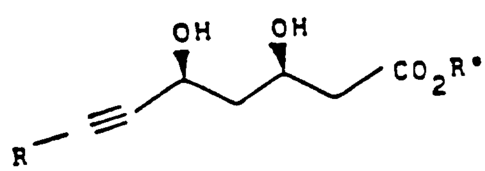

I

in which R has the meaning specified for formula I, and R° is alkyl having 1 to 6 carbon atoms, and, where appropriate, hydrolyzing a resulting compound to a compound of the formula I in which R° represents an alkali metal, liberating therefrom where appropriate the free acid (R° = hydrogen), and converting the free acid where appropriate into a compound of the formula I in which R° has the meanings specified for formula I with the exception of hydrogen,

c) and converting a resulting compound of the formula I where appropriate into a lactone of the formula II

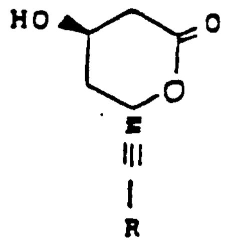

II

in which R has the specified meanings.

**2.** The process as claimed in claim 1, which comprises preparing a compound of the formula I or formula II in which R is

a) a radical of the formula a in which

$R^1$ is a straight-chain or branched alkyl radical having up to 4 carbon atoms or a cycloalkyl radical having 3-6 carbon atoms,

$R^2$ is a phenyl radical which is optionally substituted by 1-3 identical or different radicals selected from the group comprising $C_1$-$C_4$-alkyl, fluorine, chlorine, alkoxy having 1-4 carbon atoms and hydroxyl, and

X=Y-Z is a group of the formula $CR^3=CR^4-CR^5$, $N=CR^4-CR^5$, $N=N-CR^5$ or $N=CR^4-N$,

in which

$R^3$, $R^5$ are, independently of one another, hydrogen, a straight-chain or branched alkyl radical having up to 4 carbon atoms, a cycloalkyl radical having 3-6 carbon atoms or a phenyl radical which is optionally substituted by 1-3 identical or different radicals selected from the group comprising $C_1$-$C_4$-alkyl, fluorine, chlorine, alkoxy having 1-4 carbon atoms and hydroxyl, and

$R^4$ is a straight-chain or branched alkyl radical having up to 4 carbon atoms, a cycloalkyl radical having 3-6 carbon atoms, or a phenyl radical which is optionally substituted by 1-3 identical or different radicals selected from the group comprising $C_1$-$C_4$-alkyl, fluorine, chlorine, alkoxy having 1-4 carbon atoms and hydroxyl,

b) a radical of the formula b in which

$R^6$ is a straight-chain or branched alkyl radical having up to 4 carbon atoms, a cycloalkyl radical having 3-6 carbon atoms,

$R^7$ is a phenyl radical which is optionally substituted by 1-3 identical or different radicals selected from the group comprising $C_1$-$C_4$-alkyl, fluorine, chlorine, alkoxy having 1-4 carbon atoms and hydroxyl, and

U-V-W is a group of the formula $C-NR^9-CR^8$

in which

$R^8$ is hydrogen, a straight-chain or branched alkyl radical having up to 4 carbon atoms, a cycloalkyl radical having 3-6 carbon atoms, or a phenyl radical which is optionally substituted by 1-3 identical or different radicals selected from the group comprising $C_1$-$C_4$-alkyl, fluorine and chlorine, and

$R^9$ is a straight-chain or branched alkyl radical having up to 4 carbon atoms, a cycloalkyl radical having 3-6 carbon atoms or a phenyl radical which is optionally substituted by 1-3 identical or different radicals selected from the group comprising $C_1$-$C_4$-alkyl, fluorine and chlorine,

c) or a radical of the formula c in which

A-B is a group of the formula CH-CH or C=C,

$R^{11}$ is a straight-chain or branched alkyl radical having up to 6 carbon atoms, a cycloalkyl radical having 3-6 carbon atoms, and

$R^{12}$, $R^{13}$ are, independently of one another, a straight-chain or branched alkyl radical having up to 6 carbon atoms, a cycloalkyl radical having 3-6 carbon atoms, a phenyl radical which is optionally substituted by 1-3 identical or different radicals selected from the group comprising $C_1$-$C_4$-alkyl, halogen, alkoxy having 1-4 carbon atoms and hydroxyl,

and $R^\circ$ is hydrogen, a straight-chain or branched alkyl radical having up to 4 carbon atoms, sodium, potassium.

**3.** The process as claimed in claim 1, which comprises preparing a compound of the formula I or formula II in which R is

a) a radical of the formula a in which
X=Y-Z is a group of the formula $CR^3=CR^4-CR^5$ in which
$R^3$ denotes hydrogen,
$R^4$ denotes isopropyl, tert.-butyl, phenyl or 4-fluorophenyl, and
$R^5$ denotes hydrogen, and
$R^1$ is isopropyl and
$R^2$ is 4-fluorophenyl,
X=Y-Z is a group of the formula $N=CR^4-CR^5$ in which
$R^4$ denotes isopropyl, tert.-butyl, phenyl or 4-fluorophenyl and
$R^5$ denotes hydrogen, and
$R^1$ is isopropyl or cyclopropyl and
$R^2$ is 4-fluorophenyl,
X=Y-Z is a group of the formula $N=N-CR^5$ in which
$R^5$ denotes phenyl, 4-fluorophenyl, and
$R^1$ is isopropyl and
$R^2$ is 4-fluorophenyl,
X=Y-Z is a group of the formula $N=CR^4-N$ in which
$R^4$ denotes isopropyl, tert.-butyl, phenyl or 4-fluorophenyl, and
$R^1$ is isopropyl and
$R^2$ is fluorophenyl,
b) a radical of the formula b in which
$R^6$ is isopropyl
$R^7$ is 4-fluorophenyl, and
U-V-W is a group of the formula $C-NR^9-CR^8$ in which
$R^8$ denotes hydrogen
$R^9$ denotes isopropyl or phenyl, or
c) a radical of the formula c in which
A-B is a group of the formula C=C,
$R^{11}$ is isopropyl and
$R^{12} = R^{13}$ are 4-fluorophenyl or 4-fluoro-3-methylphenyl, and
R° is hydrogen, methyl, ethyl, tert.-butyl, sodium or potassium.

**4.** A compound as claimed in claim 1, which has the formula I.

**5.** A process for the preparation of a pharmaceutical product, wherein a compound obtainable as claimed in claim 1 is converted in a further process step into a suitable administration form.

**6.** A compound of the formula III

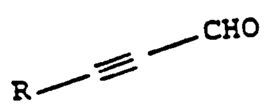

in which R has the meaning specified for formula I in claim 1.

**7.** A compound of the formula IV in which R has the meaning specified for formula I in claim 1, and R° is alkyl having 1 to 6 carbon atoms.

**Revendications**

**Revendications pour les Etats contractants suivants : AT, BE, CH, DE, FR, GB, IT, LI, LU, NL, SE**

**1.** Acides 3,5-dihydroxyhept-6-ynoïques substitués en position 7, et leurs dérivés, de formule I

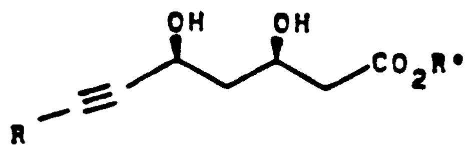

I

ainsi que les lactones correspondantes de formule II

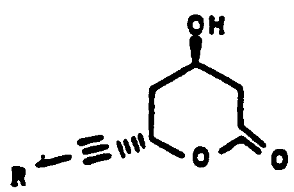

II

dans les formules I et II, R représentant

a) un radical de formule a,

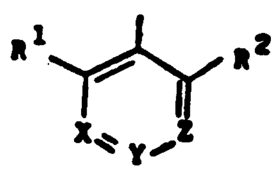

a

dans laquelle

$R^1$ et $R^2$ sont, indépendamment l'un de l'autre,

un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée, ayant jusqu'à 4 atomes de carbone, alcoxy ayant jusqu'à 4 atomes de carbone et hydroxy, et

X=Y-Z est un groupe de formules $CR^3=CR^4-CR^5$, $N=CR^4-CR^5$, $N=N-CR^5$, $N=CR^4-N$, dans lesquelles

$R^3$, $R^4$, $R^5$ représentent, indépendamment les uns des autres, un atome d'hydrogène, un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, alcoxy ayant jusqu'à 4 atomes de carbone et hydroxy,

b) un radical de formule b

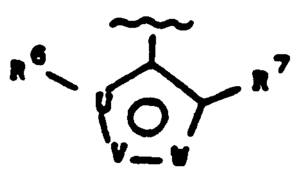

b

dans laquelle $R^6$ et $R^7$ représentent, indépendamment l'un de l'autre, un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, et alcoxy ayant jusqu'à 4 atomes de carbone, et

U-V-W est un groupe de formule C-$NR^9$-$CR^8$, C-O-$CR^8$, C-S-$CR^8$, C-$NR^9$-N, C-O-N (= C-N-O), C-S-N (= C-N-S), N-$CR^{10}$=$CR^8$, N-N=$CR^8$ ou N-$CR^{10}$=N, formules dans lesquelles $R^8$ est un atome d'hydrogène ou un radical alkyle ou alcényle à chaîne droite ou ramifiée ayant jusqu'à 6 atomes de carbone, un radical hydrocarboné cyclique saturé ou jusqu'à deux fois insaturé ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, alcoxy ayant 1-4 atomes de carbone et hydroxy, et $R^9$, $R^{10}$ sont, indépendamment l'un de l'autre un radical alkyle ou alcényle à chaîne droite ou ramifiée ayant jusqu'à 6 atomes de carbone, un radical hydrocarboné cyclique saturé ou jusqu'à deux fois insaturé ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone et alcoxy ayant jusqu'à 4 atomes de carbone, ou

c) un radical de formule c

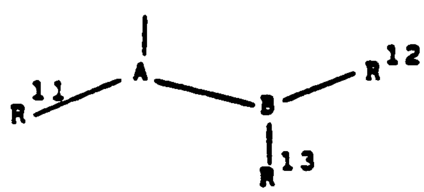

c

dans laquelle

A-B est un groupe de formule CH-CH ou C=C, et

$R^{11}$, $R^{12}$, $R^{13}$ représentent, indépendamment les uns des autres, un radical alkyle ou alcényle à chaîne droite ou ramifiée ayant jusqu'à 20 atomes de carbone, un radical hydrocarboné cyclique saturé ou jusqu'à deux fois insaturé ayant 3-6 atomes de carbone, ou un radical phényle qui est éventuellement substitué par 1-3 substituants choisis parmi des atomes d'halogène et des groupes alkyle à chaîne droite ou ramifiée ayant jusqu'à 4 atomes de carbone, alcoxy ayant jusqu'à 4 atomes de carbone et hydroxy, et

$R^o$ représentant un atome d'hydrogène, un radical alkyle à chaîne droite ou ramifiée ayant jusqu'à 6 atomes de carbone, un métal alcalin ou l'ion ammonium.

**2.** Composé selon la revendication 1, caractérisé en ce que R représente

a) un radical de formule a, dans lequel

$R^1$ est un radical alkyle à chaîne droite ou ramifiée ayant jusqu'à 4 atomes de carbone, ou un radical cycloalkyle ayant 3-6 atomes de carbone,

$R^2$ est un radical phényle, qui est éventuellement substitué par 1-3 substituants identiques ou différents, choisis parmi les atomes de fluor et de chlore et des groupes alkyle en $C_1$-$C_4$, alcoxy ayant 1-4 atomes de carbone et hydroxy, et

X=Y-Z est un groupe de formule $CR^3$=$CR^4$-$CR^5$, N=$CR^4$-$CR^5$, N=N-$CR^5$ ou N=$CR^4$-N, formules dans lesquelles

$R^3$, $R^5$ représentent, indépendamment l'un de l'autre, un atome d'hydrogène ou un radical alkyle à chaîne droite ou ramifiée ayant jusqu'à 4 atomes de carbone, un radical cycloalkyle ayant 3-6 atomes de carbone ou un radical phényle qui est éventuellement substitué par 1-3 substituants identiques ou différents choisis parmi les atomes de fluor et de chlore et des groupes alkyle en $C_1$-$C_4$, alcoxy ayant 1-4 atomes de carbone et hydroxy, et

$R^4$ est un radical alkyle à chaîne droite ou ramifiée ayant jusqu'à 4 atomes de carbone, un radical cycloalkyle ayant 3-6 atomes de carbone, ou un radical phényle qui est éventuellement substitué par 1-3 substituants identiques ou différents, choisis parmi

les atomes de fluor et de chlore et des groupes alkyle en $C_1$-$C_4$, alcoxy ayant 1-4 atomes de carbone et hydroxy,

b) un radical de formule b, dans laquelle

$R^6$ est un radical alkyle à chaîne droite ou ramifiée ayant jusqu'à 4 atomes de carbone, un radical cycloalkyle ayant 3-6 atomes de carbone,

$R^7$ représente un radical phényle qui est éventuellement substitué par 1-3 substituants identiques ou différents, choisis parmi les atomes de fluor et de chlore et des groupes alkyle en $C_1$-$C_4$, alcoxy ayant 1-4 atomes de carbone et hydroxy, et

U-V-W est un groupe de formule C-$NR^9$-$CR^8$, dans laquelle

$R^8$ représente un atome d'hydrogène ou un radical alkyle à chaîne droite ou ramifiée ayant jusqu'à 4 atomes de carbone, un radical cycloalkyle ayant 3-6 atomes de carbone, ou un radical phényle qui est éventuellement substitué par 1-3 substituants identiques ou différents, choisis parmi les atomes de fluor et de chlore et des groupes alkyle en $C_1$-$C_4$, et

$R^9$ est un radical alkyle à chaîne droite ou ramifiée ayant jusqu'à 4 atomes de carbone, un radical cycloalkyle ayant 3-6 atomes de carbone ou un radical phényle qui est éventuellement substitué par 1-3 radicaux identiques ou différents, choisis parmi les atomes de fluor et de chlore et des groupes alkyle en $C_1$-$C_4$,

c) ou un radical de formule c, dans laquelle

A-B est un groupe de formule CH-CH ou C = C,

$R^{11}$ est un radical alkyle à chaîne droite ou ramifiée ayant jusqu'à 6 atomes de carbone, un radical cycloalkyle ayant 3-6 atomes de carbone et

$R^{12}$, $R^{13}$ représentent, indépendamment l'un de l'autre, un radical alkyle à chaîne droite ou ramifiée ayant jusqu'à 6 atomes de carbone, un radical cycloalkyle ayant 3-6 atomes de carbone, un radical phényle qui est éventuellement substitué par 1-3 radicaux identiques ou différents, choisis parmi des atomes d'halogène et des groupes alkyle en $C_1$-$C_4$, alcoxy ayant 1-4 atomes de carbone et hydroxy;

et $R^o$ est un atome d'hydrogène, de sodium ou de potassium ou un radical alkyle à chaîne droite ou ramifiée ayant jusqu'à 4 atomes de carbone.

**3.** Composé selon la revendication 1, caractérisé en ce que R représente

a) un radical de formule a, dans laquelle

X = Y-Z est un groupe de formule $CR^3$ = $CR^4$-$CR^5$, dans laquelle

$R^3$ représente un atome d'hydrogène,

$R^4$ représente le groupe isopropyle, tert-butyle, phényle ou 4-fluorophényle, et

$R^5$ représente un atome d'hydrogène, et

$R^1$ est le groupe isopropyle et

$R^2$ est le groupe 4-fluorophényle,

X = Y-Z est un groupe de formule N = $CR^4$-$CR^5$, dans laquelle

$R^4$ représente le groupe isopropyle, tert-butyle, phényle ou 4-fluorophényle, et

$R^5$ représente un atome d'hydrogène, et

$R^1$ représente le groupe isopropyle ou cyclopropyle, et

$R^2$ représente le groupe 4-fluorophényle,

X = Y-Z est un groupe de formule N = N-$CR^5$, dans laquelle

$R^5$ représente le groupe phényle ou 4-fluorophényle, et

$R^1$ représente le groupe isopropyle et

$R^2$ représente le groupe 4-fluorophényle,

X = Y-Z est un groupe de formule N = $CR^4$-N, dans laquelle

$R^4$ représente le groupe isopropyle, tert-butyle, phényle ou 4-fluorophényle, et

$R^1$ est le groupe isopropyle et $R^2$ est le groupe fluorophényle,

b) un radical de formule b, dans laquelle

$R^6$ est le groupe isopropyle,

$R^7$ est le groupe 4-fluorophényle et

U-V-W est un groupe de formule C-$NR^9$-$CR^8$, dans laquelle

$R^8$ représente un atome d'hydrogène,

$R^9$ représente le groupe isopropyle ou phényle,

c) un radical de formule c, dans laquelle

A-B est un groupe de formule C = C,

$R^{11}$ est le groupe isopropyle et

$R^{12} = R^{13}$ et représentent chacun le groupe 4-fluorophényle ou 4-fluoro-3-méthylphényle, et

R° est un atome d'hydrogène, de sodium ou de potassium ou le radical méthyle, éthyle ou tert-butyle.

**4.** Composés selon la revendication 1, caractérisés en ce qu'ils correspondent à la formule I.

**5.** Procédé pour la préparation des composés de formules générales I et II selon la revendication 1, caractérisé en ce que

a) on convertit des aldéhydes de formule III

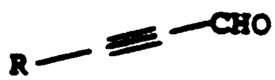

III

dans laquelle R a la signification donnée, en les hydroxycétoesters correspondants, de formule générale IV

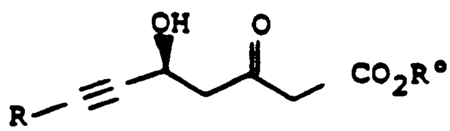

IV

dans laquelle R a la signification donnée, et R° représente un groupe alkyle ayant de 1 à 6 atomes de carbone,

b) on convertit les hydroxycétoesters de formule IV en les composés 3,5-dihydroxy correspondants, de formule I

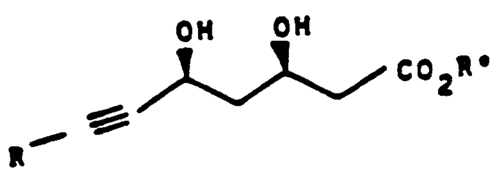

I

dans laquelle R a la signification donnée à propos de la formule I, et R° est un groupe alkyle ayant de 1 à 6 atomes de carbone, et éventuellement on saponifie un composé obtenu pour aboutir à un composé de formule I dans lequel R° représente un métal alcalin, on libère éventuellement à partir de celui-ci l'acide libre (R° est un atome d'hydrogène) et éventuellement on convertit l'acide libre en un composé de formule I, dans lequel R° a les significations données à propos de la formule I, à l'exception de celle d'un atome d'hydrogène,

c) et éventuellement on convertit un composé obtenu de formule générale I en une lactone de formule II

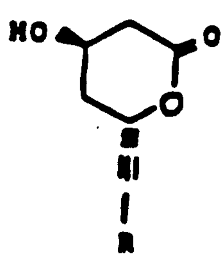

II

dans laquelle R a la signification donnée.

6. Compositions pharmaceutiques, contenant un composé selon la revendication 1.

7. Utilisation d'un composé selon la revendication 1, pour la fabrication d'un médicament pour la prophylaxie et le traitement de l'hypercholestérolémie.

8. Composés de formule III

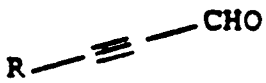

dans laquelle R a la signification donnée à propos de la formule I dans la revendication 1.

9. Composés de formule IV, dans lesquels R a la signification donnée à propos de la formule I dans la revendication 1, et $R^o$ est un groupe alkyle ayant de 1 à 6 atomes de carbone.

**Revendications pour l'Etat contractant suivant : ES**

1. Procédé pour la préparation d'acides 3,5-dihydroxyhept-6-ynoïques substitués en position 7, et de leurs dérivés, de formule I

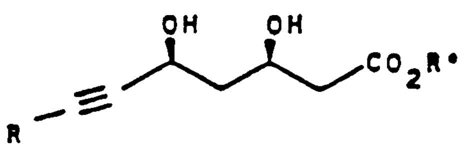

I

ainsi que les lactones correspondantes de formule II

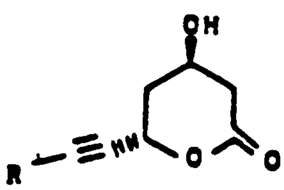

II

dans les formules I et II, R représentant
a) un radical de formule a,

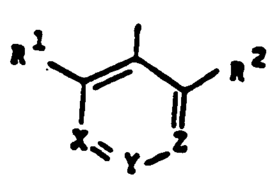

a

dans laquelle
$R^1$ et $R^2$ sont, indépendamment l'un de l'autre,
un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée, ayant jusqu'à 4 atomes de carbone, alcoxy ayant jusqu'à
4 atomes de carbone et hydroxy, et

X=Y-Z est un groupe de formules $CR^3=CR^4-CR^5$, $N=CR^4-CR^5$, $N=N-CR^5$, $N=CR^4-N$, dans lesquelles
$R^3$, $R^4$, $R^5$ représentent, indépendamment les uns des autres, un atome d'hydrogène, un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, alcoxy ayant jusqu'à 4 atomes de carbone et hydroxy,
b) un radical de formule b

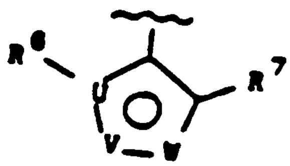

b

dans laquelle $R^6$ et $R^7$ représentent, indépendamment l'un de l'autre, un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, et alcoxy ayant jusqu'à 4 atomes de carbone, et
U-V-W est un groupe de formule $C-NR^9-CR^8$, $C-O-CR^8$, $C-S-CR^8$, $C-NR^9-N$, C-O-N (= C-N-O), C-S-N (= C-N-S), $N-CR^{10}=CR^8$, $N-N=CR^8$ ou $N-CR^{10}=N$, formules dans lesquelles $R^8$ est un atome d'hydrogène ou un radical alkyle ou alcényle à chaîne droite ou ramifiée ayant jusqu'à 6 atomes de carbone, un radical hydrocarboné cyclique saturé ou jusqu'à deux fois insaturé ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, alcoxy ayant 1-4 atomes de carbone et hydroxy, et $R^9$, $R^{10}$ sont, indépendamment l'un de l'autre un radical alkyle ou alcényle à chaîne droite ou ramifiée ayant jusqu'à 6 atomes de carbone, un radical hydrocarboné cyclique saturé ou jusqu'à deux fois insaturé ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone et alcoxy ayant jusqu'à 4 atomes de carbone, ou
c) un radical de formule c

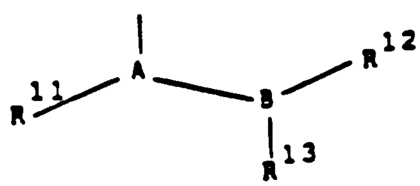

c

dans laquelle
A-B est un groupe de formule CH-CH ou C=C, et
$R^{11}$, $R^{12}$, $R^{13}$ représentent, indépendamment les uns des autres, un radical alkyle ou alcényle à chaîne droite ou ramifiée ayant jusqu'à 20 atomes de carbone, un radical hydrocarboné cyclique saturé ou jusqu'à deux fois insaturé ayant 3-6 atomes de carbone, ou un radical phényle qui est éventuellement substitué par 1-3 substituants choisis parmi des atomes d'halogène et des groupes alkyle à chaîne droite ou ramifiée ayant jusqu'à 4 atomes de carbone, alcoxy ayant jusqu'à 4 atomes de carbone et hydroxy, et

- $R^o$ représentant un atome d'hydrogène, un radical alkyle à chaîne droite ou ramifiée ayant jusqu'à 6 atomes de carbone, un métal alcalin ou l'ion ammonium,

caractérisé en ce que

a) on convertit des aldéhydes de formule III

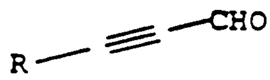

III

dans laquelle R a la signification donnée, en les hydroxycétoesters correspondants, de formule générale IV

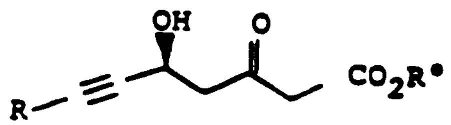

IV

dans laquelle R a la signification donnée, et $R^o$ représente un groupe alkyle ayant de 1 à 6 atomes de carbone,

b) on convertit les hydroxycétoesters de formule IV en les composés 3,5-dihydroxy correspondants, de formule I

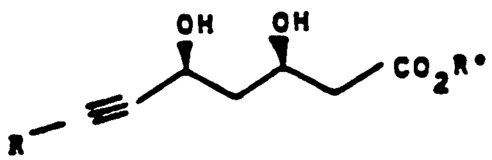

I

dans laquelle R a la signification donnée à propos de la formule I, et $R^o$ est un groupe alkyle ayant de 1 à 6 atomes de carbone, et éventuellement on saponifie un composé obtenu pour aboutir à un composé de formule I dans lequel $R^o$ représente un métal alcalin, on libère éventuellement à partir de celui-ci l'acide libre ($R^o$ est un atome d'hydrogène) et éventuellement on convertit l'acide libre en un composé de formule I, dans lequel $R^o$ a les significations données à propos de la formule I, à l'exception de celle d'un atome d'hydrogène,

c) et éventuellement on convertit un composé obtenu de formule générale I en une lactone de formule II

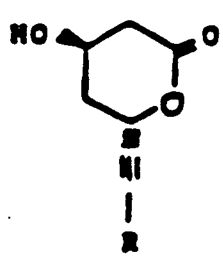

II

dans laquelle R a la signification donnée.

**2.** Procédé selon la revendication 1, caractérisé en ce que l'on prépare un composé de formule I ou II, dans lequel R représente

a) un radical de formule a, dans lequel

$R^1$ est un radical alkyle à chaîne droite ou ramifiée ayant jusqu'à 4 atomes de carbone, ou un radical cycloalkyle ayant 3-6 atomes de carbone,

$R^2$ est un radical phényle, qui est éventuellement substitué par 1-3 substituants identiques ou différents, choisis parmi les atomes de fluor et de chlore et des groupes alkyle en $C_1$-$C_4$, alcoxy ayant 1-4 atomes de carbone et hydroxy, et

X=Y-Z est un groupe de formule $CR^3=CR^4-CR^5$, $N=CR^4-CR^5$, $N=N-CR^5$ ou $N=CR^4-N$, formules dans lesquelles

$R^3$, $R^5$ représentent, indépendamment l'un de l'autre, un atome d'hydrogène ou un radical alkyle à chaîne droite ou ramifiée ayant jusqu'à 4 atomes de carbone, un radical cycloalkyle ayant 3-6 atomes de carbone ou un radical phényle qui est éventuellement substitué par 1-3 substituants identiques ou différents choisis parmi les atomes de fluor et de chlore et des groupes alkyle en $C_1$-$C_4$, alcoxy ayant 1-4 atomes de carbone et hydroxy, et

$R^4$ est un radical alkyle à chaîne droite ou ramifiée ayant jusqu'à 4 atomes de carbone, un radical cycloalkyle ayant 3-6 atomes de carbone, ou un radical phényle qui est éventuellement substitué par 1-3 substituants identiques ou différents, choisis parmi les atomes de fluor et de chlore et des groupes alkyle en $C_1$-$C_4$, alcoxy ayant 1-4 atomes de carbone et hydroxy,

b) un radical de formule b, dans laquelle

$R^6$ est un radical alkyle à chaîne droite ou ramifiée ayant jusqu'à 4 atomes de carbone, un radical cycloalkyle ayant 3-6 atomes de carbone,

$R^7$ représente un radical phényle qui est éventuellement substitué par 1-3 substituants identiques ou différents, choisis parmi les atomes de fluor et de chlore et des groupes alkyle en $C_1$-$C_4$, alcoxy ayant
1-4 atomes de carbone et hydroxy, et

U-V-W est un groupe de formule C-$NR^9$-$CR^8$, dans laquelle

$R^8$ représente un atome d'hydrogène ou un radical alkyle à chaîne droite ou ramifiée ayant jusqu'à 4 atomes de carbone, un radical cycloalkyle ayant 3-6 atomes de carbone, ou un radical phényle qui est éventuellement substitué par 1-3 substituants identiques ou différents, choisis parmi les atomes de fluor et de chlore et des groupes alkyle en $C_1$-$C_4$, et

$R^9$ est un radical alkyle à chaîne droite ou ramifiée ayant jusqu'à 4 atomes de carbone, un radical cycloalkyle ayant 3-6 atomes de carbone ou un radical phényle qui est éventuellement substitué par 1-3 radicaux identiques ou différents, choisis parmi les atomes de fluor et de chlore et des groupes alkyle en $C_1$-$C_4$,

c) ou un radical de formule c, dans laquelle

A-B est un groupe de formule CH-CH ou C=C,

$R^{11}$ est un radical alkyle à chaîne droite ou ramifiée ayant jusqu'à 6 atomes de carbone, un radical cycloalkyle ayant 3-6 atomes de carbone et

$R^{12}$, $R^{13}$ représentent, indépendamment l'un de l'autre, un radical alkyle à chaîne droite ou ramifiée ayant jusqu'à 6 atomes de carbone, un radical cycloalkyle ayant 3-6 atomes de carbone, un radical phényle qui est éventuellement substitué par 1-3 radicaux identiques ou différents, choisis parmi des atomes d'halogène et des groupes alkyle en $C_1$-$C_4$, alcoxy ayant 1-4 atomes de carbone et hydroxy;

et $R^o$ est un atome d'hydrogène, de sodium ou de potassium ou un radical alkyle à chaîne droite ou ramifiée ayant jusqu'à 4 atomes de carbone.

**3.** Procédé selon la revendication 1, caractérisé en ce que l'on prépare un composé de formule I ou II, dans lequel R représente

a) un radical de formule a, dans laquelle

X=Y-Z est un groupe de formule $CR^3$=$CR^4$-$CR^5$, dans laquelle

$R^3$ représente un atome d'hydrogène,

$R^4$ représente le groupe isopropyle, tert-butyle, phényle ou 4-fluorophényle, et

$R^5$ représente un atome d'hydrogène, et

$R^1$ est le groupe isopropyle et

$R^2$ est le groupe 4-fluorophényle,

X=Y-Z est un groupe de formule N=$CR^4$-$CR^5$, dans laquelle

$R^4$ représente le groupe isopropyle, tert-butyle, phényle ou 4-fluorophényle, et

$R^5$ représente un atome d'hydrogène, et

$R^1$ représente le groupe isopropyle ou cyclopropyle, et

$R^2$ représente le groupe 4-fluorophényle,

X=Y-Z est un groupe de formule N=N-$CR^5$, dans laquelle

$R^5$ représente le groupe phényle ou 4-fluorophényle, et

$R^1$ représente le groupe isopropyle et

$R^2$ représente le groupe 4-fluorophényle,

X=Y-Z est un groupe de formule $N=CR^4$-N, dans laquelle
$R^4$ représente le groupe isopropyle, tert-butyle, phényle ou 4-fluorophényle, et
$R^1$ est le groupe isopropyle et $R^2$ est le groupe fluorophényle,
b) un radical de formule b, dans laquelle
$R^6$ est le groupe isopropyle,
$R^7$ est le groupe 4-fluorophényle et
U-V-W est un groupe de formule C-$NR^9$-$CR^8$, dans laquelle
$R^8$ représente un atome d'hydrogène,
$R^9$ représente le groupe isopropyle ou phényle,
c) un radical de formule c, dans laquelle
A-B est un groupe de formule C=C,
$R^{11}$ est le groupe isopropyle et
$R^{12}$ = $R^{13}$ et représentent chacun le groupe 4-fluorophényle ou 4-fluoro-3-méthylphényle, et
$R^o$ est un atome d'hydrogène, de sodium ou de potassium ou le radical méthyle, éthyle ou tert-butyle.

**4.** Procédé selon la revendication 1, caractérisés en ce que l'ou prépare un composé de formule I.

**5.** Procédé pour la préparation d'une composition pharmaceutique, caractérisé en ce que, dans une autre étape du procédé, on convertit en une forme d'administration appropriée un composé pouvant être obtenu selon la revendication 1.

**6.** Procédé pour la preparation de composé de formule III

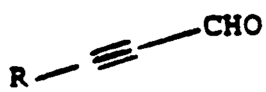

dans laquelle R a la signification donnée à propos de la formule I dans la revendication 1,
caractétisé en se que l'on convertit des acides carboxyliques de formule VIII R-≡-$CO_2H$ en les aldéhydes de formule III par des méthodes connus.

**Revendications pour l'Etat contractant suivant : GR**

**1.** Procédé pour la préparation d'acides 3,5-dihydroxyhept-6-ynoïques substitués en position 7, et de leurs dérivés, de formule I

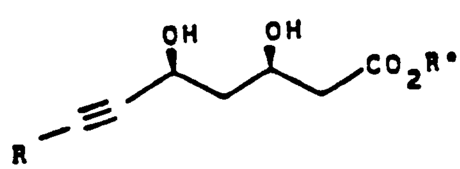

I

ainsi que des lactones correspondantes de formule II

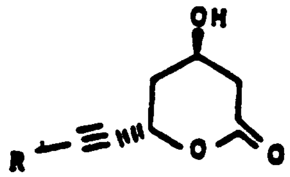

II

dans les formules I et II, R représentant

a) un radical de formule a,

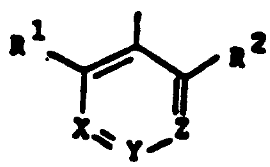

a

dans laquelle
$R^1$ et $R^2$ sont, indépendamment l'un de l'autre,
un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée, ayant jusqu'à 4 atomes de carbone, alcoxy ayant jusqu'à
4 atomes de carbone et hydroxy, et
X=Y-Z est un groupe de formules $CR^3=CR^4-CR^5$, $N=CR^4-CR^5$, $N=N-CR^5$, $N=CR^4-N$, dans lesquelles
$R^3$, $R^4$, $R^5$ représentent, indépendamment les uns des autres, un atome d'hydrogène, un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, alcoxy ayant jusqu'à 4 atomes de carbone et hydroxy,
b) un radical de formule b

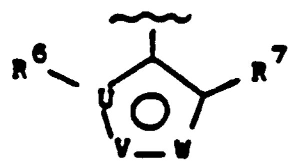

b

dans laquelle $R^6$ et $R^7$ représentent, indépendamment l'un de l'autre, un radical alkyle ou alcényle à chaîne droite ou ramifiée ayant jusqu'à 6 atomes de carbone, un radical hydrocarboné cyclique saturé ou jusqu'à deux fois insaturé, ayant 3-6 atomes de carbone, un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, et alcoxy ayant jusqu'à 4 atomes de carbone, et
U-V-W est un groupe de formule $C-NR^9-CR^8$, $C-O-CR^8$, $C-S-CR^8$, $C-NR^9-N$, C-O-N (= C-N-O), C-S-N (= C-N-S), $N-CR^{10}=CR^8$, $N-N=CR^8$ ou $N-CR^{10}=N$, formules dans lesquelles $R^8$ est un atome d'hydrogène ou un radical alkyle ou alcényle à chaîne droite ou ramifiée ayant jusqu'à 6 atomes de carbone, un radical hydrocarboné cyclique saturé ou jusqu'à deux fois insaturé ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone, alcoxy ayant 1-4 atomes de carbone et hydroxy, et $R^9$, $R^{10}$ sont, indépendamment l'un de l'autre un radical alkyle ou alcényle à chaîne droite ou ramifiée ayant jusqu'à 6 atomes de carbone, un radical hydrocarboné cyclique saturé ou jusqu'à deux fois insaturé ayant 3-6 atomes de carbone, ou un radical phényle qui est éventuellement substitué par 1-3 substituants identiques ou différents, choisis parmi des atomes d'halogène et des groupes alkyle à chaîne droite ou ramifiée ayant jusqu'à 4 atomes de carbone et alcoxy ayant jusqu'à 4 atomes de carbone, ou

c) un radical de formule c

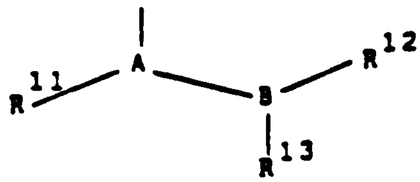

c

dans laquelle
A-B est un groupe de formule CH-CH ou C = C, et
$R^{11}$, $R^{12}$, $R^{13}$ représentent, indépendamment les uns des autres, un radical alkyle ou alcényle à chaîne droite ou ramifiée ayant jusqu'à 20 atomes de carbone, un radical hydrocarboné cyclique saturé ou jusqu'à deux fois insaturé ayant 3-6 atomes de carbone, ou un radical phényle qui est éventuellement substitué par 1-3 substituants choisis parmi des atomes d'halogène et des groupes alkyle à chaîne droite ou ramifiée ayant jusqu'à 4 atomes de carbone, alcoxy ayant jusqu'à 4 atomes de carbone et hydroxy, et

$R^o$ représentant un atome d'hydrogène, un radical alkyle à chaîne droite ou ramifiée ayant jusqu'à 6 atomes de carbone, un métal alcalin ou l'ion ammonium,

caractérisé en ce que

a) on convertit des aldéhydes de formule III

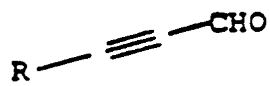

III

dans laquelle R a la signification donnée, en les hydroxycétoesters correspondants, de formule générale IV

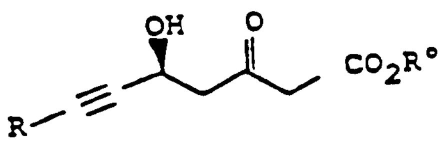

IV

dans laquelle R a la signification donnée, et $R^o$ représente un groupe alkyle ayant de 1 à 6 atomes de carbone,

b) on convertit les hydroxycétoesters de formule IV en les composés 3,5-dihydroxy correspondants, de formule I

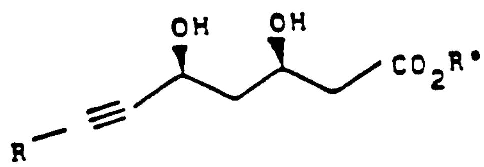

I

dans laquelle R a la signification donnée à propos de la formule I, et $R^o$ est un groupe alkyle ayant de 1 à 6 atomes de carbone, et éventuellement on saponifie un composé obtenu pour aboutir à un composé de formule I dans lequel $R^o$ représente un métal alcalin, on libère éventuellement à partir de celui-ci l'acide libre ($R^o$ est un atome d'hydrogène) et éventuellement on convertit l'acide libre en un composé de formule I, dans lequel $R^o$ a les significations données à propos de la formule I, à l'exception de celle d'un atome d'hydrogène,

c) et éventuellement on convertit un composé obtenu de formule générale I en une lactone de formule II

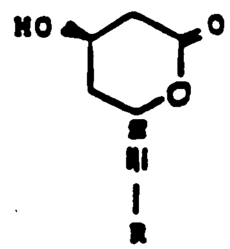

II

dans laquelle R a la signification donnée.

**2.** Procédé selon la revendication 1, caractérisé en ce que l'on prépare un composé de formule I ou II, dans lequel R représente

a) un radical de formule a, dans lequel

$R^1$ est un radical alkyle à chaîne droite ou ramifiée ayant jusqu'à 4 atomes de carbone, ou un radical cycloalkyle ayant 3-6 atomes de carbone,

$R^2$ est un radical phényle, qui est éventuellement substitué par 1-3 substituants identiques ou différents, choisis parmi les atomes de fluor et de chlore et des groupes alkyle en $C_1$-$C_4$, alcoxy ayant 1-4 atomes de carbone et hydroxy, et

X=Y-Z est un groupe de formule $CR^3=CR^4-CR^5$, $N=CR^4-CR^5$, $N=N-CR^5$ ou $N=CR^4-N$, formules dans lesquelles

$R^3$, $R^5$ représentent, indépendamment l'un de l'autre, un atome d'hydrogène ou un radical alkyle à chaîne droite ou ramifiée ayant jusqu'à 4 atomes de carbone, un radical cycloalkyle ayant 3-6 atomes de carbone ou un radical phényle qui est éventuellement substitué par 1-3 substituants identiques ou différents choisis parmi les atomes de fluor et de chlore et des groupes alkyle en $C_1$-$C_4$, alcoxy ayant 1-4 atomes de carbone et hydroxy, et

$R^4$ est un radical alkyle à chaîne droite ou ramifiée ayant jusqu'à 4 atomes de carbone, un radical cycloalkyle ayant 3-6 atomes de carbone, ou un radical phényle qui est éventuellement substitué par 1-3 substituants identiques ou différents, choisis parmi les atomes de fluor et de chlore et des groupes alkyle en $C_1$-$C_4$, alcoxy ayant 1-4 atomes de carbone et hydroxy,

b) un radical de formule b, dans laquelle

$R^6$ est un radical alkyle à chaîne droite ou ramifiée ayant jusqu'à 4 atomes de carbone, un radical cycloalkyle ayant 3-6 atomes de carbone,

$R^7$ représente un radical phényle qui est éventuellement substitué par 1-3 substituants identiques ou différents, choisis parmi les atomes de fluor et de chlore et des groupes alkyle en $C_1$-$C_4$, alcoxy ayant 1-4 atomes de carbone et hydroxy, et

U-V-W est un groupe de formule $C-NR^9-CR^8$, dans laquelle

$R^8$ représente un atome d'hydrogène ou un radical alkyle à chaîne droite ou ramifiée ayant jusqu'à 4 atomes de carbone, un radical cycloalkyle ayant 3-6 atomes de carbone, ou un radical phényle qui est éventuellement substitué par 1-3 substituants identiques ou différents, choisis parmi les atomes de fluor et de chlore et des groupes alkyle en $C_1$-$C_4$, et

$R^9$ est un radical alkyle à chaîne droite ou ramifiée ayant jusqu'à 4 atomes de carbone, un radical cycloalkyle ayant 3-6 atomes de carbone ou un radical phényle qui est éventuellement substitué par 1-3 radicaux identiques ou différents, choisis parmi les atomes de fluor et de chlore et des groupes alkyle en $C_1$-$C_4$,

c) ou un radical de formule c, dans laquelle

A-B est un groupe de formule CH-CH ou C=C,

$R^{11}$ est un radical alkyle à chaîne droite ou ramifiée ayant jusqu'à 6 atomes de carbone, un radical cycloalkyle ayant 3-6 atomes de carbone et

$R^{12}$, $R^{13}$ représentent, indépendamment l'un de l'autre, un radical alkyle à chaîne droite ou ramifiée ayant jusqu'à 6 atomes de carbone, un radical cycloalkyle ayant 3-6 atomes de carbone, un radical phényle qui est éventuellement substitué par 1-3 radicaux identiques ou différents, choisis parmi des atomes d'halogène et des groupes alkyle en $C_1$-$C_4$, alcoxy ayant 1-4 atomes de carbone et hydroxy;

et $R^o$ est un atome d'hydrogène, de sodium ou de potassium ou un radical alkyle à chaîne droite ou ramifiée ayant jusqu'à 4 atomes de carbone.

**3.** Procédé selon la revendication 1, caractérisé en ce que l'on prépare un composé de formule I ou II, dans lequel R représente

a) un radical de formule a, dans laquelle

X = Y-Z est un groupe de formule $CR^3 = CR^4\text{-}CR^5$, dans laquelle

- $R^3$ représente un atome d'hydrogène,
- $R^4$ représente le groupe isopropyle, tert-butyle, phényle ou 4-fluorophényle, et
- $R^5$ représente un atome d'hydrogène, et
- $R^1$ est le groupe isopropyle et
- $R^2$ est le groupe 4-fluorophényle,

X = Y-Z est un groupe de formule $N = CR^4\text{-}CR^5$, dans laquelle

- $R^4$ représente le groupe isopropyle, tert-butyle, phényle ou 4-fluorophényle, et
- $R^5$ représente un atome d'hydrogène, et
- $R^1$ représente le groupe isopropyle ou cyclopropyle, et
- $R^2$ représente le groupe 4-fluorophényle,

X = Y-Z est un groupe de formule $N = N\text{-}CR^5$, dans laquelle

- $R^5$ représente le groupe phényle ou 4-fluorophényle, et
- $R^1$ représente le groupe isopropyle et
- $R^2$ représente le groupe 4-fluorophényle,

X = Y-Z est un groupe de formule $N = CR^4\text{-}N$, dans laquelle

- $R^4$ représente le groupe isopropyle, tert-butyle, phényle ou 4-fluorophényle, et
- $R^1$ est le groupe isopropyle et $R^2$ est le groupe fluorophényle,

b) un radical de formule b, dans laquelle

- $R^6$ est le groupe isopropyle,
- $R^7$ est le groupe 4-fluorophényle et

U-V-W est un groupe de formule $C\text{-}NR^9\text{-}CR^8$, dans laquelle

- $R^8$ représente un atome d'hydrogène,
- $R^9$ représente le groupe isopropyle ou phényle,

c) un radical de formule c, dans laquelle

A-B est un groupe de formule C = C,

- $R^{11}$ est le groupe isopropyle et
- $R^{12}$ = $R^{13}$ et représentent chacun le groupe 4-fluoro-phényle ou 4-fluoro-3-méthylphényle, et

$R^o$ est un atome d'hydrogène, de sodium ou de potassium ou le radical méthyle, éthyle ou tert-butyle.

**4.** Composés selon la revendication 1, caractérisé en ce qu'ils correspondent à la formule I.

**5.** Procédé pour la préparation d'une composition pharmaceutique, caractérisé en ce que, dans une autre étape du procédé, on convertit en une forme d'administration appropriée un composé pouvant être obtenu selon la revendication 1.

**6.** Composés de formule III

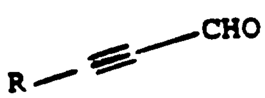

dans laquelle R a la signification donnée à propos de la formule I dans la revendication 1.

**7.** Composés de formule IV, dans lesquels R a la signification donnée à propose de la formule I dans la revendication 1, et $R^o$ est un groupe alkyle ayant de 1 à 6 atomes de carbone.